# EUROPÄISCHE PATENTSCHRIFT

(11) **EP 0 882 030 B1**
(45) Veröffentlichungstag und Bekanntmachung des Hinweises auf die Patenterteilung: **12.09.2001**
(21) Anmeldenummer: 97905065.5
(22) Anmeldetag: 20.02.1997
(51) Int. Cl.: C07D 285/14, C07D 271/12, C07D 417/00, C07D 413/00, A61K 31/41

(54) **2,1,3-BENZOTHIA(OXA)DIAZOLDERIVATE MIT ENDOTHELINREZEPTOR-ANTAGONISTISCHER WIRKUNG**
2,1,3-BENZOTHIA(OXA)DIAZOLE DERIVATIVES HAVING AN ENDOTHELIN RECEPTOR ANTAGONISTIC EFFECT
DERIVES DE 2,1,3-BENZOTHIA(OXA)DIAZOLE A EFFET ANTAGONISTE A L'EGARD DU RECEPTEUR D'ENDOTHELINE

(30) Priorität: 24.02.1996 DE 19607096
(43) Veröffentlichungstag der Anmeldung: 09.12.1998
(73) Patentinhaber: MERCK PATENT GmbH, 64293 Darmstadt (DE)
(72) Erfinder: DORSCH, Dieter, D-64372 Ober-Ramstadt (DE); OSSWALD, Mathias, D-64673 Zwingenberg (DE); MEDERSKI, Werner, D-64390 Erzhausen (DE); WILM, Claudia, D-64367 Mühltal (DE); SCHMITGES, Claus, D-64367 Mühltal (DE); CHRISTADLER, Maria, D-63322 Rödermark (DE); ANZALI, Soheila, D-64297 Darmstadt (DE)
(86) Internationale Anmeldenummer: EP9700818
(87) Internationale Veröffentlichungsnummer: WO9730982

(56) Entgegenhaltungen:
- EP-A- 0 733 626
- WO-A-95/05376

## Beschreibung

Die Erfindung betrifft Verbindungen der Formel I 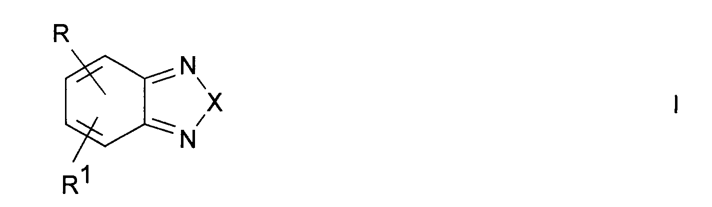 worin
- R: 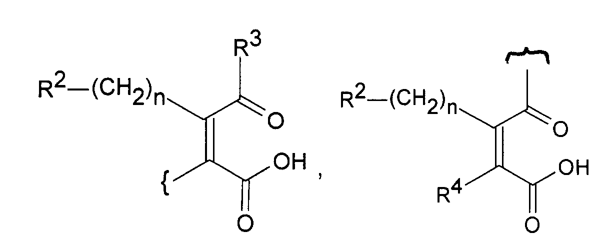 oder 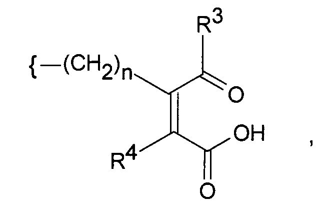
- x: O oder S,
- R¹: H, Hal, OH, OA, A, Alkylen-O-A, NO₂, NH₂, NHAcyl, SO₂NH₂, SO₃-A, SO₂NHA, CN oder Formyl,
- R², R³, R⁴: jeweils unabhängig voneinander eine unsubstituierte oder ein- oder mehrfach durch Hal, OH, OA, O-Alkylen-R⁵, A, S-A, SOA, SO₂A, SOR⁵, SO₂R⁵, NO₂, NH₂, NHA, NA₂, NHAcyl, NHSO₂A, NHSO₂R⁵, NASO₂A, NASO₂-R⁵, NH(CO)NH₂, NH(CO)NHA, Formyl, NH(CO)NHR⁵, NHCOOA, NAAcyl, NHCOOCH₂R⁵, NHSO₂CH₂R⁵, NHCOO-Alkylen-OA, NH(CO)NA₂, 1-Piperidinyl-CO-NH, 1-Pyrrolidinyl-CONH, O(CH₂)ₙCOOA, O(CH₂)ₙCOOH, O(CH₂)ₙOH, O(CH₂)ₙOA, CH₂OH, CH₂OA, COOH, COOA, CH₂COOH oder CH₂COOA substituierte Phenylgruppe,
eine 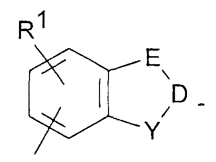 oder eine 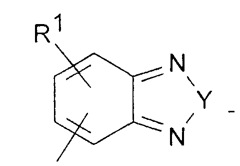 Gruppe, wobei
R² noch zusätzlich A oder Cycloalkyl bedeutet,
- R⁵: eine unsubstituierte oder ein- oder mehrfach durch Hal, OH, OA, A, S-A, NO₂, NH₂, NHA, NA₂, NHAcyl, NHSO₂A, NASO₂A, NH(CO)NH₂, NH(CO)NHA, Formyl,NHCOOA, NAAcyl, NHCOO-Alkylen-OA, NH(CO)NA₂, N-Piperidinyl-CO-NH, N-Pyrrolidinyl-CONH, O(CH₂)ₙCOOA, O(CH₂)ₙCOOH, O(CH₂)ₙOH, O(CH₂)ₙOA, CH₂OH, CH₂OA, COOH, COOA, CH₂COOH oder CH₂COOA substituierte Phenylgruppe,
- A: Alkyl mit 1-6 C-Atomen, worin eine oder zwei CH₂-Gruppen durch O- oder S-Atome oder durch -CR⁶=CR^{6'}-Gruppen und /oder 1-7 H-Atome durch F ersetzt sein können,
- D: Carbonyl oder [C(R⁶R^{6'})]ₘ,
- E: CH₂, S oder O,
- Y: O oder S,
- R⁶ und R^{6'}: jeweils unabhängig voneinander H, F oder A,
- Hal: Fluor, Chlor, Brom oder lod,
- n: 1 oder 2 und
- m: 1 oder 2 bedeutet,
oder eine tautomere ringgeschlossene Form, sowie die (E)-lsomeren und die Salze aller Isomeren.

Die tautomere ringgeschlossene Hydroxylactonform 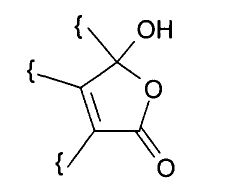 liegt vor, wenn die Verbindungen der Formel I als Carbonsäuren isoliert werden. Fallen die Verbindungen der Formel I als Salze (Carboxylate) an, so erhält man das offenkettige Tautomere.

Ähnliche Verbindungen sind aus WO 95/05376 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Endothelinrezeptor-antagonistische Eigenschaften und können daher zur Behandlung von Krankheiten wie Hypertonie, Herzinsuffizienz, koronare Herzerkrankung, renale, cerebrale und myocardiale Ischämie, Niereninsuffizienz, Hirninfarkt, subarachnoidale Hämorrhagie, Arteriosklerose, pulmonaler Hochdruck, Entzündungen, Asthma, Prostatahyperplasie, endotoxischer Schock und bei Komplikationen nach der Verabreichung von Substanzen wie z.B. Cyclosporin, sowie anderen, mit Endothelin-Aktivitäten assoziierten Krankheiten eingesetzt werden.

Die Verbindungen zeigen u.a. eine hohe Affinität zu den Endothelin-Subrezeptoren ET_{A} und ET_{B}. Diese Wirkungen können nach üblichen in vitro- oder in vivo-Methoden ermittelt werden, wie z.B. beschrieben von P.D. Stein et al., J. Med. Chem. 37, 1994, 329-331 und E. Ohlstein et al., Proc. Natl. Acad. Sci. USA 91, 1994, 8052-8056.

Eine geeignete Methode zur Bestimmung der blutdrucksenkenden Wirkung wird z. B. beschrieben von M.K. Bazil et al., J. Cardiovasc. Pharmacol. 22, 1993, 897-905 und J. Lange et al., Lab Animal 20, 1991, Appl. Note 1016.

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie und Herzinsuffizienz.

Gegenstand der Erfindung sind die Verbindungen der Formel I und ihre Salze sowie ein Verfahren zur Herstellung dieser Verbindungen sowie ihrer Salze,
worin
a) R 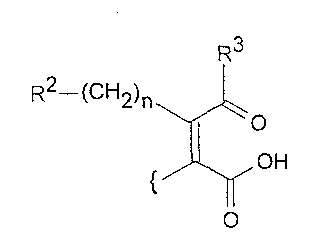 bedeutet,
   dadurch gekennzeichnet, daß man eine Verbindung der Formel II 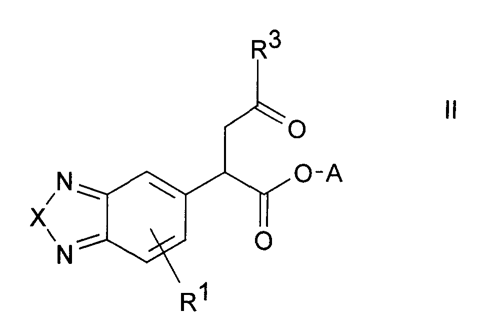 worin
   R¹, R³ und X die in Anspruch 1 angegebene Bedeutung haben, und A Alkyl mit 1-4 C-Atomen oder Benzyl bedeutet,
   mit einer Verbindung der Formel III

   R²-(CH₂)ₖ-CHO III

   worin
   - R²: die in Anspruch 1 angegebene Bedeutung hat und
   - k: 0 oder 1 bedeutet,
   umsetzt,
   und anschließend den Ester spaltet,
   oder daß man zur Herstellung von Verbindungen der Formel I nach Anspruch 1 sowie ihrer Salze,
   worin
b) R 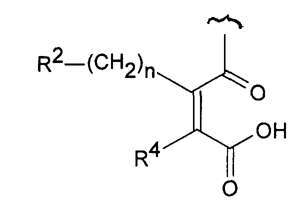 bedeutet,
   eine Verbindung der Formel IV 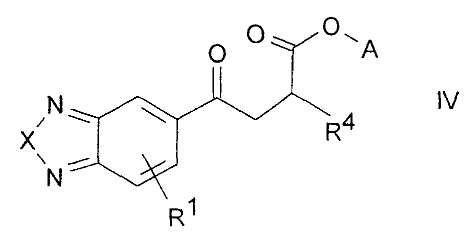 worin
   R¹, R⁴ und X die in Anspruch 1 angegebene Bedeutung haben, und A Alkyl mit 1-4 C-Atomen oder Benzyl bedeutet,
   mit einer Verbindung der Formel III, wie angegeben,
   umsetzt,
   und anschließend den Ester spaltet,
   oder daß man zur Herstellung von Verbindungen der Formel I nach Anspruch 1 sowie ihrer Salze,
   worin
c) R 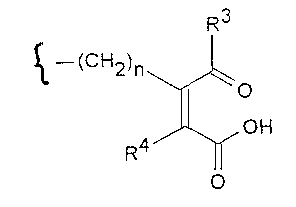 bedeutet,
   eine Verbindung der Formel V 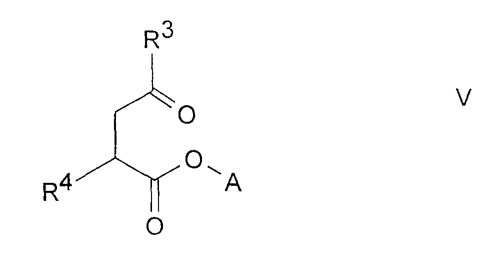 worin
   R³ und R⁴ die in Anspruch 1 angegebene Bedeutung haben, und A Alkyl mit 1-4 C-Atomen oder Benzyl bedeutet,
   mit einer Verbindung der Formel VI, 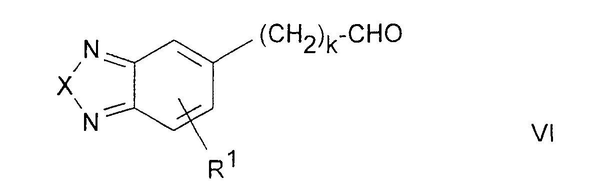 worin
   R¹ und X die in Anspruch 1 angegebenen Bedeutungen haben, und k 0 oder 1 bedeutet,
   umsetzt,
   und anschließend den Ester spaltet,
   und/oder daß man in einer Verbindung der Formel I einen oder mehrere Rest(e) R¹, R², R³ und/oder R⁴ in einen oder mehrere Reste R¹, R², R³ und/oder R⁴ umwandelt,
   indem man beispielsweise
   i) eine Nitrogruppe zu einer Aminogruppe reduziert,
   ii) eine Aminogruppe acyliert oder alkyliert,
   iii) eine Aminogruppe in eine Sulfonamidogruppe umwandelt, und/oder eine Base oder Säure der Formel I in eines ihrer Salze umwandelt.

Für alle Reste, die mehrfach auftreten, wie z. B. R³, R⁴ oder R⁵ gilt, daß deren Bedeutungen unabhängig voneinander sind.

Für X und Y gilt ebenfalls, daß deren Bedeutungen unabhängig voneinander sind.

Vor- und nachstehend haben die Reste bzw. Parameter R, X, R¹, R², R³, R⁴, A, k und n die bei den Formeln I bis VI angegebenen Bedeutungen, falls nicht ausdrücklich etwas anderes angegeben ist.

In den vorstehenden Formeln bedeutet A Alkyl und hat 1 bis 6, vorzugsweise 1, 2, 3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1- , 1,2-, 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiterhin Trifluormethyl, Pentafluorethyl, Allyl oder Crotyl.

Cycloalkyl ist vorzugsweise Cyclopropyl, Cyclobutyl, Cyclopentyl oder Cyclohexyl.

Alkylen bedeutet vorzugsweise Methylen, Ethylen, Propylen, Butylen, ferner Pentylen oder Hexylen.

Acyl bedeutet vorzugsweise Formyl, Acetyl, Propionyl, ferner auch Butyryl, Pentanoyl oder Hexanoyl.

E bedeutet vorzugsweise O, ferner auch CH₂ oder S.

D bedeutet vorzugsweise CH₂, ebenso ist auch Carbonyl bevorzugt.

Hal bedeutet vorzugsweise F, Cl oder Br, aber auch I.

R¹ bedeutet vorzugsweise H, Fluor, Chlor, Brom, Iod, Methoxy, Ethoxy, Propoxy, Methoxymethyl, Nitro, Amino, Formamido, Acetamido, Sulfonamido, Methylsulfonamido, N-Methylsulfonamido, Cyan ferner auch Formyl.

R², R³ und R⁴ bedeuten jeweils unabhängig voneinander unsubstituiertes Phenyl, vorzugsweise durch Fluor, Chlor, Brom, lod, Hydroxy, Methoxy, Ethoxy, Propoxy, Butoxy, Pentyloxy, Hexyloxy, Benzyloxy, Phenethyloxy, Methylthio, Ethylthio, Methylsulfinyl, Ethylsulfinyl, Methylsulfonyl, Ethylsulfonyl, Phenylsulfinyl, Phenylsulfonyl, Nitro, Amino, Methylamino, Ethylamino, Dimethylamino, Diethylamino, Formamido, Acetamido, N-Methylacetamido, N-Ethylacetamido, N-Propylacetamido, N-Butylacetamido, Propionylamino, Butyrylamino, Methylsulfonamido, Ethylsulfonamido, Propylsulfonamido, Butylsulfonamido, N-Methyl-methylsulfonamido, N-Methyl-ethylsulfonamido, N-Ethyl-methylsulfonamido, N-Ethyl-ethylsulfonamido, N-Propyl-methylsulfonamido, N-Propyl-ethylsulfonamido, N-Butylmethylsulfonamido, N-Butyl-ethylsulfonamido, Phenylsulfonamido, (4-Methylphenyl)-sulfonamido, Ureido, Methylureido, Phenylureido, Methoxycarbonylamino, Ethoxycarbonylamino, Formyl, Hydroxymethyl, Methoxymethyl, Ethoxymethyl, Anilino, Phenoxycarbonylamino, Benzyloxycarbonylamino, Benzylsulfonamido, N,N-Dimethylureido, 1-Piperidinyl-CONH-, 1-Pyrrolidinyl-CONH, Hydroxyethoxycarbonylamino, Methoxyethoxycarbonylamino, Carboxymethoxy, Carboxyethoxy, Methoxycarbonylmethoxy, Methoxycarbonylethoxy, Hydroxyethoxy, Methoxyethoxy, Carboxy, Methoxycarbonyl, Ethoxycarbonyl, Carboxymethyl, Methoxycarbonylmethyl oder Ethoxycarbonylmethyl monosubstituiertes Phenyl, wobei R² weiter bevorzugt A oder Cycloalkyl bedeutet.

R², R³ und R⁴ bedeuten bevorzugt o-, m- oder p-Tolyl, o-, m- oder p-Ethylphenyl, o-, m- oder p-Propylphenyl, o-, m- oder p-lsopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-Acetamidophenyl, o-, m- oder p-Methoxyphenyl, o-, m- oder p-Ethoxyphenyl, o-, m- oder p-(N,N-Dimethylamino)-phenyl, o-, m- oder p-(N-Ethylamino)-phenyl, o-, m- oder p-(N,N-Diethylamino)-phenyl, o-, m-oder p-Fluorphenyl, o-, m- oder p-Bromphenyl, o-, m- oder p- Chlorphenyl, o-, m- oder p-Formylphenyl, o-, m- oder p-(Phenylsulfonamido)-phenyl, o-, m- oder p-(Methylsulfonamido)-phenyl, o-, m- oder p-Methylthiophenyl, o-, m- oder p-Benzyloxyphenyl, o-, m- oder p-Ureidophenyl, o-, m- oder p-(N-Methylureido)-phenyl, o-, m- oder p-(Hydroxymethyl)-phenyl, o-, m- oder p-(Methoxymethyl)-phenyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl, 2,3-, 2,4- 2,5-, 2,6-, 3,4- oder 3,5-(Difluormethoxy)-(carboxymethyloxy)-phenyl, 2,3-, 2,4- 2,5-, 2,6-, 3,4- oder 3,5-Methoxy-(carboxymethyloxy)-phenyl, 2,3-, 2,4- 2,5-, 2,6-, 3,4- oder 3,5-Hydroxy-(carboxymethyloxy)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, ferner bevorzugt 2,3-Dihydrobenzofuranyl, 2,3-Dihydro-2-oxo-furanyl, 2,1,3-Benzothiadiazol-4-oder -5-yl oder 2,1,3-Benzoxadiazol-5-yl.

R⁵ bedeutet unsubstituiertes Phenyl, vorzugsweise durch Fluor, Chlor, Brom, lod, Hydroxy, Methoxy, Ethoxy, Propoxy, Butoxy, Pentyloxy, Hexyloxy, Methylthio, Ethylthio, Nitro, Amino, Methylamino, Ethylamino, Dimethylamino, Diethylamino, Formamido, Acetamido, N-Methylacetamido, N-Ethylacetamido, N-Propylacetamido, N-Butylacetamido, Propionylamino, Butyrylamino, Methylsulfonamido, Ethylsulfonamido, Propylsulfonamido, Butylsulfonamido, N-Methyl-methylsulfonamido, N-Methyl-ethylsulfonamido, N-Ethyl-methylsulfonamido, N-Ethyl-ethylsulfonamido, N-Propylmethylsulfonamido, N-Propyl-ethylsulfonamido, N-Butyl-methylsulfonamido, N-Butyl-ethylsulfonamido, Ureido, Methylureido, Phenylureido, Methoxycarbonylamino, Ethoxycarbonylamino, Formyl, Hydroxymethyl, Methoxymethyl, Ethoxymethyl, N,N-Dimethylureido, N-Piperidinyl-CONH-, N-Pyrrolidinyl-CONH, Hydroxyethoxycarbonylamino, Methoxyethoxycarbonylamino, Carboxymethoxy, Carboxyethoxy, Methoxycarbonylmethoxy, Methoxycarbonylethoxy, Hydroxyethoxy oder Methoxyethoxy monosubstituiertes Phenyl. R⁵ bedeutet bevorzugt o-, m- oder p-Tolyl, o-, m- oder p-Ethylphenyl, o-, m- oder p-Propylphenyl, o-, m- oder p-lsopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-Acetamidophenyl, o-, m- oder p-Methoxyphenyl, o-, m- oder p-Ethoxyphenyl, o-, m- oder p-(N,N-Dimethylamino)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m-oder p-Formylphenyl, o-, m- oder p-(Methylsulfonamido)-phenyl, o-, m-oder p-Methylthiophenyl, o-, m- oder p-Ureidophenyl, o-, m- oder p-(N-Methylureido)-phenyl, o-, m- oder p-(Hydroxymethyl)-phenyl, o-, m- oder p-(Methoxymethyl)-phenyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl, 2,3-, 2,4- 2,5-, 2,6-, 3,4- oder 3,5-(Difluormethoxy)-(carboxymethyloxy)-phenyl, 2,3-, 2,4- 2,5-, 2,6-, 3,4- oder 3,5-Methoxy-(carboxymethyloxy)-phenyl, 2,3-, 2,4- 2,5-, 2,6-, 3,4- oder 3,5-Hydroxy-(carboxymethyloxy)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-Iodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl oder 3-Chlor-4-acetamidophenyl.

Die Verbindungen der Formel I können ein oder mehrere chirale Zentren besitzen und daher in verschiedenen stereoisomeren Formen vorkommen. Die Formel I umschließt alle diese Formen.

Besonders bevorzugt sind die Z-Isomeren der Formel I, d.h. die Verbindungen, in denen die C=C-Doppelbindung im Rest R in der Z-Konfiguration vorliegt.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis le ausgedrückt werden, die der Formel I entsprechen und worin die nicht näher bezeichneten Reste die bei der Formel I angegebene Bedeutung haben, worin jedoch
in la
   - R: 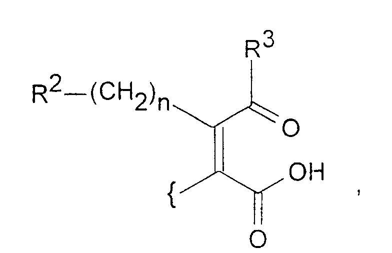
   - R¹: H,
   - x: S und
   - n: 1 bedeutet;
in Ib
   - R: 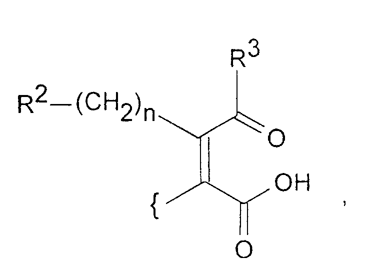
   - R¹: H,
   - x: O und
   - n: 1 bedeutet;
in Ic
   - R: 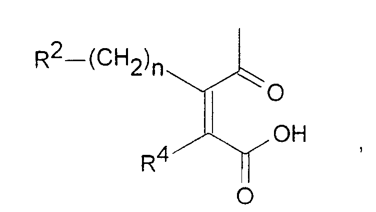
   - R¹: H,
   - x: S und
   - n: 1 bedeutet;
in Id
   - R: 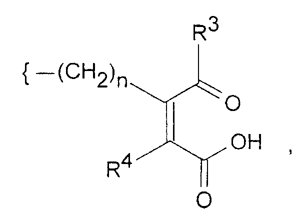
   - R¹: H,
   - x: S und
   - n: 1 bedeutet;
in le
   - R: 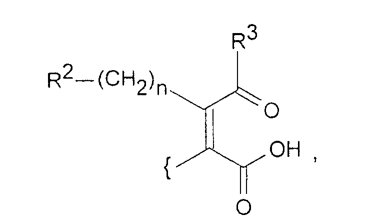
   - R¹: H,
   - R² und R³: jeweils unabhängig voneinander eine unsubstituierte oder ein- oder mehrfach durch Hal, OH, OA, O-Alkylen-R⁵, A, S-A, SOA, SO₂A, SOR⁵, SO₂R⁵, NO₂, NH₂, NHA, NA₂, NHAcyl, NHSO₂A, NHSO₂R⁵, NASO₂A, NASO₂-R⁵, NH(CO)NH₂, NH(CO)NHA, Formyl, NH(CO)NHR⁵, NHCOOA, NAAcyl, NHCOOCH₂R⁵, NHSO₂CH₂R⁵, NHCOO-Alkylen-OA, NH(CO)NA₂, 1-Piperidinyl-CO-NH, 1-Pyrrolidinyl-CONH, O(CH₂)ₙCOOA, O(CH₂)ₙCOOH, O(CH₂)ₙOH, O(CH₂)ₙOA, CH₂OH, CH₂OA, COOH, COOA, CH₂COOH, CH₂COOA, -O-CH₂-CH₂-O-, -O-CH₂-CH₂-, -O-CF₂-O- oder -O-CF₂-CF₂-O- substituierte Phenylgruppe,
   eine 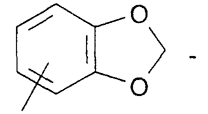 oder eine 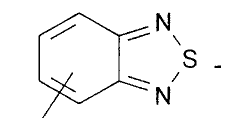 Gruppe,
   wobei R² noch zusätzlich A oder Cycloalkyl bedeutet,
   - x: S und
   - n: 1 oder 2 bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I, worin R 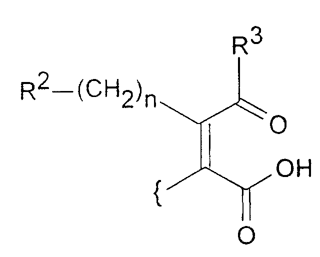 deutet,
können vorzugsweise erhalten werden, indem man Verbindungen der Formel II mit Verbindungen der Formel III umsetzt, und anschließend den Ester spaltet.

Die Umsetzung erfolgt in der Regel in einem inerten Lösungsmittel, vorzugsweise in Gegenwart einer Base. Als Base dient z.B. ein Kalium- oder Natriumalkoholat wie Kalium- oder Natriummethylat, -ethylat oder -tert.-butylat. Als Lösungsmittel sind besonders die zugrundeliegenden Alkohole bevorzugt.
Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Ausgangsverbindungen der Formel II sind in der Regel neu, während die der Formel III in der Regel bekannt sind.
Die Verbindungen der Formel II können aber nach an sich bekannten Methoden hergestellt werden. So kann z.B. 2-(2,1,3-Benzothiadiazol-5-yl)-4-(4-methoxyphenyl)-4-oxo-butansäureethylester durch Umsetzung von 2-(2,1,3-Benzothiadiazol-5-yl)-essigsäureethylester mit 2'-Brom-4-methoxyacetophenon in einem inerten Lösungsmittel unter Zusatz eines säurebindenden Mittels, vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums, erhalten werden. Auch der Zusatz einer organischen Base wie Triethylamin, Dimethylanilin, Pyridin oder Chinolin kann günstig sein.

Vorzugsweise arbeitet man bei Temperaturen zwischen 0° und 150°. Als inerte Lösungsmittel eignen sich die schon oben erwähnten.

Verbindungen der Formel I, worin R 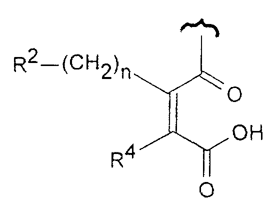 bedeutet,
können vorzugsweise erhalten werden, indem man Verbindungen der Formel IV mit Verbindungen der Formel III umsetzt, und anschließend den Ester spaltet.

Die Umsetzung erfolgt in der Regel in einem inerten Lösungsmittel, in Gegenwart eines säurebindenden Mittels und bei Temperaturen wie oben angegeben.

Die Ausgangsverbindungen der Formel IV sind in der Regel neu, können aber nach an sich bekannten Methoden hergestellt werden:

So erhält man z.B. durch Umsetzung von Verbindungen der Formel Vll 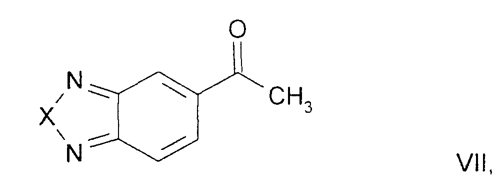
- worin X: O oder S bedeutet,
mit Verbindungen der Formel VIII

R⁴-CHO VIII,

worin R⁴ die in Anspruch 1 angegebene Bedeutung hat,
Verbindungen der Formel IX 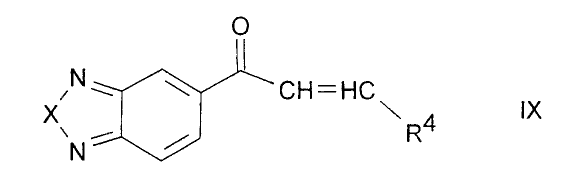 worin X und R⁴ die angegebenen Bedeutungen haben.
Durch anschließende Addition von Blausäure erhält man Verbindungen der Formel X 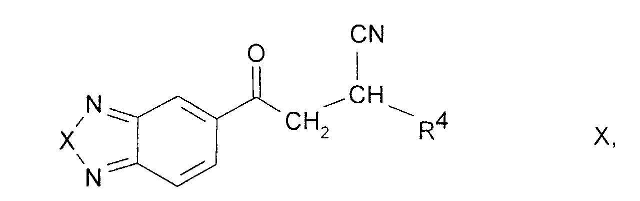 die durch nachfolgende Umwandlung der Nitrilgruppe in eine COOH-Gruppe und anschließender Veresterung in Verbindungen der Formel IV, worin
- R¹: H bedeutet,
überführt werden können.

Verbindungen der Formel I, worin R 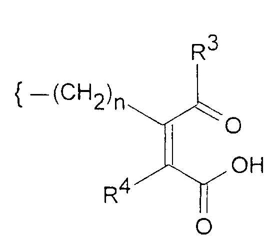 bedeutet,
können vorzugsweise erhalten werden, indem man Verbindungen der Formel V mit Verbindungen der Formel VI umsetzt, und anschließend den Ester spaltet.

Die Umsetzung erfolgt in der Regel in einem inerten Lösungsmittel, in Gegenwart eines säurebindenden Mittels und bei Temperaturen wie oben angegeben.

Ester können z.B. mit Essigsäure oder mit NaOH oder KOH in Wasser, Wasser-THF oder Wasser-Dioxan bei Temperaturen zwischen 0 und 100° verseift werden.

Die Ausgangsverbindungen der Formel V sind in der Regel neu, können aber analog der Herstellung der Verbindungen der Formel X mit anschließender Umwandlung der Nitrilgruppe in eine Esterfunktion, erhalten werden.
Die Ausgangsverbindungen der Formel VI sind in der Regel bekannt oder können nach bekannten Verfahren hergestellt werden.

Es ist ferner möglich, eine Verbindung der Formel t in eine andere Verbindung der Formel I umzuwandeln, indem man einen oder mehrere Rest(e) R¹, R², R³ und/oder R⁴ in einen oder mehrere Reste R¹, R², R³ und/oder R⁴ umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°

Gewünschtenfalls kann in einer Verbindung der Formel I eine funktionell abgewandelte Amino- und /oder Hydroxygruppe durch Solvolyse oder Hydrogenolyse nach üblichen Methoden in Freiheit gesetzt werden. So kann z.B. eine Verbindung der Formel I, die eine NH-Acyl- oder eine COOA-Gruppe enthält, in die entsprechende Verbindung der Formel I umgewandelt werden, die stattdessen eine NH₂**-** oder eine HOOC-Gruppe enthält.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, insbesondere von Hypertonie und Herzinsuffizienz verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: El (Elektronenstoß-lonisation) M⁺
FAB (Fast Atom Bombardment) (M+H)⁺

### Beispiel 1

Zu einer Lösung von 80 mg Natrium in 5 ml Methanol gibt man 0,38 g Benzaldehyd und 1,20 g 2-(2,1,3-Benzothiadiazol-5-yl)-4-(4-methoxyphenyl)-4-oxo-butansäureethylester ("A"), F. 89° (erhältlich durch Umsetzung von 5,5 g 2-(2,1,3-Benzothiadiazol-5-yl)-essigsäureethylester mit 5,5, g 2'-Brom-4-methoxyacetophenon und 4 g Kaliumcarbonat in 200 ml Aceton, 18 Stunden unter Rückfluß; 2-(2,1,3-Benzothiadiazol-5-yl)-essigsäureethylester, F. 40-41° erhält man durch Reaktion von 24,3 g 3,4-Diaminophenylessigsäureethylester und 26,9 ml Thionylanilin in 80 ml Toluol, 4 Stunden unter Rückfluß) und erhitzt eine Stunde unter Rückfluß. Nach Zugabe von 5 ml Essigsäure wird weitere 16 Stunden erhitzt. Das Lösungsmittel wird entfernt und wie üblich aufgearbeitet. Man erhält 3-(2,1,3-Benzothiadiazol-5-yl)-4-benzyl-5-hydroxy-5-(4-methoxyphenyl)-5H-furan-2-on, FAB 431.

Analog erhält man durch Umsetzung von "A"
mit 2-Methoxybenzaldehyd
   3-(2,1,3-Benzothiadiazol-5-yl)-4-(2-methoxybenzyl)-5-hydroxy-5-(4-methoxyphenyl)-5H-furan-2-on, F. 131°
mit 3-Methoxybenzaldehyd
   3-(2,1,3-Benzothiad iazol-5-yl)-4-(3-methoxybenzyl)-5-hydroxy-5-(4-methoxyphenyl)-5H-furan-2-on, FAB 460
mit 4-Methoxybenzaldehyd
   3-(2,1,3-Benzothiadiazol-5-yl)-4-(4-methoxybenzyl)-5-hydroxy-5-(4-methoxyphenyl)-5H-furan-2-on, F. 70° und als Nebenprodukt 2-(2,1,3-Benzothiadiazol-5-yl)-3-(4-methoxybenzyl)-4-(4-methoxyphenyl)-4-oxo-2-butensäure, F. 184°
mit 3,4-Dimethoxybenzaldehyd
   3-(2,1,3-Benzothiadiazol-5-yl)-4-(3,4-dimethoxybenzyl)-5-hydroxy-5-(4-methoxyphenyl)-5H-furan-2-on, F. 62°
mit 3,4,5-Trimethoxybenzaldehyd
   3-(2,1,3-Benzothiadiazol-5-yl)-4-(3,4,5-trimethoxybenzyl)-5-hydroxy-5-(4-methoxyphenyl)-5H-furan-2-on, F. 164°, FAB 543
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(4-methoxyphenyl)-5H-furan-2-on, F. 136°
mit 3,4,5-Triisopropoxybenzaldehyd
   3-(2,1,3-Benzothiadiazol-5-yl)-4-(3,45-triisopropoxybenzyl)-5-hydroxy-5-(4-methoxyphenyl)-5H-furan-2-on, F. 149°
mit 4-Trifluormethylbenzaldehyd
   3-(2,1,3-Benzothiadiazol-5-yl)-4-(4-trifluormethylbenzyl)-5-hydroxy-5-(4-methoxyphenyl)-5H-furan-2-on,
mit 4-Cyanbenzaldehyd
   3-(2,1,3-Benzothiadiazol-5-yl)-4-(4-cyanbenzyl)-5-hydroxy-5-(4-methoxyphenyl)-5H-furan-2-on,
mit 4-Methylbenzaldehyd
   3-(2,1,3-Benzothiadiazol-5-yl)-4-(4-methylbenzyl)-5-hydroxy-5-(4-methoxyphenyl)-5H-furan-2-on, F. 58°
mit 3-Methyl-4-methoxybenzaldehyd
   3-(2,1,3-Benzothiadiazol-5-yl)-4-(3-methyl-4-methoxybenzyl)-5-hydroxy-5-(4-methoxyphenyl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(4-methoxyphenyl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(4-methoxyphenyl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)benzyl]-5-hydroxy-5-(4-methoxyphenyl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(4-methoxyphenyl)-5H-furan-2-on
mit 4-Benzyloxybenzaldehyd
   3-(2,1,3-Benzothiadiazol-5-yl)-4-(4-benzyloxybenzyl)-5-hydroxy-5-(4-methoxyphenyl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(4-methoxyphenyl)-5H-furan-2-on, F. 70°
mit 4-Nitrobenzaldehyd
   3-(2,1,3-Benzothiadiazol-5-yl)-4-(4-nitrobenzyl)-5-hydroxy-5-(4-methoxyphenyl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(4-methoxyphenyl)-5H-furan-2-on, F. 67°
mit 3-Fluor-4-methoxybenzaldehyd
   3-(2,1,3-Benzothiadiazol-5-yl)-4-(3-fluor-4-methoxybenzyl)-5-hydroxy-5-(4-methoxyphenyl)-5H-furan-2-on, F. 66°
mit 3-(N,N-Dimethylamino)-benzaldehyd
   3-(2,1,3-Benzothiadiazol-5-yl)-4-[3-(N,N-dimethylamino)benzyl]-5-hydroxy-5-(4-methoxyphenyl)-5H-furan-2-on,
mit Cyclohexancarbaldehyd
   3-(2,1,3-Benzothiadiazol-5-yl)-4-cyclohexylmethyl-5-hydroxy-5-(4-methoxyphenyl)-5H-furan-2-on, F. 153° und
mit Cyclopentancarbaldehyd
   3-(2,1,3-Benzothiadiazol-5-yl)-4-cyclopentylmethyl-5-hydroxy-5-(4-methoxyphenyl)-5H-furan-2-on.

Analog erhält man durch Umsetzung von 2-(2,1,3-Benzothiadiazol-5-yl)-4-(1,4-benzodioxan-6-yl)-4-oxo-butansäureethylester
mit Benzaldehyd
   3-(2,1,3-Benzothiadiazol-5-yl)-4-benzyl-5-hydroxy-5-(1,4-benzodioxan-6-yl)-5H-furan-2-on, F. 98°
mit 2-Methoxybenzaldehyd
   3-(2,1,3-Benzothiadiazol-5-yl)-4-(2-methoxybenzyl)-5-hydroxy-5-(1,4-benzodioxan-6-yl)-5H-furan-2-on,
mit 3-Methoxybenzaldehyd
   3-(2,1,3-Benzothiadiazol-5-yl)-4-(3-methoxybenzyl)-5-hydroxy-5-(1,4-benzodioxan-6-yl)-5H-furan-2-on,
mit 4-Methoxybenzaldehyd
   3-(2,1,3-Benzothiadiazol-5-yl)-4-(4-methoxybenzyl)-5-hydroxy-5-(1,4-benzodioxan-6-yl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(1,4-benzodioxan-6-yl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(1,4-benzodioxan-6-yl)-5H-furan-2-on, F. 82°
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(1,4-benzodioxan-6-yl)-5H-furan-2-on, F. 173°
mit 3,4,5-Triisopropoxybenzaldehyd
   3-(2,1,3-Benzothiadiazol-5-yl)-4-(3,4,5-triisopropoxybenzyl)-5-hydroxy-5-(1,4-benzodioxan-6-yl)-5H-furan-2-on,
mit 4-Trifluormethylbenzaldehyd
   3-(2,1,3-Benzothiadiazol-5-yl)-4-(4-trifluormethylbenzyl)-5-hydroxy-5-(1,4-benzodioxan-6-yl)-5H-furan-2-on,
mit 4-Cyanbenzaldehyd
   3-(2,1,3-Benzothiadiazol-5-yl)-4-(4-cyanbenzyl)-5-hydroxy-5-(1,4-benzodioxan-6-yl)-5H-furan-2-on,
mit 4-Methylbenzaldehyd
   3-(2,1,3-Benzothiadiazol-5-yl)-4-(4-methylbenzyl)-5-hydroxy-5-(1,4-benzodioxan-6-yl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(1,4-benzodioxan-6-yl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(1,4-benzodioxan-6-yl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(1,4-benzodioxan-6-yl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)benzyl]-5-hydroxy-5-(1,4-benzodioxan-6-yl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(1,4-benzodioxan-6-yl)-5H-furan-2-on,
mit 4-Benzyloxybenzaldehyd
   3-(2,1,3-Benzothiadiazol-5-yl)-4-(4-benzyloxybenzyl)-5-hydroxy-5-(1,4-benzodioxan-6-yl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(1,4-benzodioxan-6-yl)-5H-furan-2-on,
mit 4-Nitrobenzaldehyd
   3-(2,1,3-Benzothiadiazol-5-yl)-4-(4-nitrobenzyl)-5-hydroxy-5-(1,4-benzodioxan-6-yl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(1,4-benzodioxan-6-yl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(1,4-benzodioxan-6-yl)-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-(1,4-benzodioxan-6-yl)-5H-furan-2-on,
mit Cyclohexancarbaldehyd
   3-(2,1,3-Benzothiadiazol-5-yl)-4-cyclohexylmethyl-5-hydroxy-5-(1,4-benzodioxan-6-yl)-5H-furan-2-on, F. 81° und
mit Cyclopentancarbaldehyd
   3-(2,1,3-Benzothiadiazol-5-yl)-4-cyclopentylmethyl-5-hydroxy-5-(1,4-benzodioxan-6-yl)-5H-furan-2-on.

Analog erhält man durch Umsetzung von 2-(2,1,3-Benzothiadiazol-5-yl)-4-(4-methylphenyl)-4-oxo-butansäureethylester
mit Benzaldehyd
   3-(2,1,3-Benzothiadiazol-5-yl)-4-benzyl-5-hydroxy-5-(4-methylphenyl)-5H-furan-2-on,
mit 2-Methoxybenzaldehyd
   3-(2,1,3-Benzothiadiazol-5-yl)-4-(2-methoxybenzyl)-5-hydroxy-5-(4-methylphenyl)-5H-furan-2-on,
mit 3-Methoxybenzaldehyd
   3-(2,1,3-Benzothiadiazol-5-yl)-4-(3-methoxybenzyl)-5-hydroxy-5-(4-methylphenyl)-5H-furan-2-on,
mit 4-Methoxybenzaldehyd
   3-(2,1,3-Benzothiadiazol-5-yl)-4-(4-methoxybenzyl)-5-hydroxy-5-(4-methylphenyl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(4-methylphenyl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(4-methylphenyl)-5H-furan-2-on,
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(4-methylphenyl)-5H-furan-2-on,
mit 3,4,5-Triisopropoxybenzaldehyd
   3-(2,1,3-Benzothiadiazol-5-yl)-4-(3,4,5-triisopropoxybenzyl)-5-hydroxy-5-(4-methylphenyl)-5H-furan-2-on,
mit 4-Trifluormethylbenzaldehyd
   3-(2,1,3-Benzothiadiazol-5-yl)-4-(4-trifluormethylbenzyl)-5-hydroxy-5-(4-methylphenyl)-5H-furan-2-on,
mit 4-Cyanbenzaldehyd
   3-(2,1,3-Benzothiadiazol-5-yl)-4-(4-cyanbenzyl)-5-hydroxy-5-(4-methylphenyl)-5H-furan-2-on,
mit 4-Methylbenzaldehyd
   3-(2,1,3-Benzothiadiazol-5-yl)-4-(4-methylbenzyl)-5-hydroxy-5-(4-methylphenyl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(4-methylphenyl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(4-methylphenyl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(4-methylphenyl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)benzyl]-5-hydroxy-5-(4-methylphenyl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(4-methylphenyl)-5H-furan-2-on,
mit 4-Benzyloxybenzaldehyd
   3-(2,1,3-Benzothiadiazol-5-yl)-4-(4-benzyloxybenzyl)-5-hydroxy-5-(4-methylphenyl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(4-methylphenyl)-5H-furan-2-on,
mit 4-Nitrobenzaldehyd
   3-(2,1,3-Benzothiadiazol-5-yl)-4-(4-nitrobenzyl)-5-hydroxy-5-(4-methylphenyl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(4-methylphenyl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(4-methylphenyl)-5H-furan-2-on,
mit 3-(N,N-Dimethylamino)-benzaldehyd
   3-(2,1,3-Benzothiadiazol-5-yl)-4-[3-(N, N-dimethylamino)benzyl]-5-hydroxy-5-(4-methylphenyl)-5H-furan-2-on,
mit Cyclohexancarbaldehyd
   3-(2,1,3-Benzothiadiazol-5-yl)-4-(cyclohexylmethyl)-5-hydroxy-5-(4-methylphenyl)-5H-furan-2-on und
mit Cyclopentancarbaldehyd
   3-(2,1,3-Benzothiadiazol-5-yl)-4-(cyclopentylmethyl)-5-hydroxy-5-(4-methylphenyl)-5H-furan-2-on.

Analog erhält man durch Umsetzung von 2-(2,1,3-Benzothiadiazol-5-yl)-4-phenyl-4-oxo-butansäureethylester
mit Benzaldehyd
   3-(2,1,3-Benzothiadiazol-5-yl)-4-benzyl-5-hydroxy-5-phenyl-5H-furan-2-on,
mit 2-Methoxybenzaldehyd
   3-(2,1,3-Benzothiadiazol-5-yl)-4-(2-methoxybenzyl)-5-hydroxy-5-phenyl-5H-furan-2-on,
mit 3-Methoxybenzaldehyd
   3-(2,1,3-Benzothiadiazol-5-yl)-4-(3-methoxybenzyl)-5-hydroxy-5-phenyl-5H-furan-2-on,
mit 4-Methoxybenzaldehyd
   3-(2,1,3-Benzothiad iazol-5-yl)-4-(4-methoxybenzyl)-5-hydroxy-5-phenyl-5H-furan-2-on,
mit 3,4-Dimethoxybenzaldehyd
   3-(2,1,3-Benzothiadiazol-5-yl)-4-(3,4-dimethoxybenzyl)-5-hydroxy-5-phenyl-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-phenyl-5H-furan-2-on, F. 174°
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-phenyl-5H-furan-2-on,
mit 3,4,5-Triisopropoxybenzaldehyd
   3-(2,1,3-Benzothiadiazol-5-yl)-4-(3,4,5-triisopropoxybenzyl)-5-hydroxy-5-phenyl-5H-furan-2-on,
mit 4-Trifluormethylbenzaldehyd
   3-(2,1,3-Benzothiadiazol-5-yl)-4-(4-trifluormethylbenzyl)-5-hydroxy-5-phenyl-5H-furan-2-on,
mit 4-Cyanbenzaldehyd
   3-(2,1,3-Benzothiadiazol-5-yl)-4-(4-cyanbenzyl)-5-hydroxy-5-phenyl-5H-furan-2-on,
mit 4-Methylbenzaldehyd
   3-(2,1,3-Benzothiadiazol-5-yl)-4-(4-methylbenzyl)-5-hydroxy-5-phenyl-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-phenyl-5H-furan-2-on,
mit 4-tert.-Butylbenzaldehyd
   3-(2,1,3-Benzothiadiazol-5-yl)-4-(4-tert.-butylbenzyl)-5-hydroxy-5-phenyl-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-phenyl-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)benzyl]-5-hydroxy-5-phenyl-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-phenyl-5H-furan-2-on,
mit 4-Benzyloxybenzaldehyd
   3-(2,1,3-Benzothiadiazol-5-yl)-4-(4-benzyloxybenzyl)-5-hydroxy-5-phenyl-5H-furan-2-on,
mit 4-Dimethylaminobenzaldehyd
   3-(2,1,3-Benzothiadiazol-5-yl)-4-(4-dimethylaminobenzyl)-5-hydroxy-5-phenyl-5H-furan-2-on,
mit 4-Nitrobenzaldehyd
   3-(2,1,3-Benzothiadiazol-5-yl)-4-(4-nitrobenzyl)-5-hydroxy-5-phenyl-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-phenyl-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-phenyl-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-phenyl-5H-furan-2-on,
mit Cyclohexancarbaldehyd
   3-(2,1,3-Benzothiadiazol-5-yl)-4-cyclohexylmethyl-5-hydroxy-5-phenyl-5H-furan-2-on und
mit Cyclopentancarbaldehyd
   3-(2,1,3-Benzothiadiazol-5-yl)-4-cyclopentylmethyl-5-hydroxy-5-phenyl-5H-furan-2-on.

Analog erhält man durch Umsetzung von 2-(2,1,3-Benzothiadiazol-5-yl)-4-(4-isopropoxyphenyl)-4-oxo-butansäureethylester
mit Benzaldehyd
   3-(2,1,3-Benzothiadiazol-5-yl)-4-benzyl-5-hydroxy-5-(4-isopropoxyphenyl)-5H-furan-2-on,
mit 2-Methoxybenzaldehyd
   3-(2,1,3-Benzothiadiazol-5-yl)-4-(2-methoxybenzyl)-5-hydroxy-5-(4-isopropoxyphenyl)-5H-furan-2-on,
mit 3-Methoxybenzaldehyd
   3-(2,1,3-Benzothiadiazol-5-yl)-4-(3-methoxybenzyl)-5-hydroxy-5-(4-isopropoxyphenyl)-5H-furan-2-on,
mit 4-Methoxybenzaldehyd
   3-(2,1,3-Benzothiadiazol-5-yl)-4-(4-methoxybenzyl)-5-hydroxy-5-(4-isopropoxyphenyl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(4-isopropoxyphenyl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(4-isopropoxyphenyl)-5H-furan-2-on, F. 180°
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(4-isopropoxyphenyl)-5H-furan-2-on, F. 170°
mit 3,4,5-Triisopropoxybenzaldehyd
   3-(2,1,3-Benzothiadiazol-5-yl)-4-(3,4,5-triisopropoxybenzyl)-5-hydroxy-5-(4-isopropoxyphenyl)-5H-furan-2-on,
mit 4-Trifluormethylbenzaldehyd
   3-(2,1,3-Benzothiadiazol-5-yl)-4-(4-trifluormethylbenzyl)-5-hydroxy-5-(4-isopropoxyphenyl)-5H-furan-2-on,
mit 4-Cyanbenzaldehyd
   3-(2,1,3-Benzothiadiazol-5-yl)-4-(4-cyanbenzyl)-5-hydroxy-5-(4-isopropoxyphenyl)-5H-furan-2-on,
mit 4-Methylbenzaldehyd
   3-(2,1,3-Benzothiadiazol-5-yl)-4-(4-methylbenzyl)-5-hydroxy-5-(4-isopropoxyphenyl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(4-isopropoxyphenyl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(4-isopropoxyphenyl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(4-isopropoxyphenyl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)-benzyl]-5-hydroxy-5-(4-isopropoxyphenyl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(4-isopropoxyphenyl)-5H-furan-2-on,
mit 4-Benzyloxybenzaldehyd
   3-(2,1,3-Benzothiadiazol-5-yl)-4-(4-benzyloxybenzyl)-5-hydroxy-5-(4-isopropoxyphenyl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(4-isopropoxyphenyl)-5H-furan-2-on,
mit 4-Nitrobenzaldehyd
   3-(2,1,3-Benzothiadiazol-5-yl)-4-(4-nitrobenzyl)-5-hydroxy-5-(4-isop ropoxyphenyl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(4-isopropoxyphenyl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(4-isopropoxyphenyl)-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-(4-isopropoxyphenyl)-5H-furan-2-on,
mit Cyclohexancarbaldehyd
   3-(2,1,3-Benzothiadiazol-5-yl)-4-cyclohexylmethyl-5-hydroxy-5-(4-isopropoxyphenyl)-5H-furan-2-on und
mit Cyclopentancarbaldehyd
   3-(2,1,3-Benzothiadiazol-5-yl)-4-cyclopentylmethyl-5-hydroxy-5-(4-isopropoxyphenyl)-5H-furan-2-on.

Analog erhält man durch Umsetzung von 2-(2,1,3-Benzothiadiazol-5-yl)-4-(4-benzyloxyphenyl)-4-oxo-butansäureethylester
mit Benzaldehyd
   3-(2,1,3-Benzothiadiazol-5-yl)-4-benzyl-5-hydroxy-5-(4-benzyloxyphenyl)-5H-furan-2-on,
mit 2-Methoxybenzaldehyd
   3-(2,1,3-Benzothiadiazol-5-yl)-4-(2-methoxybenzyl)-5-hydroxy-5-(4-benzyloxyphenyl)-5H-furan-2-on,
mit 3-Methoxybenzaldehyd
   3-(2,1,3-Benzothiadiazol-5-yl)-4-(3-methoxybenzyl)-5-hydroxy-5-(4-benzyloxyphenyl)-5H-furan-2-on,
mit 4-Methoxybenzaldehyd
   3-(2,1,3-Benzothiadiazol-5-yl)-4-(4-methoxybenzyl)-5-hydroxy-5-(4-benzyloxyphenyl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(4-benzyloxyphenyl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(4-benzyloxyphenyl)-5H-furan-2-on,
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(4-benzyloxyphenyl)-5H-furan-2-on,
mit 3,4,5-Triisopropoxybenzaldehyd
   3-(2,1,3-Benzothiadiazol-5-yl)-4-(3,4,5-triisopropoxybenzyl)-5-hydroxy-5-(4-benzyloxyphenyl)-5H-furan-2-on,
mit 4-Trifluormethylbenzaldehyd
   3-(2,1,3-Benzothiadiazol-5-yl)-4-(4-trifluormethylbenzyl)-5-hydroxy-5-(4-benzyloxyphenyl)-5H-furan-2-on,
mit 4-Cyanbenzaldehyd
   3-(2,1,3-Benzothiadiazol-5-yl)-4-(4-cyanbenzyl)-5-hydroxy-5-(4-benzyloxyphenyl)-5H-furan-2-on,
mit 4-Methylbenzaldehyd
   3-(2,1,3-Benzothiadiazol-5-yl)-4-(4-methylbenzyl)-5-hydroxy-5-(4-benzyloxyphenyl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(4-benzyloxyphenyl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(4-benzyloxyphenyl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(4-benzyloxyphenyl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)-benzyl]-5-hydroxy-5-(4-benzyloxyphenyl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(4-benzyloxyphenyl)-5H-furan-2-on,
mit 4-Benzyloxybenzaldehyd
   3-(2,1,3-Benzothiadiazol-5-yl)-4-(4-benzyloxybenzyl)-5-hydroxy-5-(4-benzyloxyphenyl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(4-benzyloxyphenyl)-5H-furan-2-on,
mit 4-Nitrobenzaldehyd
   3-(2,1,3-Benzothiadiazol-5-yl)-4-(4-nitrobenzyl)-5-hydroxy-5-(4-benzyloxyphenyl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(4-benzyloxyphenyl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(4-benzyloxyphenyl)-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-(4-benzyloxyphenyl)-5H-furan-2-on,
mit Cyclohexancarbaldehyd
   3-(2,1,3-Benzothiadiazol-5-yl)-4-cyclohexylmethyl-5-hydroxy-5-(4-benzyloxyphenyl)-5H-furan-2-on und
mit Cyclopentancarbaldehyd
   3-(2,1,3-Benzothiadiazol-5-yl)-4-cyclopentylmethyl-5-hydroxy-5-(4-benzyloxyphenyl)-5H-furan-2-on.

Analog erhält man durch Umsetzung von 2-(2,1,3-Benzothiadiazol-5-yl)-4-(3,4-dimethylphenyl)-4-oxo-butansäureethylester
mit Benzaldehyd
   3-(2,1,3-Benzothiadiazol-5-yl)-4-benzyl-5-hydroxy-5-(3,4-dimethylphenyl)-5H-furan-2-on,
mit 2-Methoxybenzaldehyd
   3-(2,1,3-Benzothiadiazol-5-yl)-4-(2-methoxybenzyl)-5-hydroxy-5-(3,4-dimethylphenyl)-5H-furan-2-on,
mit 3-Methoxybenzaldehyd
   3-(2,1,3-Benzothiadiazol-5-yl)-4-(3-methoxybenzyl)-5-hydroxy-5-(3,4-dimethylphenyl)-5H-furan-2-on,
mit 4-Methoxybenzaldehyd
   3-(2,1,3-Benzothiadiazol-5-yl)-4-(4-methoxybenzyl)-5-hydroxy-5-(3,4-dimethylphenyl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(3,4-dimethylphenyl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(3,4-dimethylphenyl)-5H-furan-2-on,
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(3,4-dimethylphenyl)-5H-furan-2-on,
mit 3,4,5-Triisopropoxybenzaldehyd
   3-(2,1,3-Benzothiadiazol-5-yl)-4-(3,4,5-triisopropoxybenzyl)-5-hydroxy-5-(3,4-dimethylphenyl)-5H-furan-2-on,
mit 4-Trifluormethylbenzaldehyd
   3-(2,1,3-Benzothiadiazol-5-yl)-4-(4-trifluormethylbenzyl)-5-hydroxy-5-(3,4-dimethylphenyl)-5H-furan-2-on,
mit 4-Cyanbenzaldehyd
   3-(2,1,3-Benzothiadiazol-5-yl)-4-(4-cyanbenzyl)-5-hydroxy-5-(3,4-dimethylphenyl)-5H-furan-2-on,
mit 4-Methylbenzaldehyd
   3-(2,1,3-Benzothiadiazol-5-yl)-4-(4-methylbenzyl)-5-hydroxy-5-(3,4-dimethylphenyl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(3,4-dimethylphenyl)-5H-furan-2-on,
mit 4-tert-Butylbenzaldehyd
   3-(2,1,3-Benzothiadiazol-5-yl)-4-(4-tert.-butylbenzyl)-5-hydroxy-5-(3,4-dimethylphenyl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(3,4-dimethylphenyl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)-benzyl]-5-hydroxy-5-(3,4-dimethylphenyl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(3,4-dimethylphenyl)-5H-furan-2-on,
mit 4-Benzyloxybenzaldehyd
   3-(2,1,3-Benzothiadiazol-5-yl)-4-(4-benzyloxybenzyl)-5-hydroxy-5-(3,4-dimethylphenyl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(3,4-dimethylphenyl)-5H-furan-2-on,
mit 4-Nitrobenzaldehyd
   3-(2,1,3-Benzothiadiazol-5-yl)-4-(4-nitrobenzyl)-5-hydroxy-5-(3,4-dimethylphenyl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(3,4-dimethylphenyl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(3,4-dimethylphenyl)-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-(3,4-dimethylphenyl)-5H-furan-2-on,
mit Cyclohexancarbaldehyd
   3-(2,1,3-Benzothiadiazol-5-yl)-4-cyclohexylmethyl-5-hydroxy-5-(3,4-dimethylphenyl)-5H-furan-2-on und
mit Cyclopentancarbaldehyd
   3-(2,1,3-Benzothiadiazol-5-yl)-4-cyclopentylmethyl-5-hydroxy-5-(3,4-dimethylphenyl)-5H-furan-2-on.

Analog erhält man durch Umsetzung von 2-(2,1,3-Benzothiadiazol-5-yl)-4-(1,3-benzodioxol-5-yl)-4-oxo-butansäureethylester
mit Benzaldehyd
   3-(2,1,3-Benzothiadiazol-5-yl)-4-benzyl-5-hydroxy-5-(1,3-benzodioxol-5-yl)-5H-furan-2-on, F. 71°
mit 2-Methoxybenzaldehyd
   3-(2,1,3-Benzothiadiazol-5-yl)-4-(2-methoxybenzyl)-5-hydroxy-5-(1,3-benzodioxol-5-yl)-5H-furan-2-on,
mit 3-Methoxybenzaldehyd
   3-(2,1,3-Benzothiadiazol-5-yl)-4-(3-methoxybenzyl)-5-hydroxy-5-(1,3-benzodioxol-5-yl)-5H-furan-2-on,
mit 4-Methoxybenzaldehyd
   3-(2,1,3-Benzothiadiazol-5-yl)-4-(4-methoxybenzyl)-5-hydroxy-5-(1,3-benzodioxol-5-yl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(1,3-benzodioxol-5-yl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(1,3-benzodioxol-5-yl)-5H-furan-2-on, F. 86°
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(1,3-benzodioxol-5-yl)-5H-furan-2-on,
mit 3,4,5-Triisopropoxybenzaldehyd
   3-(2,1,3-Benzothiadiazol-5-yl)-4-(3,4,5-triisopropoxybenzyl)-5-hydroxy-5-(1,3-benzodioxol-5-yl)-5H-furan-2-on,
mit 4-Trifluormethylbenzaldehyd
   3-(2,1,3-Benzothiadiazol-5-yl)4-(4-trifluormethylbenzyl)-5-hydroxy-5-(1,3-benzod ioxol-5-yl)-5H-furan-2-on,
mit 4-Cyanbenzaldehyd
   3-(2,1,3-Benzothiadiazol-5-yl)-4-(4-cyanbenzyl)-5-hydroxy-5-(1,3-benzodioxol-5-yl)-5H-furan-2-on,
mit 4-Methylbenzaldehyd
   3-(2,1,3-Benzothiadiazol-5-yl)-4-(4-methylbenzyl)-5-hydroxy-5-(1,3-benzodioxol-5-yl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(1,3-benzodioxol-5-yl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(1,3-benzodioxol-5-yl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(1,3-benzodioxol-5-yl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)-benzyl]-5-hydroxy-5-(1,3-benzodioxol-5-yl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(1,3-benzodioxol-5-yl)-5H-furan-2-on,
mit 4-Benzyloxybenzaldehyd
   3-(2,1,3-Benzothiadiazol-5-yl)-4-(4-benzyloxybenzyl)-5-hydroxy-5-(1,3-benzodioxol-5-yl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(1,3-benzodioxol-5-yl)-5H-fu ran-2-on,
mit 4-Nitrobenzaldehyd
   3-(2,1,3-Benzothiadiazol-5-yl)-4-(4-nitrobenzyl)-5-hydroxy-5-(1,3-benzodioxol-5-yl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(1,3-benzodioxol-5-yl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(1,3-benzodioxol-5-yl)-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-(1,3-benzodioxol-5-yl)-5H-furan-2-on,
mit Cyclohexancarbaldehyd
   3-(2,1,3-Benzothiadiazol-5-yl)-4-cyclohexylmethyl-5-hydroxy-5-(1,3-benzodioxol-5-yl)-5H-furan-2-on und
mit Cyclopentancarbaldehyd
   3-(2,1,3-Benzothiadiazol-5-yl)-4-cyclopentylmethyl-5-hydroxy-5-(1,3-benzodioxol-5-yl)-5H-furan-2-on.

Analog erhält man durch Umsetzung von 2-(2,1,3-Benzothiadiazol-5-yl)-4-(3,4,5-trimethoxyphenyl)-4-oxo-butansäureethylester
mit Benzaldehyd
   3-(2,1,3-Benzothiadiazol-5-yl)-4-benzyl-5-hydroxy-5-(3,4, 5-trimethoxyphenyl)-5H-furan-2-on,
mit 2-Methoxybenzaldehyd
   3-(2,1,3-Benzothiadiazol-5-yl)-4-(2-methoxybenzyl)-5-hydroxy-5-(3,4,5-trimethoxyphenyl)-5H-furan-2-on,
mit 3-Methoxybenzaldehyd
   3-(2,1,3-Benzothiadiazol-5-yl)-4-(3-methoxybenzyl)-5-hydroxy-5-(3,4,5-trimethoxyphenyl)-5H-furan-2-on,
mit 4-Methoxybenzaldehyd
   3-(2,1,3-Benzothiadiazol-5-yl)-4-(4-methoxybenzyl)-5-hydroxy-5-(3,4,5-trimethoxyphenyl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(3,4,5-trimethoxyphenyl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(3,4,5-trimethoxyphenyl)-5H-furan-2-on, F. 70°
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(3,4,5-trimethoxyphenyl)-5H-furan-2-on, F. 61°
mit 3,4,5-Triisopropoxybenzaldehyd
   3-(2,1,3-Benzothiadiazol-5-yl)-4-(3,4,5-triisopropoxybenzyl)-5-hydroxy-5-(3,4,5-trimethoxyphenyl)-5H-furan-2-on,
mit 4-Trifluormethylbenzaldehyd
   3-(2,1,3-Benzothiadiazol-5-yl)-4-(4-trifluormethylbenzyl)-5-hydroxy-5-(3,4, 5-trimethoxyphenyl)-5H-furan-2-on,
mit 4-Cyanbenzaldehyd
   3-(2,1,3-Benzothiadiazol-5-yl)-4-(4-cyanbenzyl)-5-hydroxy-5-(3,4,5-trimethoxyphenyl)-5H-furan-2-on,
mit 4-Methylbenzaldehyd
   3-(2,1,3-Benzothiadiazol-5-yl)-4-(4-methylbenzyl)-5-hydroxy-5-(3,4,5-trimethoxyphenyl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(3,4,5-trimethoxyphenyl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(3,4,5-trimethoxyphenyl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(3,4,5-trimethoxyphenyl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)-benzyl]-5-hydroxy-5-(3,4,5-trimethoxyphenyl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(3,4,5-trimethoxyphenyl)-5H-furan-2-on,
mit 4-Benzyloxybenzaldehyd
   3-(2,1,3-Benzothiadiazol-5-yl)-4-(4-benzyloxybenzyl)-5-hydroxy-5-(3,4,5-trimethoxyphenyl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(3,4,5-trimethoxyphenyl)-5H-furan-2-on,
mit 4-Nitrobenzaldehyd
   3-(2,1,3-Benzothiadiazol-5-yl)-4-(4-nitrobenzyl)-5-hydroxy-5-(3,4,5-trimethoxyphenyl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(3,4,5-trimethoxyphenyl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(3,4,5-trimethoxyphenyl)-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-(3,4,5-trimethoxyphenyl)-5H-furan-2-on,
mit Cyclohexancarbaldehyd
   3-(2,1,3-Benzothiadiazol-5-yl)-4-cyclohexylmethyl-5-hydroxy-5-(3,4,5-trimethoxyphenyl)-5H-furan-2-on und
mit Cyclopentancarbaldehyd
   3-(2,1,3-Benzothiadiazol-5-yl)-4-cyclopentylmethyl-5-hydroxy-5-(3,4,5-trimethoxyphenyl)-5H-furan-2-on.

Analog erhält man durch Umsetzung von 2-(2,1,3-Benzothiadiazol-5-yl)-4-(3,4-dimethoxyphenyl)-4-oxo-butansäureethylester
mit Benzaldehyd
   3-(2,1,3-Benzothiadiazol-5-yl)-4-benzyl-5-hydroxy-5-(3,4-dimethoxyphenyl)-5H-furan-2-on,
mit 2-Methoxybenzaldehyd
   3-(2,1,3-Benzothiadiazol-5-yl)-4-(2-methoxybenzyl)-5-hydroxy-5-(3,4-dimethoxyphenyl)-5H-furan-2-on,
mit 3-Methoxybenzaldehyd
   3-(2,1,3-Benzothiadiazol-5-yl)-4-(3-methoxybenzyl)-5-hydroxy-5-(3,4-dimethoxyphenyl)-5H-furan-2-on,
mit 4-Methoxybenzaldehyd
   3-(2,1,3-Benzothiadiazol-5-yl)-4-(4-methoxybenzyl)-5-hydroxy-5-(3,4-dimethoxyphenyl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(3,4-dimethoxyphenyl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(3,4-dimethoxyphenyl)-5H-furan-2-on,
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(3,4-dimethoxyphenyl)-5H-furan-2-on,
mit 3,4,5-Triisopropoxybenzaldehyd
   3-(2,1,3-Benzothiadiazol-5-yl)-4-(3,4,5-triisopropoxybenzyl)-5-hydroxy-5-(3,4-dimethoxyphenyl)-5H-furan-2-on,
mit 4-Trifluormethylbenzaldehyd
   3-(2,1,3-Benzothiadiazol-5-yl)-4-(4-trifluormethylbenzyl)-5-hydroxy-5-(3,4-dimethoxyphenyl)-5H-furan-2-on,
mit 4-Cyanbenzaldehyd
   3-(2,1,3-Benzothiadiazol-5-yl)-4-(4-cyanbenzyl)-5-hydroxy-5-(3,4-d imethoxyphenyl)-5H-furan-2-on,
mit 4-Methylbenzaldehyd
   3-(2,1,3-Benzothiadiazol-5-yl)-4-(4-methylbenzyl)-5-hydroxy-5-(3,4-dimethoxyphenyl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(3,4-dimethoxyphenyl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(3,4-dimethoxyphenyl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(3,4-dimethoxyphenyl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)-benzyl]-5-hydroxy-5-(3,4-dimethoxyphenyl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(3,4-dimethoxyphenyl)-5H-furan-2-on,
mit 4-Benzyloxybenzaldehyd
   3-(2,1,3-Benzothiadiazol-5-yl)-4-(4-benzyloxybenzyl)-5-hydroxy-5-(3,4-dimethoxyphenyl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(3,4-dimethoxyphenyl)-5H-furan-2-on,
mit 4-Nitrobenzaldehyd
   3-(2,1,3-Benzothiadiazol-5-yl)-4-(4-nitrobenzyl)-5-hydroxy-5-(3,4-dimethoxyphenyl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(3,4-dimethoxyphenyl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(3,4-dimethoxyphenyl)-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-(3,4-dimethoxyphenyl)-5H-furan-2-on,
mit Cyclohexancarbaldehyd
   3-(2,1,3-Benzothiadiazol-5-yl)-4-cyclohexylmethyl-5-hydroxy-5-(3,4-dimethoxyphenyl)-5H-furan-2-on und
mit Cyclopentancarbaldehyd
   3-(2,1,3-Benzothiadiazol-5-yl)-4-cyclopentylmethyl-5-hydroxy-5-(3,4-dimethoxyphenyl)-5H-furan-2-on.

Analog erhält man durch Umsetzung von 2-(2,1,3-Benzothiadiazol-5-yl)-4-(4-methylthiophenyl)-4-oxo-butansäureethylester
mit Benzaldehyd
   3-(2,1,3-Benzothiadiazol-5-yl)-4-benzyl-5-hydroxy-5-(4-methylthiophenyl)-5H-furan-2-on,
mit 2-Methoxybenzaldehyd
   3-(2,1,3-Benzothiadiazol-5-yl)-4-(2-methoxybenzyl)-5-hydroxy-5-(4-methylthiophenyl)-5H-furan-2-on,
mit 3-Methoxybenzaldehyd
   3-(2,1,3-Benzothiadiazol-5-yl)-4-(3-methoxybenzyl)-5-hydroxy-5-(4-methylthiophenyl)-5H-furan-2-on,
mit 4-Methoxybenzaldehyd
   3-(2,1,3-Benzothiadiazol-5-yl)-4-(4-methoxybenzyl)-5-hydroxy-5-(4-methylthiophenyl)-5H-furan-2-on,
mit 3,4-Dimethoxybenzaldehyd
   3-(2,1,3-Benzothiadiazol-5-yl)-4-(3,4-dimethoxybenzyl)-5-hydroxy-5-(4-methylthiophenyl)-5H-furan-2-on,
mit 3,4,5-Trimethoxybenzaldehyd
   3-(2,1,3-Benzothiadiazol-5-yl)-4-(3,4,5-trimethoxybenzyl)-5-hydroxy-5-(4-methylthiophenyl)-5H-furan-2-on,
mit 3,4-Diisopropoxy-5-methoxybenzaldehyd
   3-(2,1,3-Benzothiadiazol-5-yl)-4-(3,4-diisopropoxy-5-methoxybenzyl)-5-hydroxy-5-(4-methylthiophenyl)-5H-furan-2-on,
mit 3,4,5-Triisopropoxybenzaldehyd
   3-(2,1,3-Benzothiadiazol-5-yl)-4-(3,4,5-triisopropoxybenzyl)-5-hydroxy-5-(4-methylthiophenyl)-5H-furan-2-on,
mit 4-Trifluormethylbenzaldehyd
   3-(2,1,3-Benzothiadiazol-5-yl)-4-(4-trifluormethylbenzyl)-5-hydroxy-5-(4-methylthiophenyl)-5H-furan-2-on,
mit 4-Cyanbenzaldehyd
   3-(2,1,3-Benzothiadiazol-5-yl)-4-(4-cyanbenzyl)-5-hydroxy-5-(4-methylthiophenyl)-5H-furan-2-on,
mit 4-Methylbenzaldehyd
   3-(2,1,3-Benzothiadiazol-5-yl)-4-(4-methylbenzyl)-5-hydroxy-5-(4-methylthiophenyl)-5H-furan-2-on,
mit 3-Methyl-4-methoxybenzaldehyd
   3-(2,1,3-Benzothiadiazol-5-yl)-4-(3-methyl-4-methoxybenzyl)-5-hydroxy-5-(4-methylthiophenyl)-5H-furan-2-on,
mit 4-tert.-Butylbenzaldehyd
   3-(2,1,3-Benzothiadiazol-5-yl)-4-(4-tert.-butylbenzyl)-5-hydroxy-5-(4-methylthiophenyl)-5H-furan-2-on,
mit (2-Methoxycarbonylmethyloxy-4-methoxy)benzaldehyd
   3-(2,1,3-Benzothiadiazol-5-yl)-4-[(2-methoxycarbonylmethyloxy-4-methoxy)benzyl]-5-hydroxy-5-(4-methylthiophenyl)-5H-furan-2-on,
mit (2-Carboxymethyloxy-4-methoxy)benzaldehyd
   3-(2,1,3-Benzothiadiazol-5-yl)-4-[(2-carboxymethyloxy-4-methoxy)-benzyl]-5-hydroxy-5-(4-methylthiophenyl)-5H-furan-2-on,
mit 4-Ethoxycarbonylbenzaldehyd
   3-(2,1,3-Benzothiadiazol-5-yl)-4-(4-ethoxycarbonylbenzyl)-5-hydroxy-5-(4-methylthiophenyl)-5H-furan-2-on,
mit 4-Benzyloxybenzaldehyd
   3-(2,1,3-Benzothiadiazol-5-yl)-4-(4-benzyloxybenzyl)-5-hydroxy-5-(4-methylthiophenyl)-5H-furan-2-on,
mit 4-Dimethylaminobenzaldehyd
   3-(2,1,3-Benzothiadiazol-5-yl)-4-(4-dimethylaminobenzyl)-5-hydroxy-5-(4-methylthiophenyl)-5H-furan-2-on,
mit 4-Nitrobenzaldehyd
   3-(2,1,3-Benzothiadiazol-5-yl)-4-(4-nitrobenzyl)-5-hydroxy-5-(4-methylthiophenyl)-5H-furan-2-on,
mit (1,3-Benzodioxol-5-yl)-carbaldehyd
   3-(2,1,3-Benzothiadiazol-5-yl)-4-[(1,3-benzodioxol-5-yl)-methyl]-5-hydroxy-5-(4-methylthiophenyl)-5H-furan-2-on,
mit 3-Fluor-4-methoxybenzaldehyd
   3-(2,1,3-Benzothiadiazol-5-yl)-4-(3-fluor-4-methoxybenzyl)-5-hydroxy-5-(4-methylthiophenyl)-5H-furan-2-on,
mit 3-(N,N-Dimethylamino)-benzaldehyd
   3-(2,1,3-Benzothiadiazol-5-yl)-4-[3-(N,N-dimethylamino)benzyl]-5-hydroxy-5-(4-methylthiophenyl)-5H-furan-2-on,
mit Cyclohexancarbaldehyd
   3-(2,1,3-Benzothiadiazol-5-yl)-4-cyclohexylmethyl-5-hydroxy-5-(4-methylthiophenyl)-5H-furan-2-on und
mit Cyclopentancarbaldehyd
   3-(2,1,3-Benzothiadiazol-5-yl)-4-cyclopentylmethyl-5-hydroxy-5-(4-methylthiophenyl)-5H-furan-2-on.

Analog erhält man durch Umsetzung von 2-(2,1,3-Benzoxadiazol-5-yl)-4-(4-methoxyphenyl)-4-oxo-butansäureethylester
mit Benzaldehyd
   3-(2,1,3-Benzoxadiazol-5-yl)-4-benzyl-5-hydroxy-5-(4-methoxyphenyl)-5H-furan-2-on,
mit 2-Methoxybenzaldehyd
   3-(2,1,3-Benzoxadiazol-5-yl)-4-(2-methoxybenzyl)-5-hydroxy-5-(4-methoxyphenyl)-5H-furan-2-on,
mit 3-Methoxybenzaldehyd
   3-(2,1,3-Benzoxadiazol-5-yl)-4-(3-methoxybenzyl)-5-hydroxy-5-(4-methoxyphenyl)-5H-furan-2-on,
mit 4-Methoxybenzaldehyd
   3-(2,1,3-Benzoxadiazol-5-yl)-4-(4-methoxybenzyl)-5-hydroxy-5-(4-methoxyphenyl)-5H-furan-2-on,
mit 3,4-Dimethoxybenzaldehyd
   3-(2,1,3-Benzoxadiazol-5-yl)-4-(3,4-dimethoxybenzyl)-5-hydroxy-5-(4-methoxyphenyl)-5H-furan-2-on,
mit 3,4,5-Trimethoxybenzaldehyd
   3-(2,1,3-Benzoxadiazol-5-yl)-4-(3,4,5-trimethoxybenzyl)-5-hydroxy-5-(4-methoxyphenyl)-5H-furan-2-on,
mit 3,4-Diisopropoxy-5-methoxybenzaldehyd
   3-(2,1,3-Benzoxadiazol-5-yl)-4-(3,4-diisopropoxy-5-methoxybenzyl)-5-hydroxy-5-(4-methoxyphenyl)-5H-furan-2-on,
mit 3,4,5-Triisopropoxybenzaldehyd
   3-(2,1,3-Benzoxadiazol-5-yl)-4-(3,4,5-triisopropoxybenzyl)-5-hydroxy-5-(4-methoxyphenyl)-5H-furan-2-on,
mit 4-Trifluormethylbenzaldehyd
   3-(2,1,3-Benzoxadiazol-5-yl)-4-(4-trifluormethylbenzyl)-5- hydroxy-5-(4-methoxyphenyl)-5H-furan-2-on,
mit 4-Cyanbenzaldehyd
   3-(2,1,3-Benzoxadiazol-5-yl)-4-(4-cyanbenzyl)-5-hydroxy-5-(4-methoxyphenyl)-5H-furan-2-on,
mit 4-Methylbenzaldehyd
   3-(2,1,3-Benzoxadiazol-5-yl)-4-(4-methylbenzyl)-5-hydroxy-5-(4-methoxyphenyl)-5H-furan-2-on,
mit 3-Methyl-4-methoxybenzaldehyd
   3-(2,1,3-Benzoxadiazol-5-yl)-4-(3-methyl-4-methoxybenzyl)-5-hydroxy-5-(4-methoxyphenyl)-5H-furan-2-on,
mit 4-tert-Butylbenzaldehyd
   3-(2,1,3-Benzoxadiazol-5-yl)-4-(4-tert.-butylbenzyl)-5-hydroxy-5-(4-methoxyphenyl)-5H-furan-2-on,
mit (2-Methoxycarbonylmethyloxy-4-methoxy)benzaldehyd
   3-(2,1,3-Benzoxadiazol-5-yl)-4-[(2-methoxycarbonylmethyloxy-4-methoxy)benzyl]-5-hydroxy-5-(4-methoxyphenyl)-5H-furan-2-on,
mit (2-Carboxymethyloxy-4-methoxy)benzaldehyd
   3-(2,1,3-Benzoxadiazol-5-yl)-4-[(2-carboxymethyloxy-4-methoxy)benzyl]-5-hydroxy-5-(4-methoxyphenyl)-5H-furan-2-on,
mit 4-Ethoxycarbonylbenzaldehyd
   3-(2,1,3-Benzoxadiazol-5-yl)-4-(4-ethoxycarbonylbenzyl)-5-hydroxy-5-(4-methoxyphenyl)-5H-furan-2-on,
mit 4-Benzyloxybenzaldehyd
   3-(2,1,3-Benzoxadiazol-5-yl)-4-(4-benzyloxybenzyl)-5-hydroxy-5-(4-methoxyphenyl)-5H-furan-2-on,
mit 4-Dimethylaminobenzaldehyd
   3-(2,1,3-Benzoxadiazol-5-yl)-4-(4-dimethylaminobenzyl)-5-hydroxy-5-(4-methoxyphenyl)-5H-furan-2-on,
mit 4-Nitrobenzaldehyd
   3-(2,1,3-Benzoxadiazol-5-yl)-4-(4-nitrobenzyl)-5-hydroxy-5-(4-methoxyphenyl)-5H-furan-2-on,
mit (1,3-Benzodioxol-5-yl)-carbaldehyd
   3-(2,1,3-Benzoxadiazol-5-yl)-4-[(1,3-benzodioxol-5-yl)-methyl]-5-hydroxy-5-(4-methoxyphenyl)-5H-furan-2-on,
mit 3-Fluor-4-methoxybenzaldehyd
   3-(2,1,3-Benzoxadiazol-5-yl)-4-(3-fluor-4-methoxybenzyl)-5-hydroxy-5-(4-methoxyphenyl)-5H-furan-2-on,
mit 3-(N,N-Dimethylamino)-benzaldehyd
   3-(2,1,3-Benzoxadiazol-5-yl)-4-[3-(N,N-dimethylamino)benzyl]-5-hydroxy-5-(4-methoxyphenyl)-5H-furan-2-on,
mit Cyclohexancarbaldehyd
   3-(2,1,3-Benzoxadiazol-5-yl)-4-cyclohexylmethyl-5-hydroxy-5-(4-methoxyphenyl)-5H-furan-2-on und
mit Cyclopentancarbaldehyd
   3-(2,1,3-Benzoxadiazol-5-yl)-4-cyclopentylmethyl-5-hydroxy-5-(4-methoxyphenyl)-5H-furan-2-on.

### Beispiel 2

Zu einer Suspension von 30 mg 3-(2,1,3-Benzothiadiazol-5-yl)-4-benzyl-5-hydroxy-5-(4-methoxyphenyl)-5H-furan-2-on in 1 ml Methanol gibt man 0,7 ml 0,1 N NaOH und rührt bei Raumtemperatur. Das Lösungsmittel wird entfernt, der Rückstand zwischen Wasser und Diethylether verteilt, und die wässrige Phase wird anschließend lyophilisiert Man erhält 2-(2,1,3-Benzothiadiazol-5-yl)-3-benzyl-4-(4-methoxyphenyl)-4-oxo-2-butensäure Natriumsalz, FAB 453.

Analog erhält man durch Behandlung der in Beispiel 1 genannten Furanderivate mit NaOH die Natriumsalze der entsprechenden offenkettigen 4-Oxo-2-butensäurederivate:
2-(2,1,3-Benzothiadiazol-5-yl)-3-(2-methoxybenzyl)-4-(4-methoxyphenyl)-4-oxo-2-butensäure Natriumsalz,
2-(2,1,3-Benzothiadiazol-5-yl)-3-(3-methoxybenzyl)-4-(4-methoxyphenyl)-4-oxo-2-butensäure Natriumsalz,
2-(2,1,3-Benzothiadiazol-5-yl)-3-(4-methoxybenzyl)-4-(4-methoxyphenyl)-4-oxo-2-butensäure Natriumsalz,
2-(2,1,3-Benzothiadiazol-5-yl)-3-(3,4-dimethoxybenzyl)-4-(4-methoxyphenyl)-4-oxo-2-butensäure Natriumsalz,
2-(2,1,3-Benzothiadiazol-5-yl)-3-(3,4,5-trimethoxybenzyl)-4-(4-methoxyphenyl)-4-oxo-2-butensäure Natriumsalz, F. 245° (Zers.)
2-(2,1,3-Benzothiadiazol-5-yl)-3-(3,4-diisopropoxy-5-methoxybenzyl)-4-(4-methoxyphenyl)-4-oxo-2-butensäure Natriumsalz, FAB 599
2-(2,1,3-Benzothiadiazol-5-yl)-3-(3,4,5-triisopropoxybenzyl)-4-(4-methoxyphenyl)-4-oxo-2-butensäure Natriumsalz,
2-(2,1,3-Benzothiadiazol-5-yl)-3-(4-trifluormethylbenzyl)-4-(4-methoxyphenyl)-4-oxo-2-butensäure Natriumsalz,
2-(2,1,3-Benzothiadiazol-5-yl)-3-(4-cyanbenzyl)-4-(4-methoxyphenyl)-4-oxo-2-butensäure Natriumsalz,
2-(2,1,3-Benzothiadiazol-5-yl)-3-(4-methylbenzyl)-4-(4-methoxyphenyl)-4-oxo-2-butensäure Natriumsalz,
2-(2,1,3-Benzothiadiazol-5-yl)-3-(3-methyl-4-methoxybenzyl)-4-(4-methoxyphenyl)-4-oxo-2-butensäure Natriumsalz,
2-(2,1,3-Benzothiadiazol-5-yl)-3-(4-tert-butylbenzyl)-4-(4-methoxyphenyl)-4-oxo-2-butensäure Natriumsalz,
2-(2,1,3-Benzothiadiazol-5-yl)-3-[(2-methoxycarbonylmethyloxy-4-methoxy)benzyl]-4-(4-methoxyphenyl)-4-oxo-2-butensäure Natriumsalz,
2-(2,1,3-Benzothiadiazol-5-yl)-3-[(2-carboxymethyloxy-4-methoxy)benzyl]-4-(4-methoxyphenyl)-4-oxo-2-butensäure Natriumsalz,
2-(2,1,3-Benzothiadiazol-5-yl)-3-(4-ethoxycarbonylbenzyl)-4-(4-methoxyphenyl)-4-oxo-2-butensäure Natriumsalz,
2-(2,1,3-Benzothiadiazol-5-yl)-3-(4-benzyloxybenzyl)-4-(4-methoxyphenyl)-4-oxo-2-butensäure Natriumsalz,
2-(2,1,3-Benzothiadiazol-5-yl)-3-(4-dimethylaminobenzyl)-4-(4-methoxyphenyl)-4-oxo-2-butensäure Natriumsalz,
2-(2,1,3-Benzothiadiazol-5-yl)-3-(4-nitrobenzyl)-4-(4-methoxyphenyl)-4-oxo-2-butensäure Natriumsalz,
2-(2,1,3-Benzothiadiazol-5-yl)-3-[(1,3-benzodioxol-5-yl)-methyl]-4-(4-methoxyphenyl)-4-oxo-2-butensäure Natriumsalz,
2-(2,1,3-Benzothiadiazol-5-yl)-3-(3-fluor-4-methoxybenzyl)-4-(4-methoxyphenyl)-4-oxo-2-butensäure Natriumsalz,
2-(2,1,3-Benzothiadiazol-5-yl)-3-[3-(N,N-dimethylamino)benzyl]-4-(4-methoxyphenyl)-4-oxo-2-butensäure Natriumsalz,
2-(2,1,3-Benzothiadiazol-5-yl)-3-cyclohexylmethyl-4-(4-methoxyphenyl)-4-oxo-2-butensäure Natriumsalz,
2-(2,1,3-Benzothiadiazol-5-yl)-3-cyclopentylmethyl-4-(4-methoxyphenyl)-4-oxo-2-butensäure Natriumsalz,
2-(2,1,3-Benzothiadiazol-5-yl)-3-benzyl-4-(1,4-benzodioxan-6-yl)-4-oxo-2-butensäure Natriumsalz, FAB 481
2-(2,1,3-Benzothiadiazol-5-yl)-3-(2-methoxybenzyl)-4-(1,4-benzodioxan-6-yl)-4-oxo-2-butensäure Natriumsalz,
2-(2,1,3-Benzothiadiazol-5-yl)-3-(3-methoxybenzyl)-4-(1,4-benzodioxan-6-yl)-4-oxo-2-butensäure Natriumsalz,
2-(2,1,3-Benzothiadiazol-5-yl)-3-(4-methoxybenzyl)-4-(1,4-benzodioxan-6-yl)-4-oxo-2-butensäure Natriumsalz,
2-(2,1,3-Benzothiadiazol-5-yl)-3-(3,4-dimethoxybenzyl)-4-(1,4-benzodioxan-6-yl)-4-oxo-2-butensäure Natriumsalz,
2-(2,1,3-Benzothiadiazol-5-yl)-3-(3,4,5-trimethoxybenzyl)-4-(1,4-benzodioxan-6-yl)-4-oxo-2-butensäure Natriumsalz, F. 76°
2-(2,1,3-Benzothiadiazol-5-yi)-3-(3,4-diisopropoxy-5-methoxybenzyl)-4-(1,4-benzodioxan-6-yl)-4-oxo-2-butensäure Natriumsalz, F. 70°
2-(2,1,3-Benzothiadiazol-5-yl)-3-(3,4,5-triisopropoxybenzyl)-4-(1,4-benzodioxan-6-yl)-4-oxo-2-butensäure Natriumsalz,
2-(2,1,3-Benzothiadiazol-5-yl)-3-(4-trifluormethylbenzyl)-4-(1,4-benzodioxan-6-yl)-4-oxo-2-butensäure Natriumsalz,
2-(2,1,3-Benzothiadiazol-5-yl)-3-(4-cyanbenzyl)-4-(1,4-benzodioxan-6-yl)-4-oxo-2-butensäure Natriumsalz,
2-(2,1,3-Benzothiadiazol-5-yl)-3-(4-methylbenzyl)-4-(1,4-benzodioxan-6-yl)-4-oxo-2-butensäure Natriumsalz,
2-(2,1,3-Benzothiadiazol-5-yl)-3-(3-methyl-4-methoxybenzyl)-4-(1,4-benzodioxan-6-yl)-4-oxo-2-butensäure Natriumsalz,
2-(2,1,3-Benzothiadiazol-5-yl)-3-(4-tert. -butylbenzyl)-4-(1,4-benzodioxan-6-yl)-4-oxo-2-butensäure Natriumsalz,
2-(2,1,3-Benzothiadiazol-5-yl)-3-[(2-methoxycarbonylmethyloxy-4-methoxy)benzyl]-4-(1,4-benzodioxan-6-yl)-4-oxo-2-butensäure Natriumsalz,
2-(2,1,3-Benzothiadiazol-5-yl)-3-[(2-carboxymethyloxy-4-methoxy)benzyl]-4-(1,4-benzodioxan-6-yl)-4-oxo-2-butensäure Natriumsalz,
2-(2,1,3-Benzothiadiazol-5-yl)-3-(4-ethoxycarbonylbenzyl)-4-(1,4-benzodioxan-6-yl)-4-oxo-2-butensäure Natriumsalz,
2-(2,1,3-Benzothiadiazol-5-yl)-3-(4-benzyloxybenzyl)-4-(1,4-benzodioxan-6-yl)-4-oxo-2-butensäure Natriumsalz,
2-(2,1,3-Benzothiadiazol-5-yl)-3-(4-dimethylaminobenzyl)-5-hydroxy-5-(1,4-benzodioxan-6-yl)-4-oxo-2-butensäure Natriumsalz,
2-(2,1,3-Benzothiadiazol-5-yl)-3-(4-nitrobenzyl)-4-(1,4-benzodioxan-6-yl)-4-oxo-2-butensäure Natriumsalz,
2-(2,1,3-Benzothiadiazol-5-yl)-3-[(1,3-benzodioxol-5-yl)-methyl]-4-(1,4-benzodioxan-6-yl)-4-oxo-2-butensäure Natriumsalz,
2-(2,1,3-Benzothiadiazol-5-yl)-3-(3-fluor-4-methoxybenzyl)- 4-(1,4-benzodioxan-6-yl)-4-oxo-2-butensäure Natriumsalz,
2-(2,1,3-Benzothiadiazol-5-yl)-3-[3-(N,N-dimethylamino)benzyl]-4-(1,4-benzodioxan-6-yl)-4-oxo-2-butensäure Natriumsalz,
2-(2,1,3-Benzothiadiazol-5-yl)-3-(cyclohexylmethyl)-4-(1,4-benzodioxan-6-yl)-4-oxo-2-butensäure Natriumsalz,
2-(2,1,3-Benzothiadiazol-5-yl)-3-(cyclopentylmethyl)-4-(1,4-benzodioxan-6-yl)-4-oxo-2-butensäure Natriumsalz,
2-(2,1,3-Benzothiadiazol-5-yl)-3-benzyl-4-(4-methylphenyl)-4-oxo-2-butensäure Natriumsalz,
2-(2,1,3-Benzothiadiazol-5-yl)-3-(2-methoxybenzyl)-4-(4-methylphenyl)-4-oxo-2-butensäure Natriumsalz,
2-(2,1,3-Benzothiadiazol-5-yl)-3-(3-methoxybenzyl)-4-(4-methylphenyl)-4-oxo-2-butensäure Natriumsalz,
2-(2,1,3-Benzothiadiazol-5-yl)-3-(4-methoxybenzyl)-4-(4-methylphenyl)-4-oxo-2-butensäure Natriumsalz,
2-(2,1,3-Benzothiadiazol-5-yl)-3-(3,4-dimethoxybenzyl)-4-(4-methylphenyl)-4-oxo-2-butensäure Natriumsalz,
2-(2,1,3-Benzothiadiazol-5-yl)-3-(3,4,5-trimethoxybenzyl)-4-(4-methylphenyl)-4-oxo-2-butensäure Natriumsalz,
2-(2,1,3-Benzothiadiazol-5-yl)-3-(3,4-diisopropoxy-5-methoxybenzyl)-4-(4-methylphenyl)-4-oxo-2-butensäure Natriumsalz,
2-(2,1,3-Benzothiadiazol-5-yl)-3-(3,4,5-triisopropoxybenzyl)-4-(4-methylphenyl)-4-oxo-2-butensäure Natriumsalz,
2-(2,1,3-Benzothiadiazol-5-yl)-3-(4-trifluormethylbenzyl)-5-hydroxy-5-(4-methylphenyl)-4-oxo-2-butensäure Natriumsalz,
2-(2,1,3-Benzothiadiazol-5-yl)-3-(4-cyanbenzyl)-4-(4-methylphenyl)-4-oxo-2-butensäure Natriumsalz,
2-(2,1,3-Benzothiadiazol-5-yl)-3-(4-methylbenzyl)-4-(4-methylphenyl)-4-oxo-2-butensäure Natriumsalz,
2-(2,1,3-Benzothiadiazol-5-yl)-3-(3-methyl-4-methoxybenzyl)-4-(4-methylphenyl)-4-oxo-2-butensäure Natriumsalz,
2-(2,1,3-Benzothiadiazol-5-yl)-3-(4-tert.-butylbenzyl)-4-(4-methylphenyl)-4-oxo-2-butensäure Natriumsalz,
2-(2,1,3-Benzothiadiazol-5-yl)-3-[(2-methoxycarbonylmethyloxy-4-methoxy)benzyl]-4-(4-methylphenyl)-4-oxo-2-butensäure Natriumsalz,
2-(2,1,3-Benzothiadiazol-5-yl)-3-[(2-carboxymethyloxy-4-methoxy)benzyl]-4-(4-methylphenyl)-4-oxo-2-butensäure Natriumsalz,
2-(2,1,3-Benzothiadiazol-5-yl)-3-(4-ethoxycarbonylbenzyl)-4-(4-methylphenyl)-4-oxo-2-butensäure Natriumsalz,
2-(2,1,3-Benzothiadiazol-5-yl)-3-(4-benzyloxybenzyl)-4-(4-methylphenyl)-4-oxo-2-butensäure Natriumsalz,
2-(2,1,3-Benzothiadiazol-5-yl)-3-(4-dimethylaminobenzyl)-4-(4-methylphenyl)-4-oxo-2-butensäure Natriumsalz,
2-(2,1,3-Benzothiadiazol-5-yl)-3-(4-nitrobenzyl)-4-(4-methylphenyl)-4-oxo-2-butensäure Natriumsalz,
2-(2,1,3-Benzothiadiazol-5-yl)-3-[(1,3-benzodioxol-5-yl)-methyl]-4-(4-methylphenyl)-4-oxo-2-butensäure Natriumsalz,
2-(2,1,3-Benzothiadiazol-5-yl)-3-(3-fluor-4-methoxybenzyl)- 4-(4-methylphenyl)-4-oxo-2-butensäure Natriumsalz,
2-(2,1,3-Benzothiadiazol-5-yl)-3-[3-(N,N-dimethylamino)benzyl]-4-(4-methylphenyl)-4-oxo-2-butensäure Natriumsalz,
2-(2,1,3-Benzothiadiazol-5-yl)-3-(cyclohexylmethyl)-4-(4-methylphenyl)-4-oxo-2-butensäure Natriumsalz und
2-(2,1,3-Benzothiadiazol-5-yl)-3-(cyclopentylmethyl)-4-(4-methylphenyl)-4-oxo-2-butensäure Natriumsalz.
2-(2,1,3-Benzothiadiazol-5-yl)-3-benzyl-4-phenyl-4-oxo-2-butensäure Natriumsalz,
2-(2,1,3-Benzothiadiazol-5-yl)-3-(2-methoxybenzyl)-4-phenyl-4-oxo-2-butensäure Natriumsalz,
2-(2,1,3-Benzothiadiazol-5-yl)-3-(3-methoxybenzyl)-4-phenyl-4-oxo-2-butensäure Natriumsalz,
2-(2,1,3-Benzothiadiazol-5-yl)-3-(4-methoxybenzyl)-4-phenyl-4-oxo-2-butensäure Natriumsalz,
2-(2,1,3-Benzothiadiazol-5-yl)-3-(3,4-dimethoxybenzyl)-4-phenyl-4-oxo-2-butensäure Natriumsalz,
2-(2,1,3-Benzothiadiazol-5-yl)-3-(3,4,5-trimethoxybenzyl)-4-phenyl-4-oxo-2-butensäure Natriumsalz,
2-(2,1,3-Benzothiadiazol-5-yl)-3-(3,4-diisopropoxy-5-methoxybenzyl)-4-phenyl-4-oxo-2-butensäure Natriumsalz,
2-(2,1,3-Benzothiadiazol-5-yl)-3-(3,4,5-triisopropoxybenzyl)-4-phenyl-4-oxo-2-butensäure Natriumsalz,
2-(2,1,3-Benzothiadiazol-5-yl)-3-(4-trifluormethylbenzyl)-5-hydroxy-5-phenyl-4-oxo-2-butensäure Natriumsalz,
2-(2,1,3-Benzothiadiazol-5-yl)-3-(4-cyanbenzyl)-4-phenyl-4-oxo-2-butensäure Natriumsalz,
2-(2,1,3-Benzothiadiazol-5-yl)-3-(4-methylbenzyl)-4-phenyl-4-oxo-2-butensäure Natriumsalz,
2-(2,1,3-Benzothiadiazol-5-yl)-3-(3-methyl-4-methoxybenzyl)-4-phenyl-4-oxo-2-butensäure Natriumsalz,
2-(2,1,3-Benzothiadiazol-5-yl)-3-(4-tert.-butylbenzyl)-4-phenyl-4-oxo-2-butensäure Natriumsalz,
2-(2,1,3-Benzothiadiazol-5-yl)-3-[(2-methoxycarbonylmethyloxy-4-methoxy)benzyl]-4-phenyl-4-oxo-2-butensäure Natriumsalz,
2-(2,1,3-Benzothiadiazol-5-yl)-3-[(2-carboxymethyloxy-4-methoxy)benzyl]-4-phenyl-4-oxo-2-butensäure Natriumsalz,
2-(2,1,3-Benzothiadiazol-5-yl)-3-(4-ethoxycarbonylbenzyl)-4-phenyl-4-oxo-2-butensäure Natriumsalz,
2-(2,1,3-Benzothiadiazol-5-yl)-3-(4-benzyloxybenzyl)-4-phenyl-4-oxo-2-butensäure Natriumsalz,
2-(2,1,3-Benzothiadiazol-5-yl)-3-(4-dimethylaminobenzyl)-4-phenyl-4-oxo-2-butensäure Natriumsalz,
2-(2,1,3-Benzothiadiazol-5-yl)-3-(4-nitrobenzyl)-4-phenyl-4-oxo-2-butensäure Natriumsalz,
2-(2,1,3-Benzothiadiazol-5-yl)-3-[(1,3-benzodioxol-5-yl)-methyl]-4-phenyl-4-oxo-2-butensäure Natriumsalz,
2-(2,1,3-Benzothiadiazol-5-yl)-3-(3-fluor-4-methoxybenzyl)- 4-phenyl-4-oxo-2-butensäure Natriumsalz,
2-(2,1,3-Benzothiadiazol-5-yl)-3-[3-(N,N-dimethylamino)benzyl]-4-phenyl-4-oxo-2-butensäure Natriumsalz,
2-(2,1,3-Benzothiadiazol-5-yl)-3-(cyclohexylmethyl)-4-phenyl-4-oxo-2-butensäure Natriumsalz und
2-(2,1,3-Benzothiadiazol-5-yl)-3-(cyclopentylmethyl)-4-phenyl-4-oxo-2-butensäure Natriumsalz.
2-(2,1,3-Benzothiadiazol-5-yl)-3-benzyl-4-(4-isopropoxyphenyl)-4-oxo-2-butensäure Natriumsalz,
2-(2,1,3-Benzothiadiazol-5-yl)-3-(2-methoxybenzyl)-4-(4-isopropoxyphenyl)-4-oxo-2-butensäure Natriumsalz,
2-(2,1,3-Benzothiadiazol-5-yl)-3-(3-methoxybenzyl)-4-(4-isopropoxyphenyl)-4-oxo-2-butensäure Natriumsalz,
2-(2,1,3-Benzothiadiazol-5-yl)-3-(4-methoxybenzyl)-4-(4-isopropoxyphenyl)-4-oxo-2-butensäure Natriumsalz,
2-(2,1,3-Benzothiadiazol-5-yl)-3-(3,4-dimethoxybenzyl)-4-(4-isopropoxyphenyl)-4-oxo-2-butensäure Natriumsalz,
2-(2,1,3-Benzothiadiazol-5-yl)-3-(3,4,5-trimethoxybenzyl)-4-(4-isopropoxyphenyl)-4-oxo-2-butensäure Natriumsalz,
2-(2,1,3-Benzothiadiazol-5-yl)-3-(3,4-diisopropoxy-5-methoxybenzyl)-4-(4-isopropoxyphenyl)-4-oxo-2-butensäure Natriumsalz,
2-(2,1,3-Benzothiadiazol-5-yl)-3-(3,4,5-triisopropoxybenzyl)-4-(4-isopropoxyphenyl)-4-oxo-2-butensäure Natriumsalz,
2-(2,1,3-Benzothiadiazol-5-yl)-3-(4-trifluormethylbenzyl)-5-hydroxy-5-(4-isopropoxyphenyl)-4-oxo-2-butensäure Natriumsalz,
2-(2,1,3-Benzothiadiazol-5-yl)-3-(4-cyanbenzyl)-4-(4-isopropoxyphenyl)-4-oxo-2-butensäure Natriumsalz,
2-(2,1,3-Benzothiadiazol-5-yl)-3-(4-methylbenzyl)-4-(4-isopropoxyphenyl)-4-oxo-2-butensäure Natriumsalz,
2-(2,1,3-Benzothiadiazol-5-yl)-3-(3-methyl-4-methoxybenzyl)-4-(4-isopropoxyphenyl)-4-oxo-2-butensäure Natriumsalz,
2-(2,1,3-Benzothiadiazol-5-yl)-3-(4-tert.-butylbenzyl)-4-(4-isopropoxyphenyl)-4-oxo-2-butensäure Natriumsalz,
2-(2,1,3-Benzothiadiazol-5-yl)-3-[(2-methoxycarbonylmethyloxy-4-methoxy)benzyl]-4-(4-isopropoxyphenyl)-4-oxo-2-butensäure Natriumsalz,
2-(2,1,3-Benzothiadiazol-5-yl)-3-[(2-carboxymethyloxy-4-methoxy)-benzyl]-4-(4-isopropoxyphenyl)-4-oxo-2-butensäure Natriumsalz,
2-(2,1,3-Benzothiadiazol-5-yl)-3-(4-ethoxycarbonylbenzyl)-4-(4-isopropoxyphenyl)-4-oxo-2-butensäure Natriumsalz,
2-(2,1,3-Benzothiadiazol-5-yl)-3-(4-benzyloxybenzyl)-4-(4-isopropoxyphenyl)-4-oxo-2-butensäure Natriumsalz,
2-(2,1,3-Benzothiadiazol-5-yl)-3-(4-dimethylaminobenzyl)-4-(4-isopropoxyphenyl)-4-oxo-2-butensäure Natriumsalz,
2-(2,1,3-Benzothiad iazol-5-yl)-3-(4-nitrobenzyl)-4-(4-isopropoxyphenyl)-4-oxo-2-butensäure Natriumsalz,
2-(2,1,3-Benzothiadiazol-5-yl)-3-[(1,3-benzodioxol-5-yl)-methyl]-4-(4-isopropoxyphenyl)-4-oxo-2-butensäure Natriumsalz,
2-(2,1,3-Benzothiadiazol-5-yl)-3-(3-fluor-4-methoxybenzyl)- 4-(4-isopropoxyphenyl)-4-oxo-2-butensäure Natriumsalz,
2-(2,1,3-Benzothiadiazol-5-yl)-3-[3-(N,N-dimethylamino)benzyl]-4-(4-isopropoxyphenyl)-4-oxo-2-butensäure Natriumsalz,
2-(2,1,3-Benzothiadiazol-5-yl)-3-(cyclohexylmethyl)-4-(4-isopropoxyphenyl)-4-oxo-2-butensäure Natriumsalz und
2-(2,1,3-Benzothiadiazol-5-yl)-3-(cyclopentylmethyl)-5-hydroxy-(4-isopropoxyphenyl)-4-oxo-2-butensäure Natriumsalz.
2-(2,1,3-Benzothiadiazol-5-yl)-3-benzyl-4-(4-benzyloxyphenyl)-4-oxo-2-butensäure Natriumsalz,
2-(2,1,3-Benzothiadiazol-5-yl)-3-(2-methoxybenzyl)-4-(4-benzyloxyphenyl)-4-oxo-2-butensäure Natriumsalz,
2-(2,1,3-Benzothiadiazol-5-yl)-3-(3-methoxybenzyl)-4-(4-benzyloxyphenyl)-4-oxo-2-butensäure Natriumsalz,
2-(2,1,3-Benzothiadiazol-5-yl)-3-(4-methoxybenzyl)-4-(4-benzyloxyphenyl)-4-oxo-2-butensäure Natriumsalz,
2-(2,1,3-Benzothiadiazol-5-yl)-3-(3,4-dimethoxybenzyl)-4-(4-benzyloxyphenyl)-4-oxo-2-butensäure Natriumsalz,
2-(2,1,3-Benzothiadiazol-5-yl)-3-(3,4,5-trimethoxybenzyl)-4-(4-benzyloxyphenyl)-4-oxo-2-butensäure Natriumsalz,
2-(2,1,3-Benzothiadiazol-5-yl)-3-(3,4-diisopropoxy-5-methoxybenzyl)-4-(4-benzyloxyphenyl)-4-oxo-2-butensäure Natriumsalz,
2-(2,1,3-Benzothiadiazol-5-yl)-3-(3,4,5-triisopropoxybenzyl)-4-(4-benzyloxyphenyl)-4-oxo-2-butensäure Natriumsalz,
2-(2,1,3-Benzothiadiazol-5-yl)-3-(4-trifluormethylbenzyl)-5-hydroxy-5-(4-benzyloxyphenyl)-4-oxo-2-butensäure Natriumsalz,
2-(2,1,3-Benzothiad iazol-5-yl)-3-(4-cyanbenzyl)-4-(4-benzyloxyphenyl)-4-oxo-2-butensäure Natriumsalz,
2-(2,1,3-Benzothiadiazol-5-yl)-3-(4-methylbenzyl)-4-(4-benzyloxyphenyl)-4-oxo-2-butensäure Natriumsalz,
2-(2,1,3-Benzothiadiazol-5-yl)-3-(3-methyl-4-methoxybenzyl)-4-(4-benzyloxyphenyl)-4-oxo-2-butensäure Natriumsalz,
2-(2,1,3-Benzothiadiazol-5-yl)-3-(4-tert.-butylbenzyl)-4-(4-benzyloxyphenyl)-4-oxo-2-butensäure Natriumsalz,
2-(2,1,3-Benzothiadiazol-5-yl)-3-[(2-methoxycarbonylmethyloxy-4-methoxy)benzyl]-4-(4-benzyloxyphenyl)-4-oxo-2-butensäure Natriumsalz,
2-(2,1,3-Benzothiadiazol-5-yl)-3-[(2-carboxymethyloxy-4-methoxy)-benzyl]-4-(4-benzyloxyphenyl)-4-oxo-2-butensäure Natriumsalz,
2-(2,1,3-Benzothiadiazol-5-yl)-3-(4-ethoxycarbonylbenzyl)-4-(4-benzyloxyphenyl)-4-oxo-2-butensäure Natriumsalz,
2-(2,1,3-Benzothiadiazol-5-yl)-3-(4-benzyloxybenzyl)-4-(4-benzyloxyphenyl)-4-oxo-2-butensäure Natriumsalz,
2-(2,1,3-Benzothiadiazol-5-yl)-3-(4-dimethylaminobenzyl)-4-(4-benzyloxyphenyl)-4-oxo-2-butensäure Natriumsalz,
2-(2,1,3-Benzothiadiazol-5-yl)-3-(4-nitrobenzyl)-4-(4-benzyloxyphenyl)-4-oxo-2-butensäure Natriumsalz,
2-(2,1,3-Benzothiadiazol-5-yl)-3-[(1,3-benzodioxol-5-yl)-methyl]-4-(4-benzyloxyphenyl)-4-oxo-2-butensäure Natriumsalz,
2-(2,1,3-Benzothiadiazol-5-yl)-3-(3-fluor-4-methoxybenzyl)- 4-(4-benzyloxyphenyl)-4-oxo-2-butensäure Natriumsalz,
2-(2,1,3-Benzothiadiazol-5-yl)-3-[3-(N,N-dimethylamino)benzyl]-4-(4-benzyloxyphenyl)-4-oxo-2-butensäure Natriumsalz,
2-(2,1,3-Benzothiadiazol-5-yl)-3-(cyclohexylmethyl)-4-(4-benzyloxyphenyl)-4-oxo-2-butensäure Natriumsalz und
2-(2,1,3-Benzothiadiazol-5-yl)-3-(cyclopentylmethyl)-5-hydroxy-(4-benzyloxyphenyl)-4-oxo-2-butensäure Natriumsalz.
2-(2,1,3-Benzothiadiazol-5-yl)-3-benzyl-4-(3,4-dimethylphenyl)-4-oxo-2-butensäure Natriumsalz,
2-(2,1,3-Benzothiadiazol-5-yl)-3-(2-methoxybenzyl)-4-(3,4-dimethylphenyl)-4-oxo-2-butensäure Natriumsalz,
2-(2,1,3-Benzothiadiazol-5-yl)-3-(3-methoxybenzyl)-4-(3,4-dimethylphenyl)-4-oxo-2-butensäure Natriumsalz,
2-(2,1,3-Benzothiadiazol-5-yl)-3-(4-methoxybenzyl)-4-(3,4-dimethylphenyl)-4-oxo-2-butensäure Natriumsalz,
2-(2,1,3-Benzothiadiazol-5-yl)-3-(3,4-dimethoxybenzyl)-4-(3,4-dimethylphenyl)-4-oxo-2-butensäure Natriumsalz,
2-(2,1,3-Benzothiadiazol-5-yl)-3-(3,4,5-trimethoxybenzyl)-4-(3,4-dimethylphenyl)-4-oxo-2-butensäure Natriumsalz,
2-(2,1,3-Benzothiadiazol-5-yl)-3-(3,4-diisopropoxy-5-methoxybenzyl)-4-(3,4-dimethylphenyl)-4-oxo-2-butensäure Natriumsalz,
2-(2,1,3-Benzothiadiazol-5-yl)-3-(3,4,5-triisopropoxybenzyl)-4-(3,4-dimethylphenyl)-4-oxo-2-butensäure Natriumsalz,
2-(2,1,3-Benzothiadiazol-5-yl)-3-(4-trifluormethylbenzyl)-5-hydroxy-5-(3,4-dimethylphenyl)-4-oxo-2-butensäure Natriumsalz,
2-(2,1,3-Benzothiadiazol-5-yl)-3-(4-cyanbenzyl)-4-(3,4-dimethylphenyl)-4-oxo-2-butensäure Natriumsalz,
2-(2,1,3-Benzothiadiazol-5-yl)-3-(4-methylbenzyl)-4-(3,4-dimethylphenyl)-4-oxo-2-butensäure Natriumsalz,
2-(2,1,3-Benzothiadiazol-5-yl)-3-(3-methyl-4-methoxybenzyl)-4-(3,4-dimethylphenyl)-4-oxo-2-butensäure Natriumsalz,
2-(2,1,3-Benzothiadiazol-5-yl)-3-(4-tert.-butylbenzyl)-4-(3,4-dimethylphenyl)-4-oxo-2-butensäure Natriumsalz,
2-(2,1,3-Benzothiadiazol-5-yl)-3-[(2-methoxycarbonylmethyloxy-4-methoxy)benzyl]-4-(3,4-dimethylphenyl)-4-oxo-2-butensäure Natriumsalz,
2-(2,1,3-Benzothiadiazol-5-yl)-3-[(2-carboxymethyloxy-4-methoxy)-benzyl]-4-(3,4-dimethylphenyl)-4-oxo-2-butensäure Natriumsalz,
2-(2,1,3-Benzothiadiazol-5-yl)-3-(4-ethoxycarbonylbenzyl)-4-(3,4-dimethylphenyl)-4-oxo-2-butensäure Natriumsalz,
2-(2,1,3-Benzothiadiazol-5-yl)-3-(4-benzyloxybenzyl)-4-(3,4-dimethylphenyl)-4-oxo-2-butensäure Natriumsalz,
2-(2,1,3-Benzothiadiazol-5-yl)-3-(4-dimethylaminobenzyl)-4-(3,4-dimethylphenyl)-4-oxo-2-butensäure Natriumsalz,
2-(2,1,3-Benzothiadiazol-5-yl)-3-(4-nitrobenzyl)-4-(3,4-dimethylphenyl)-4-oxo-2-butensäure Natriumsalz,
2-(2,1,3-Benzothiadiazol-5-yl)-3-[(1,3-benzodioxol-5-yl)-methyl]-4-(3,4-dimethylphenyl)-4-oxo-2-butensäure Natriumsalz,
2-(2,1,3-Benzothiadiazol-5-yl)-3-(3-fluor-4-methoxybenzyl)- 4-(3,4-dimethylphenyl)-4-oxo-2-butensäure Natriumsalz,
2-(2,1,3-Benzothiadiazol-5-yl)-3-[3-(N,N-dimethylamino)benzyl]-4-(3,4-dimethylphenyl)-4-oxo-2-butensäure Natriumsalz,
2-(2,1,3-Benzothiadiazol-5-yl)-3-(cyclohexylmethyl)-4-(3,4-dimethylphenyl)-4-oxo-2-butensäure Natriumsalz und
2-(2,1,3-Benzothiadiazol-5-yl)-3-(cyclopentylmethyl)-5-hydroxy-(3,4-dimethylphenyl)-4-oxo-2-butensäure Natriumsalz.
2-(2,1,3-Benzothiadiazol-5-yl)-3-benzyl-4-(1,3-benzodioxol-5-yl)-4-oxo-2-butensäure Natriumsalz,
2-(2,1,3-Benzothiadiazol-5-yl)-3-(2-methoxybenzyl)-4-(1,3-benzodioxol-5-yl)-4-oxo-2-butensäure Natriumsalz,
2-(2,1,3-Benzothiadiazol-5-yl)-3-(3-methoxybenzyl)-4-(1,3-benzodioxol-5-yl)-4-oxo-2-butensäure Natriumsalz,
2-(2,1,3-Benzothiadiazol-5-yl)-3-(4-methoxybenzyl)-4-(1,3-benzodioxol-5-yl)-4-oxo-2-butensäure Natriumsalz,
2-(2,1,3-Benzothiadiazol-5-yl)-3-(3,4-dimethoxybenzyl)-4-(1,3-benzodioxol-5-yl)-4-oxo-2-butensäure Natriumsalz,
2-(2,1,3-Benzothiadiazol-5-yl)-3-(34,5-trimethoxybenzyl)-4-(1,3-benzodioxol-5-yl)-4-oxo-2-butensäure Natriumsalz, FAB 557
2-(2,1,3-Benzothiadiazol-5-yl)-3-(3,4-diisopropoxy-5-methoxybenzyl)-4-(1,3-benzodioxol-5-yl)-4-oxo-2-butensäure Natriumsalz,
2-(2,1,3-Benzothiadiazol-5-yl)-3-(3,4,5-triisopropoxybenzyl)-4-(1,3-benzodioxol-5-yl)-4-oxo-2-butensäure Natriumsalz,
2-(2,1,3-Benzothiadiazol-5-yl)-3-(4-trifluormethylbenzyl)-5-hydroxy-5-(1,3-benzodioxol-5-yl)-4-oxo-2-butensäure Natriumsalz,
2-(2,1,3-Benzothiadiazol-5-yl)-3-(4-cyanbenzyl)-4-(1,3-benzodioxol-5-yl)-4-oxo-2-butensäure Natriumsalz,
2-(2,1,3-Benzothiadiazol-5-yl)-3-(4-methylbenzyl)-4-(1,3-benzodioxol-5-yl)-4-oxo-2-butensäure Natriumsalz,
2-(2,1,3-Benzothiadiazol-5-yl)-3-(3-methyl-4-methoxybenzyl)-4-(1,3-benzodioxol-5-yl)-4-oxo-2-butensäure Natriumsalz,
2-(2,1,3-Benzothiadiazol-5-yl)-3-(4-tert.-butylbenzyl)-4-(1,3-benzodioxol-5-yl)-4-oxo-2-butensäure Natriumsalz,
2-(2,1,3-Benzothiadiazol-5-yl)-3-[(2-methoxycarbonylmethyloxy-4-methoxy)benzyl]-4-(1,3-benzodioxol-5-yl)-4-oxo-2-butensäure Natriumsalz,
2-(2,1,3-Benzothiadiazol-5-yl)-3-[(2-carboxymethyloxy-4-methoxy)-benzyl]-4-(1,3-benzodioxol-5-yl)-4-oxo-2-butensäure Natriumsalz,
2-(2,1,3-Benzothiadiazol-5-yl)-3-(4-ethoxycarbonylbenzyl)-4-(1,3-benzodioxol-5-yl)-4-oxo-2-butensäure Natriumsalz,
2-(2,1,3-Benzothiadiazol-5-yl)-3-(4-benzyloxybenzyl)-4-(1,3-benzodioxol-5-yl)-4-oxo-2-butensäure Natriumsalz,
2-(2,1,3-Benzothiadiazol-5-yl)-3-(4-dimethylaminobenzyl)-4-(1,3-benzodioxol-5-yl)-4-oxo-2-butensäure Natriumsalz,
2-(2,1,3-Benzothiadiazol-5-yl)-3-(4-nitrobenzyl)-4-(1,3-benzodioxol-5-yl)-4-oxo-2-butensäure Natriumsalz,
2-(2,1,3-Benzothiadiazol-5-yl)-3-[(1,3-benzodioxol-5-yl)-methyl]-4-(1,3-benzodioxol-5-yl)-4-oxo-2-butensäure Natriumsalz,
2-(2,1,3-Benzothiadiazol-5-yl)-3-(3-fluor-4-methoxybenzyl)-4-(1,3-benzodioxol-5-yl)-4-oxo-2-butensäure Natriumsalz,
2-(2,1,3-Benzothiadiazol-5-yl)-3-[3-(N,N-dimethylamino)benzyl]-4-(1,3-benzodioxol-5-yl)-4-oxo-2-butensäure Natriumsalz,
2-(2,1,3-Benzothiadiazol-5-yl)-3-(cyclohexylmethyl)-4-(1,3-benzodioxol-5-yl)-4-oxo-2-butensäure Natriumsalz und
2-(2,1,3-Benzothiadiazol-5-yl)-3-(cyclopentylmethyl)-5-hydroxy-(1,3-benzodioxol-5-yl)-4-oxo-2-butensäure Natriumsalz.
2-(2,1,3-Benzothiadiazol-5-yl)-3-benzyl-4-(3,4,5-trimethoxyphenyl)-4-oxo-2-butensäure Natriumsalz,
2-(2,1,3-Benzothiadiazol-5-yl)-3-(2-methoxybenzyl)-4-(3,4,5-trimethoxyphenyl)-4-oxo-2-butensäure Natriumsalz,
2-(2,1,3-Benzothiadiazol-5-yl)-3-(3-methoxybenzyl)-4-(3,4,5-trimethoxyphenyl)-4-oxo-2-butensäure Natriumsalz,
2-(2,1,3-Benzothiadiazol-5-yl)-3-(4-methoxybenzyl)-4-(3,4,5-trimethoxyphenyl)-4-oxo-2-butensäure Natriumsalz,
2-(2,1,3-Benzothiadiazol-5-yl)-3-(3,4-dimethoxybenzyl)-4-(3,4,5-trimethoxyphenyl)-4-oxo-2-butensäure Natriumsalz,
2-(2,1,3-Benzothiadiazol-5-yl)-3-(3,4,5-trimethoxybenzyl)-4-(3,4,5-trimethoxyphenyl)-4-oxo-2-butensäure Natriumsalz,
2-(2,1,3-Benzothiadiazol-5-yl)-3-(3,4-diisopropoxy-5-methoxybenzyl)-4-(3,4,5-trimethoxyphenyl)-4-oxo-2-butensäure Natriumsalz,
2-(2,1,3-Benzothiadiazol-5-yl)-3-(3,4,5-triisopropoxybenzyl)-4-(3,4,5-trimethoxyphenyl)-4-oxo-2-butensäure Natriumsalz,
2-(2,1,3-Benzothiadiazol-5-yl)-3-(4-trifluormethylbenzyl)-5-hydroxy-5-(3,4,5-trimethoxyphenyl)-4-oxo-2-butensäure Natriumsalz,
2-(2,1,3-Benzothiadiazol-5-yl)-3-(4-cyanbenzyl)-4-(3,4,5-trimethoxyphenyl)-4-oxo-2-butensäure Natriumsalz,
2-(2,1,3-Benzothiadiazol-5-yl)-3-(4-methylbenzyl)-4-(3,4,5-trimethoxyphenyl)-4-oxo-2-butensäure Natriumsalz,
2-(2,1,3-Benzothiadiazol-5-yl)-3-(3-methyl-4-methoxybenzyl)-4-(3,4,5-trimethoxyphenyl)-4-oxo-2-butensäure Natriumsalz,
2-(2,1,3-Benzothiadiazol-5-yl)-3-(4-tert.-butylbenzyl)-4-(3,4,5-trimethoxyphenyl)-4-oxo-2-butensäure Natriumsalz,
2-(2,1,3-Benzothiadiazol-5-yl)-3-[(2-methoxycarbonylmethyloxy-4-methoxy)benzyl]-4-(3,4,5-trimethoxyphenyl)-4-oxo-2-butensäure Natriumsalz,
2-(2,1,3-Benzothiadiazol-5-yl)-3-[(2-carboxymethyloxy-4-methoxy)-benzyl]-4-(3,4,5-trimethoxyphenyl)-4-oxo-2-butensäure Natriumsalz,
2-(2,1,3-Benzothiadiazol-5-yl)-3-(4-ethoxycarbonylbenzyl)-4-(3,4,5-trimethoxyphenyl)-4-oxo-2-butensäure Natriumsalz,
2-(2,1,3-Benzothiadiazol-5-yl)-3-(4-benzyloxybenzyl)-4-(3,4,5-trimethoxyphenyl)-4-oxo-2-butensäure Natriumsalz,
2-(2,1,3-Benzothiadiazol-5-yl)-3-(4-dimethylaminobenzyl)-4-(3,4,5-trimethoxyphenyl)-4-oxo-2-butensäure Natriumsalz,
2-(2,1,3-Benzothiadiazol-5-yl)-3-(4-nitrobenzyl)-4-(3,4,5-trimethoxyphenyl)-4-oxo-2-butensäure Natriumsalz,
2-(2,1,3-Benzothiadiazol-5-yl)-3-[(1,3-benzodioxol-5-yl)-methyl]-4-(3,4,5-trimethoxyphenyl)-4-oxo-2-butensäure Natriumsalz,
2-(2,1,3-Benzothiadiazol-5-yl)-3-(3-fluor-4-methoxybenzyl)- 4-(3,4,5-trimethoxyphenyl)-4-oxo-2-butensäure Natriumsalz,
2-(2,1,3-Benzothiadiazol-5-yl)-3-[3-(N,N-dimethylamino)benzyl]-4-(3,4,5-trimethoxyphenyl)-4-oxo-2-butensäure Natriumsalz,
2-(2,1,3-Benzothiadiazol-5-yl)-3-(cyclohexylmethyl)-4-(3,4,5-trimethoxyphenyl)-4-oxo-2-butensäure Natriumsalz und
2-(2,1,3-Benzothiadiazol-5-yl)-3-(cyclopentylmethyl)-5-hydroxy-(3,4,5-trimethoxyphenyl)-4-oxo-2-butensäure Natriumsalz.
2-(2,1,3-Benzothiadiazol-5-yl)-3-benzyl-4-(3,4-dimethoxyphenyl)-4-oxo-2-butensäure Natriumsalz,
2-(2,1,3-Benzothiadiazol-5-yl)-3-(2-methoxybenzyl)-4-(3,4-dimethoxyphenyl)-4-oxo-2-butensäure Natriumsalz,
2-(2,1,3-Benzothiadiazol-5-yl)-3-(3-methoxybenzyl)-4-(3,4-dimethoxyphenyl)-4-oxo-2-butensäure Natriumsalz,
2-(2,1,3-Benzothiadiazol-5-yl)-3-(4-methoxybenzyl)-4-(3,4-dimethoxyphenyl)-4-oxo-2-butensäure Natriumsalz,
2-(2,1,3-Benzothiadiazol-5-yl)-3-(3,4-dimethoxybenzyl)-4-(3,4-dimethoxyphenyl)-4-oxo-2-butensäure Natriumsalz,
2-(2,1,3-Benzothiadiazol-5-yl)-3-(3,4,5-trimethoxybenzyl)-4-(34-dimethoxyphenyl)-4-oxo-2-butensäure Natriumsalz,
2-(2,1,3-Benzothiadiazol-5-yl)-3-(3,4-diisopropoxy-5-methoxybenzyl)-4-(3,4-dimethoxyphenyl)-4-oxo-2-butensäure Natriumsalz,
2-(2,1,3-Benzothiadiazol-5-yl)-3-(3,4,5-triisopropoxybenzyl)-4-(3,4-dimethoxyphenyl)-4-oxo-2-butensäure Natriumsalz,
2-(2,1,3-Benzothiadiazol-5-yl)-3-(4-trifluormethylbenzyl)-5-hydroxy-5-(3,4-dimethoxyphenyl)-4-oxo-2-butensäure Natriumsalz,
2-(2,1,3-Benzothiadiazol-5-yl)-3-(4-cyanbenzyl)-4-(3,4-dimethoxyphenyl)-4-oxo-2-butensäure Natriumsalz,
2-(2,1,3-Benzothiadiazol-5-yl)-3-(4-methylbenzyl)-4-(3,4-dimethoxyphenyl)-4-oxo-2-butensäure Natriumsalz,
2-(2,1,3-Benzothiadiazol-5-yl)-3-(3-methyl-4-methoxybenzyl)-4-(3,4-dimethoxyphenyl)-4-oxo-2-butensäure Natriumsalz,
2-(2,1,3-Benzothiadiazol-5-yl)-3-(4-tert.-butylbenzyl)-4-(3,4-dimethoxyphenyl)-4-oxo-2-butensäure Natriumsalz,
2-(2,1,3-Benzothiadiazol-5-yl)-3-[(2-methoxycarbonylmethyloxy-4-methoxy)benzyl]-4-(3,4-dimethoxyphenyl)-4-oxo-2-butensäure Natriumsalz,
2-(2,1,3-Benzothiadiazol-5-yl)-3-[(2-carboxymethyloxy-4-methoxy)-benzyl]-4-(3,4-dimethoxyphenyl)-4-oxo-2-butensäure Natriumsalz,
2-(2,1,3-Benzothiadiazol-5-yl)-3-(4-ethoxycarbonylbenzyl)-4-(3,4-dimethoxyphenyl)-4-oxo-2-butensäure Natriumsalz,
2-(2,1,3-Benzothiadiazol-5-yl)-3-(4-benzyloxybenzyl)-4-(3,4-dimethoxyphenyl)-4-oxo-2-butensäure Natriumsalz,
2-(2,1,3-Benzothiadiazol-5-yl)-3-(4-dimethylaminobenzyl)-4-(3,4-dimethoxyphenyl)-4-oxo-2-butensäure Natriumsalz,
2-(2,1,3-Benzothiadiazol-5-yl)-3-(4-nitrobenzyl)-4-(3,4-dimethoxyphenyl)-4-oxo-2-butensäure Natriumsalz,
2-(2,1,3-Benzothiadiazol-5-yl)-3-[(1,3-benzodioxol-5-yl)-methyl]-4-(3,4-dimethoxyphenyl)-4-oxo-2-butensäure Natriumsalz,
2-(2,1,3-Benzothiadiazol-5-yl)-3-(3-fluor-4-methoxybenzyl)- 4-(3,4-dimethoxyphenyl)-4-oxo-2-butensäure Natriumsalz,
2-(2,1,3-Benzothiadiazol-5-yl)-3-[3-(N,N-dimethylamino)benzyl]-4-(3,4-dimethoxyphenyl)-4-oxo-2-butensäure Natriumsalz,
2-(2,1,3-Benzothiadiazol-5-yl)-3-(cyclohexylmethyl)-4-(3,4-dimethoxyphenyl)-4-oxo-2-butensäure Natriumsalz und
2-(2,1,3-Benzothiadiazol-5-yl)-3-(cyclopentylmethyl)-5-hydroxy-(3,4-dimethoxyphenyl)-4-oxo-2-butensäure Natriumsalz.
2-(2,1,3-Benzothiadiazol-5-yl)-3-benzyl-4-(4-methylthiophenyl)-4-oxo-2-butensäure Natriumsalz,
2-(2,1,3-Benzothiadiazol-5-yl)-3-(2-methoxybenzyl)-4-(4-methylthiophenyl)-4-oxo-2-butensäure Natriumsalz,
2-(2,1,3-Benzothiadiazol-5-yl)-3-(3-methoxybenzyl)-4-(4-methylthiophenyl)-4-oxo-2-butensäure Natriumsalz,
2-(2,1,3-Benzothiadiazol-5-yl)-3-(4-methoxybenzyl)-4-(4-methylthiophenyl)-4-oxo-2-butensäure Natriumsalz,
2-(2,1,3-Benzothiadiazol-5-yl)-3-(3,4-dimethoxybenzyl)-4-(4-methylthiophenyl)-4-oxo-2-butensäure Natriumsalz,
2-(2,1,3-Benzothiadiazol-5-yl)-3-(3,4,5-trimethoxybenzyl)-4-(4-methylthiophenyl)-4-oxo-2-butensäure Natriumsalz,
2-(2,1,3-Benzothiadiazol-5-yl)-3-(3,4-diisopropoxy-5-methoxybenzyl)-4-(4-methylthiophenyl)-4-oxo-2-butensäure Natriumsalz,
2-(2,1,3-Benzothiadiazol-5-yl)-3-(3,4,5-triisopropoxybenzyl)-4-(4-methylthiophenyl)-4-oxo-2-butensäure Natriumsalz,
2-(2,1,3-Benzothiadiazol-5-yl)-3-(4-trifluormethylbenzyl)-5-hydroxy-5-(4-methylthiophenyl)-4-oxo-2-butensäure Natriumsalz,
2-(2,1,3-Benzothiadiazol-5-yl)-3-(4-cyanbenzyl)-4-(4-methylthiophenyl)-4-oxo-2-butensäure Natriumsalz,
2-(2,1,3-Benzothiadiazol-5-yl)-3-(4-methylbenzyl)-4-(4-methylthiophenyl)-4-oxo-2-butensäure Natriumsalz,
2-(2,1,3-Benzothiadiazol-5-yl)-3-(3-methyl-4-methoxybenzyl)-4-(4-methylthiophenyl)-4-oxo-2-butensäure Natriumsalz,
2-(2,1,3-Benzothiadiazol-5-yl)-3-(4-tert.-butylbenzyl)-4-(4-methylthiophenyl)-4-oxo-2-butensäure Natriumsalz,
2-(2,1,3-Benzothiadiazol-5-yl)-3-[(2-methoxycarbonylmethyloxy-4-methoxy)benzyl]-4-(4-methylthiophenyl)-4-oxo-2-butensäure Natriumsalz,
2-(2,1,3-Benzothiadiazol-5-yl)-3-[(2-carboxymethyloxy-4-methoxy)-benzyl]-4-(4-methylthiophenyl)-4-oxo-2-butensäure Natriumsalz,
2-(2,1,3-Benzothiadiazol-5-yl)-3-(4-ethoxycarbonylbenzyl)-4-(4-methylthiophenyl)-4-oxo-2-butensäure Natriumsalz,
2-(2,1,3-Benzothiadiazol-5-yl)-3-(4-benzyloxybenzyl)-4-(4-methylthiophenyl)-4-oxo-2-butensäure Natriumsalz,
2-(2,1,3-Benzothiadiazol-5-yl)-3-(4-dimethylaminobenzyl)-4-(4-methylthiophenyl)-4-oxo-2-butensäure Natriumsalz,
2-(2,1,3-Benzothiadiazol-5-yl)-3-(4-nitrobenzyl)-4-(4-methylthiophenyl)-4-oxo-2-butensäure Natriumsalz,
2-(2,1,3-Benzothiadiazol-5-yl)-3-[(1,3-benzodioxol-5-yl)-methyl]-4-(4-methylthiophenyl)-4-oxo-2-butensäure Natriumsalz,
2-(2,1,3-Benzothiadiazol-5-yl)-3-(3-fluor-4-methoxybenzyl)- 4-(4-methylthiophenyl)-4-oxo-2-butensäure Natriumsalz,
2-(2,1,3-Benzothiadiazol-5-yl)-3-[3-(N,N-dimethylamino)benzyl]-4-(4-methylthiophenyl)-4-oxo-2-butensäure Natriumsalz,
2-(2,1,3-Benzothiadiazol-5-yl)-3-(cyclohexylmethyl)-4-(4-methylthiophenyl)-4-oxo-2-butensäure Natriumsalz und
2-(2,1,3-Benzothiadiazol-5-yl)-3-(cyclopentylmethyl)-5-hydroxy-(4-methylthiophenyl)-4-oxo-2-butensäure Natriumsalz.
2-(2,1,3-Benzoxadiazol-5-yl)-3-benzyl-4-(4-methoxyphenyl)-4-oxo-2-butensäure Natriumsalz,
2-(2,1,3-Benzoxadiazol-5-yl)-3-(2-methoxybenzyl)-4-(4-methoxyphenyl)-4-oxo-2-butensäure Natriumsalz,
2-(2,1,3-Benzoxadiazol-5-yl)-3-(3-methoxybenzyl)-4-(4-methoxyphenyl)-4-oxo-2-butensäure Natriumsalz,
2-(2,1,3-Benzoxadiazol-5-yl)-3-(4-methoxybenzyl)-4-(4-methoxyphenyl)-4-oxo-2-butensäure Natriumsalz,
2-(2,1,3-Benzoxadiazol-5-yl)-3-(3,4-dimethoxybenzyl)-4-(4-methoxyphenyl)-4-oxo-2-butensäure Natriumsalz,
2-(2,1,3-Benzoxadiazol-5-yl)-3-(3,4,5-trimethoxybenzyl)-4-(4-methoxyphenyl)-4-oxo-2-butensäure Natriumsalz,
2-(2,1,3-Benzoxadiazol-5-yl)-3-(3,4-diisopropoxy-5-methoxybenzyl)-4-(4-methoxyphenyl)-4-oxo-2-butensäure Natriumsalz,
2-(2,1,3-Benzoxadiazol-5-yl)-3-(3,4,5-triisopropoxybenzyl)-4-(4-methoxyphenyl)-4-oxo-2-butensäure Natriumsalz,
2-(2,1,3-Benzoxadiazol-5-yl)-3-(4-trifluormethylbenzyl)-5- hydroxy-5-(4-methoxyphenyl)-4-oxo-2-butensäure Natriumsalz,
2-(2,1,3-Benzoxadiazol-5-yl)-3-(4-cyanbenzyl)-4-(4-methoxyphenyl)-4-oxo-2-butensäure Natriumsalz,
2-(2,1,3-Benzoxadiazol-5-yl)-3-(4-methylbenzyl)-4-(4-methoxyphenyl)-4-oxo-2-butensäure Natriumsalz,
2-(2,1,3-Benzoxadiazol-5-yl)-3-(3-methyl-4-methoxybenzyl)-4-(4-methoxyphenyl)-4-oxo-2-butensäure Natriumsalz,
2-(2,1,3-Benzoxadiazol-5-yl)-3-(4-tert.-butylbenzyl)-4-(4-methoxyphenyl)-4-oxo-2-butensäure Natriumsalz,
2-(2,1,3-Benzoxadiazol-5-yl)-3-[(2-methoxycarbonylmethyloxy-4-methoxy)benzyl]-4-(4-methoxyphenyl)-4-oxo-2-butensäure Natriumsalz,
2-(2,1,3-Benzoxad iazol-5-yl)-3-[(2-carboxymethyloxy-4-methoxy)benzyl]-4-(4-methoxyphenyl)-4-oxo-2-butensäure Natriumsalz,
2-(2,1,3-Benzoxadiazol-5-yl)-3-(4-ethoxycarbonylbenzyl)-4-(4-methoxyphenyl)-4-oxo-2-butensäure Natriumsalz,
2-(2,1,3-Benzoxadiazol-5-yl)-3-(4-benzyloxybenzyl)-4-(4-methoxyphenyl)-4-oxo-2-butensäure Natriumsalz,
2-(2,1,3-Benzoxadiazol-5-yl)-3-(4-dimethylaminobenzyl)-4-(4-methoxyphenyl)-4-oxo-2-butensäure Natriumsalz,
2-(2,1,3-Benzoxadiazol-5-yl)-3-(4-nitrobenzyl)-4-(4-methoxyphenyl)-4-oxo-2-butensäure Natriumsalz,
2-(2,1,3-Benzoxadiazol-5-yl)-3-[(1,3-benzodioxol-5-yl)-methyl]-4-(4-methoxyphenyl)-4-oxo-2-butensäure Natriumsalz,
2-(2,1,3-Benzoxadiazol-5-yl)-3-(3-fluor-4-methoxybenzyl)- 4-(4-methoxyphenyl)-4-oxo-2-butensäure Natriumsalz,
2-(2,1,3-Benzoxadiazol-5-yl)-3-[3-(N,N-dimethylamino)benzyl]-4-(4-methoxyphenyl)-4-oxo-2-butensäure Natriumsalz,
2-(2,1,3-Benzoxadiazol-5-yl)-3-cyclohexylmethyl-4-(4-methoxyphenyl)-4-oxo-2-butensäure Natriumsalz und
2-(2,1,3-Benzoxadiazol-5-yl)-3-cyclopentylmethyl-4-(4-methoxyphenyl)-4-oxo-2-butensäure Natriumsalz.

### Beispiel 3

Zu einer Lösung von 80 mg Natrium in 5 ml Methanol gibt man 0,38 g Benzaldehyd und 1,20 g 4-(2,1,3-Benzothiadiazol-5-yl)-2-(1,3-benzodioxol-5-yl)-4-oxo-butansäureethylester ("B") und erhitzt eine Stunde unter Rückfluß. Nach Zugabe von 5 ml Essigsäure wird weitere 16 Stunden erhitzt. Das Lösungsmittel wird entfernt und wie üblich aufgearbeitet. Man erhält 5-(2,1,3-Benzothiadiazol-5-yl)-4-benzyl-5- hydroxy-3-(1,3-benzodioxol-5-yl)-5H-furan-2-on, amorph.

Analog erhält man durch Umsetzung von "B"
mit 4-Methoxybenzaldehyd
   5-(2,1,3-Benzothiadiazol-5-yl)-4-(4-methoxyphenylmethyl)-5-hydroxy-3-(1,3-benzodioxol-5-yl)-5H-furan-2-on,
mit 3,4-Dimethoxybenzaldehyd
   5-(2,1,3-Benzothiadiazol-5-yl)-4-(3,4-dimethoxybenzyl)-5-hydroxy-3-(1,3-benzodioxol-5-yl)-5H-furan-2-on,
mit 3,4,5-Trimethoxybenzaldehyd
   5-(2,1,3-Benzothiadiazol-5-yl)-4-(3,4,5-trimethoxybenzyl)-5-hydroxy-3-(1,3-benzodioxol-5-yl)-5H-furan-2-on,
mit 3,4-Diisopropoxy-5-methoxybenzaldehyd
   5-(2,1,3-Benzothiadiazol-5-yl)-4-(3,4-diisopropoxy-5-methoxybenzyl)-5-hydroxy-3-(1,3-benzodioxol-5-yl)-5H-furan-2-on,
mit 3,4,5-Triisopropoxybenzaldehyd
   5-(2,1,3-Benzothiadiazol-5-yl)-4-(3,4,5-triisopropoxybenzyl)-5-hydroxy-3-(1,3-benzodioxol-5-yl)-5H-furan-2-on,
mit 4-Chlorbenzaldehyd
   5-(2,1,3-Benzothiadiazol-5-yl)-4-(4-chlorbenzyl)-5-hydroxy-3-(1,3-benzodioxol-5-yl)-5H-furan-2-on,
mit 4-Brombenzaldehyd
   5-(2,1,3-Benzothiadiazol-5-yl)-4-(4-brombenzyl)-5-hydroxy-3-(1,3-benzodioxol-5-yl)-5H-furan-2-on,
mit 4-Trifluormethylbenzaldehyd
   5-(2,1,3-Benzothiadiazol-5-yl)-4-(4-trifluormethylbenzyl)-5-hydroxy-3-(1,3-benzodioxol-5-yl)-5H-furan-2-on,
mit 4-Cyanbenzaldehyd
   5-(2,1,3-Benzothiadiazol-5-yl)-4-(4-cyanbenzyl)-5-hydroxy-3-(1,3-benzodioxol-5-yl)-5H-furan-2-on,
mit 4-Methylbenzaldehyd
   5-(2,1,3-Benzothiadiazol-5-yl)-4-(4-methylbenzyl)-5-hydroxy-3-(1,3-benzodioxol-5-yl)-5H-furan-2-on,
mit 3-Methyl-4-methoxybenzaldehyd
   5-(2,1,3-Benzothiadiazol-5-yl)-4-(3-methyl-4-methoxybenzyl)-5-hydroxy-3-(1,3-benzodioxol-5-yl)-5H-furan-2-on,
mit 4-tert-Butylbenzaldehyd
   5-(2,1,3-Benzothiadiazol-5-yl)-4-(4-tert.-butylbenzyl)-5-hydroxy-3-(1,3-benzodioxol-5-yl)-5H-furan-2-on,
mit (2-Methoxycarbonylmethyloxy-4-methoxy)benzaldehyd
   5-(2,1,3-Benzothiadiazol-5-yl)-4-[(2-methoxycarbonylmethyloxy-4-methoxy)benzyl]-5-hydroxy-3-(1,3-benzodioxol-5-yl)-5H-furan-2-on,
mit (2-Carboxymethyloxy-4-methoxy)benzaldehyd
   5-(2,1,3-Benzothiadiazol-5-yl)-4-[(2-carboxymethyloxy-4-methoxy)benzyl]-5-hydroxy-3-(1,3-benzodioxol-5-yl)-5H-furan-2-on,
mit 4-Ethoxycarbonylbenzaldehyd
   5-(2,1,3-Benzothiadiazol-5-yl)-4-(4-ethoxycarbonylbenzyl)-5-hydroxy-3-(1,3-benzodioxol-5-yl)-5H-furan-2-on,
mit 4-Methansulfonylbenzaldehyd
   5-(2,1,3-Benzothiadiazol-5-yl)-4-(4-methansulfonylbenzyl)-5-hydroxy-3-(1,3-benzodioxol-5-yl)-5H-furan-2-on,
mit 4-Benzyloxybenzaldehyd
   5-(2,1,3-Benzothiadiazol-5-yl)-4-(4-benzyloxybenzyl)-5-hydroxy-3-(1,3-benzodioxol-5-yl)-5H-furan-2-on,
mit 4-Dimethylaminobenzaldehyd
   5-(2,1,3-Benzothiadiazol-5-yl)-4-(4-dimethylaminobenzyl)-5-hydroxy-3-(1,3-benzodioxol-5-yl)-5H-furan-2-on,
mit 4-Nitrobenzaldehyd
   5-(2,1,3-Benzothiadiazol-5-yl)-4-(4-nitrobenzyl)-5-hydroxy-3-(1,3-benzodioxol-5-yl)-5H-furan-2-on,
mit 4-Formylbenzaldehyd
   5-(2,1,3-Benzothiadiazol-5-yl)-4-(4-formylbenzyl)-5-hydroxy-3-(1,3-benzodioxol-5-yl)-5H-furan-2-on,
mit (1,3-Benzodioxol-5-yl)-carbaldehyd
   5-(2,1,3-Benzothiadiazol-5-yl)-4-[(1,3-benzodioxol-5-yl)-methyl]-5-hydroxy-3-(1,3-benzodioxol-5-yl)-5H-furan-2-on,
mit 2-(1,3-Benzodioxol-5-yl-oxy)benzaldehyd
   5-(2,1,3-Benzothiadiazol-5-yl)-4-[2-(1,3-benzodioxol-5-yl-oxy)benzyl]-5-hydroxy-3-(1,3-benzodioxol-5-yl)-5H-furan-2-on,
mit Cyclohexancarbaldehyd
   5-(2,1,3-Benzothiadiazol-5-yl)-4-cyclohexylmethyl-5-hydroxy-3-(1,3-benzodioxol-5-yl)-5H-furan-2-on und
mit Cyclopentancarbaldehyd
   5-(2,1,3-Benzothiadiazol-5-yl)-4-cyclopentylmethyl-5-hydroxy-3-(1,3-benzodioxol-5-yl)-5H-furan-2-on.

### Beispiel 4

Zu einer Lösung von 80 mg Natrium in 5 ml Methanol gibt man 0,5 g (2,1,3-Benzothiadiazol-5-yl)-carbaldehyd ("C") und 1,20 g 2-(1,3-Benzodioxol-5-yl)-4-(4-methoxyphenyl)-4-oxo-butansäureethylester und erhitzt eine Stunde unter Rückfluß. Nach Zugabe von 5 ml Essigsäure wird weitere 16 Stunden erhitzt. Das Lösungsmittel wird entfernt und wie üblich aufgearbeitet. Man erhält 4-(2,1,3-Benzothiadiazol-5-yl-methyl)-3-(1,3-benzodioxol-5-yl)-5-hydroxy-5-(4-methoxyphenyl)-5H-furan-2-on, F. 172°.

Analog erhält man durch Umsetzung von "C"
mit 2-(1,3-Benzodioxol-5-yl)-3-(3,4-dimethoxyphenyl)-4-oxo-butansäureethylester
   4-(2,1,3-Benzothiadiazol-5-yl-methyl)-3-(1,3-benzodioxol-5-yl)-5-hydroxy-5-(3,4-dimethoxyphenyl)-5H-furan-2-on, mit 2-(1,3-Benzodioxol-5-yl)-3-(3,4,5-trimethoxyphenyl)-4-oxo-butansäureethylester
   4-(2,1,3-Benzothiadiazol-5-yl-methyl)-3-(1,3-benzodioxol-5-yl)-5-hydroxy-5-(3,4,5-trimethoxyphenyl)-5H-furan-2-on,
mit 2-(1,3-Benzodioxol-5-yl)-3-(3,4-diisopropoxy-5-methoxyphenyl)-4-oxobutansäureethylester
   4-(2,1,3-Benzothiadiazol-5-yl-methyl)-3-(1,3-benzodioxol-5-yl)-5-hydroxy-5-(3,4-diisopropoxy-5-methoxyphenyl)-5H-furan-2-on,
mit 2-(1,3-Benzodioxol-5-yl)-3-(3,4,5-triisopropoxyphenyl)-4-oxobutansäureethylester
   4-(2,1,3-Benzothiadiazol-5-yl-methyl)-3-(1,3-benzodioxol-5-yl)-5-hydroxy-5-(3,4,5-triisopropoxy)-5H-furan-2-on,
mit 2-(1,3-Benzodioxol-5-yl)-3-(4-chlorphenyl)-4-oxo-butansäureethylester
   4-(2,1,3-Benzothiadiazol-5-yl-methyl)-3-(1,3-benzodioxol-5-yl)-5-hydroxy-5-(4-chlorphenyl)-5H-furan-2-on,
mit 2-(1,3-Benzodioxol-5-yl)-3-(4-bromphenyl)-4-oxo-butansäureethylester
   4-(2,1,3-Benzothiadiazol-5-yl-methyl)-3-(1,3-benzodioxol-5-yl)-5-hydroxy-5-(4-bromphenyl)-5H-furan-2-on,
mit 2-(1,3-Benzodioxol-5-yl)-3-(4-trifluormethylphenyl)-4-oxo-butansäureethylester
   4-(2,1,3-Benzothiadiazol-5-yl-methyl)-3-(1,3-benzodioxol-5-yl)-5-hydroxy-5-(4-trifluormethylphenyl)-5H-furan-2-on,
mit 2-(1,3-Benzodioxol-5-yl)-3-(4-cyanphenyl)-4-oxo-butansäureethylester
   4-(2,1,3-Benzothiadiazol-5-yl-methyl)-3-(1,3-benzodioxol-5-yl)-5-hydroxy-5-(4-cyanphenyl)-5H-furan-2-on,
mit 2-(1,3-Benzodioxol-5-yl)-3-(4-methylphenyl)-4-oxo-butansäureethylester
   4-(2,1,3-Benzothiadiazol-5-yl-methyl)-3-(1,3-benzodioxol-5-yl)-5-hydroxy-5-(4-methylphenyl)-5H-furan-2-on, mit 2-(1,3-Benzodioxol-5-yl)-3-(3-methyl-4-methoxyphenyl)-4-oxobutansäureethylester
   4-(2,1,3-Benzothiadiazol-5-yl-methyl)-3-(1,3-benzodioxol-5-yl)-5-hydroxy-5-(3-methyl-4-methoxyphenyl)-5H-furan-2-on,
mit 2-(1,3-Benzodioxol-5-yl)-3-(4-tert.-butylphenyl)-4-oxo-butansäureethylester
   4-(2,1,3-Benzothiadiazol-5-yl-methyl)-3-(1,3-benzodioxol-5-yl)-5-hydroxy-5-(4-tert.-butylphenyl)-5H-furan-2-on,
mit 2-(1,3-Benzodioxol-5-yl)-3-[(2-methoxycarbonylmethyloxy-4-methoxy)-phenyl]-4-oxo-butansäureethylester
   4-(2,1,3-Benzothiadiazol-5-yl-methyl)-3-(1,3-benzodioxol-5-yl)-5-hydroxy-5-[(2-methoxycarbonylmethyloxy-4-methoxy)phenyl]-5H-furan-2-on,
mit 2-(1,3-Benzodioxol-5-yl)-3-[(2-carboxymethyloxy-4-methoxy)phenyl]-4-oxo-butansäureethylester
   4-(2,1,3-Benzothiadiazol-5-yl-methyl)-3-(1,3-benzodioxol-5-yl)-5-hydroxy-5-[(2-carboxymethyloxy-4-methoxy)phenyl]-5H-furan-2-on,
mit 2-(1,3-Benzodioxol-5-yl)-3-(4-ethoxycarbonylphenyl)-4-oxobutansäureethylester
   4-(2,1,3-Benzothiadiazol-5-yl-methyl)-3-(1,3-benzodioxol-5-yl)-5-hydroxy-5-(4-ethoxycarbonylphenyl)-5H-furan-2-on,
mit 2-(1,3-Benzodioxol-5-yl)-3-(4-methansulfonylphenyl)-4-oxobutansäureethylester
   4-(2,1,3-Benzothiadiazol-5-yl-methyl)-3-(1,3-benzodioxol-5-yl)-5-hydroxy-5-(4-methansulfonylphenyl)-5H-furan-2-on,
mit 2-(1,3-Benzodioxol-5-yl)-3-(4-benzyloxyphenyl)-4-oxo-butansäureethylester
   4-(2,1,3-Benzothiadiazol-5-yl-methyl)-3-(1,3-benzodioxol-5-yl)-5-hydroxy-5-(4-benzyloxyphenyl)-5H-furan-2-on, mit 2-(1,3-Benzodioxol-5-yl)-3-(4-dimethylaminophenyl)-4-oxo-butansäureethylester
   4-(2,1,3-Benzothiadiazol-5-yl-methyl)-3-(1,3-benzodioxol-5-yl)-5-hydroxy-5-(4-dimethylaminophenyl)-5H-furan-2-on,
mit 2-(1,3-Benzodioxol-5-yl)-3-(4-nitrophenyl)-4-oxo-butansäureethylester
   4-(2,1,3-Benzothiadiazol-5-yl-methyl)-3-(1,3-benzodioxol-5-yl)-5-hydroxy-5-(4-nitrophenyl)-5H-furan-2-on und
mit 2-(1,3-Benzodioxol-5-yl)-3-(4-formylphenyl)-4-oxo-butansäureethylester
   4-(2,1,3-Benzothiadiazol-5-yl-methyl)-3-(1,3-benzodioxol-5-yl)-5-hydroxy-5-(4-formylphenyl)-5H-furan-2-on.

### Beispiel 5

Eine Lösung von 1 g 3-(2,1,3-Benzothiadiazol-5-yl)-4-(4-nitrobenzyl)-5-hydroxy-5-(4-methoxyphenyl)-5H-furan-2-on in 25 ml Methanol wird bei Normaldruck und 20° bis zum Stillstand an 1 g Raney-Nickel hydriert. Man filtriert, entfernt das Lösungsmittel und erhält 3-(2,1,3-Benzothiadiazol-5-yl)-4-(4-aminobenzyl)-5-hydroxy-5-(4-methoxyphenyl)-5H-furan-2-on.

### Beispiel 6

Durch Umsetzung mit äquimolaren Mengen Acetylchlorid in Pyridin und katalytischer Mengen Dimethylaminopyridin erhält man
aus 3-(2,1,3-Benzothiadiazol-5-yl)-4-(4-aminobenzyl)-5-hydroxy-5-(4-methoxyphenyl)-5H-furan-2-on
3-(2,1,3-Benzothiadiazol-5-yl)-4-(4-acetamidobenzyl)-5-hydroxy-5-(4-methoxyphenyl)-5H-furan-2-on.

### Beispiel 7

Durch Umsetzung mit äquimolaren Mengen Phenylisocyanat in Dichlormethan erhält man
aus 3-(2,1,3-Benzothiadiazol-5-yl)-4-(4-aminobenzyl)-5-hydroxy-5-(4-methoxyphenyl)-5H-furan-2-on
3-(2,1,3-Benzothiadiazol-5-yl)-4-[4-(phenylureido)benzyl]-5-hydroxy-5-(4-methoxyphenyl)-5H-furan-2-on.

### Beispiel 8

Durch Umsetzung mit äquimolaren Mengen Butylisocyanat in Dichlormethan erhält man
aus 3-(2,1,3-Benzothiadiazol-5-yl)-4-(4-aminobenzyl)-5-hydroxy-5-(4-methoxyphenyl)-5H-furan-2-on
3-(2,1,3-Benzothiadiazol-5-yl)-4-[4-(butylureido)benzyl]-5-hydroxy-5-(4-methoxyphenyl)-5H-furan-2-on.

### Beispiel 9

Durch Umsetzung mit äquimolaren Mengen NaH und Butyliodid in THF erhält man
aus 3-(2,1,3-Benzothiadiazol-5-yl)-4-(4-acetamidobenzyl)-5-hydroxy-5-(4-methoxyphenyl)-5H-furan-2-on
3-(2,1,3-Benzothiadiazol-5-yl)-4-[4-(N-butylacetamido)benzyl]-5-hydroxy-5-(4-methoxyphenyl)-5H-furan-2-on.

### Beispiel 10

Durch Umsetzung mit äquimolaren Mengen Butylsulfonylchlorid und Cäsiumcarbonat in DMF erhält man
aus 3-(2,1,3-Benzothiadiazol-5-yl)-4-(4-aminobenzyl)-5-hydroxy-5-(4-methoxyphenyl)-5H-furan-2-on
3-(2,1,3-Benzothiadiazol-5-yl)-4-[4-(butylsulfonamido)benzyl]-5-hydroxy-5-(4-methoxyphenyl)-5H-furan-2-on.

Durch analoge Umsetzung mit Tolylsulfonylchlorid erhält man
aus 3-(2,1,3-Benzothiadiazol-5-yl)-4-(4-aminobenzyl)-5-hydroxy-5-(4-methoxyphenyl)-5H-furan-2-on
3-(2,1,3-Benzothiadiazol-5-yl)-4-[4-(tolylsulfonamido)benzyl]-5-hydroxy-5-(4-methoxyphenyl)-5H-furan-2-on.

### Beispiel 11

Durch Behandlung von 0,25 g 3-(2,1,3-Benzothiadiazol-5-yl)-4-[4-(N-butylacetamido)benzyl]-5-hydroxy-5-(4-methoxyphenyl)-5H-furan-2-on in 20 ml Ethanol mit 10 ml KOH-Lösung erhält man nach üblicher Aufarbeitung 2-(2,1,3-Benzothiadiazol-5-yl)-3-[4-(N-butylamino)benzyl]-4-(4-methoxyphenyl)-4-oxo-2-butensäure Kaliumsalz.

### Beispiel 12

Analog Beispiel 1 erhält man durch Umsetzung von 2-(2,1,3-Benzothiadiazol-6-methoxy-5-yl)-4-(1,4-benzodioxan-6-yl)-4-oxo-butansäureethylester mit Cyclohexancarbaldehyd
3-(2,1,3-Benzothiadiazol-6-methoxy-5-yl)-4-(cyclohexylmethyl)-5-hydroxy-5-(1,4-benzodioxan-6-yl)-5H-furan-2-on.

Durch Behandlung mit Natronlauge analog Beispiel 2 erhält man daraus 2-(2,1,3-Benzothiadiazol-6-methoxy-5-yl)-3-(cyclohexylmethyl)-4-(1,4-benzodioxan-6-yl)-4-oxo-2-butensäure Natriumsalz.

### Beispiel 13

Analog Beispiel 1 erhält man durch Umsetzung
von 2-(2,1,3-Benzothiadiazol-5-yl)-4-(3-fluor-4-methoxyphenyl)-4-oxobutansäureethylester ("D") mit Benzaldehyd
   3-(2,1,3-Benzothiadiazol-5-yl)-4-benzyl-5-hydroxy-5-(3-fluor-4-methoxyphenyl)-5H-furan-2-on, F. 65°
von "D" mit 3,4,5-Trimethoxybenzaldehyd ("E")
   3-(2,1,3-Benzothiadiazol-5-yl)-4-(3,4,5-trimethoxybenzyl)-5-hydroxy-5-(3-fluor-4-methoxyphenyl)-5H-furan-2-on, FAB 539 und daraus analog Beispiel 2
   2-(2,1,3-Benzothiadiazol-5-yl)-3-(3,4,5-trimethoxybenzyl)-4-(3-fluor-4-methoxyphenyl)-4-oxo-2-butensäure Natriumsalz, F. 254° (Zersetzung)
von "A" mit (7-Methoxy-1,3-benzodioxol-5-yl)-carbaldehyd
   3-(2,1,3-Benzothiadiazol-5-yl)-4-[(7-methoxy-1,3-benzodioxol-5-yl)-methyl]-5-hydroxy-5-(4-methoxyphenyl)-5H-furan-2-on, F. 61°
von 2-(2,1,3-Benzothiadiazol-5-yl)-4-(3-methoxyphenyl)-4-oxobutansäureethylester mit "E"
   3-(2,1,3-Benzothiadiazol-5-yl)-4-(3,4,5-trimethoxybenzyl)-5-hydroxy-5-(3-methoxyphenyl)-5H-furan-2-on, F. 61°
von 2-(2,1,3-Benzothiadiazol-5-yl)-4-(2-methoxyphenyl)-4-oxobutansäureethylester mit "E"
   3-(2,1,3-Benzothiadiazol-5-yl)-4-(3,4,5-trimethoxybenzyl)-5-hydroxy-5-(2-methoxyphenyl)-5H-furan-2-on, F. 180°
von "A" mit 4-Methylthiobenzaldehyd
   3-(2,1,3-Benzothiadiazol-5-yl)-4-(4-methylthiobenzyl)-5-hydroxy-5-(4-methoxyphenyl)-5H-furan-2-on, F. 64°
von "A" mit 3-Benzyloxy-4-methoxybenzaldehyd
   3-(2,1,3-Benzoth iadiazol-5-yl)-4-(3-benzyloxy-4-methoxybenzyl)-5-hydroxy-5-(4-methoxyphenyl)-5H-furan-2-on, F. 58°
von "A" mit (2,3-Dihydrobenzofuran-5-yl)-carbaldehyd
   3-(2,1,3-Benzothiadiazol-5-yl)-4-(2,3-dihydro-benzofuran-5-ylmethyl)-5-hydroxy-5-(4-methoxyphenyl)-5H-furan-2-on, F. 74°
von "A" mit Isobutyraldehyd
   3-(2,1,3-Benzothiadiazol-5-yl)-4-(2-methylpropyl)-5-hydroxy-5-(4-methoxyphenyl)-5H-furan-2-on, F. 129°
von "A" mit 3,5-Dimethoxybenzaldehyd
   3-(2,1,3-Benzothiadiazol-5-yl)-4-(3,5-dimethoxybenzyl)-5-hydroxy-5-(4-methoxyphenyl)-5H-furan-2-on, F. 54°
von "A" mit 4-tert.-Butoxybenzaldehyd
   3-(2,1,3-Benzothiadiazol-5-yl)-4-(4-tert.-butoxybenzyl)-5-hydroxy-5-(4-methoxyphenyl)-5H-furan-2-on, F. 68° und daraus durch Umsetzung mit TFA
   3-(2,1,3-Benzothiadiazol-5-yl)-4-(4-hydroxybenzyl)-5-hydroxy-5-(4-methoxyphenyl)-5H-furan-2-on, F. 83°
von "A" mit 4-Trifluormethoxybenzaldehyd
   3-(2,1,3-Benzothiadiazol-5-yl)-4-(4-trifluormethoxybenzyl)-5-hydroxy-5-(4-methoxyphenyl)-5H-furan-2-on, F. 56°
von "A" mit 3,5-Dimethoxy-4-isopropoxy-benzaldehyd
   3-(2,1,3-Benzothiadiazol-5-yl)-4-(3,5-dimethoxy-4-isopropoxybenzyl)-5-hydroxy-5-(4-methoxyphenyl)-5H-furan-2-on, F. 62° von "A" mit 3,5-Dimethoxy-4-pentyloxy-benzaldehyd
   3-(2,1,3-Benzothiadiazol-5-yl)-4-(3,5-dimethoxy-4-pentyloxybenzyl)-5-hydroxy-5-(4-methoxyphenyl)-5H-furan-2-on, FAB 576
von "A" mit 3,5-Dimethoxy-4-hexyloxy-benzaldehyd
   3-(2,1,3-Benzothiadiazol-5-yl)-4-(3,5-dimethoxy-4-hexyloxybenzyl)-5-hydroxy-5-(4-methoxyphenyl)-5H-furan-2-on, FAB 590
von "A" mit 4-Phenoxybenzaldehyd
   3-(2,1,3-Benzothiadiazol-5-yl)-4-(4-phenoxybenzyl)-5-hydroxy-5-(4-methoxyphenyl)-5H-furan-2-on, F. 70°
von "A" mit 4,5-Dimethoxy-3-isopropoxy-benzaldehyd
   3-(2,1,3-Benzothiadiazol-5-yl)-4-(4,5-dimethoxy-3-isopropoxybenzyl)-5-hydroxy-5-(4-methoxyphenyl)-5H-furan-2-on, El 548
von 2-(2,1,3-Benzothiadiazol-5-yl)-4-(2,5-dimethoxyphenyl)-4-oxobutansäureethylester mit "E"
   3-(2,1,3-Benzothiadiazol-5-yl)-4-(3,4,5-trimethoxybenzyl)-5-hydroxy-5-(2,5-dimethoxyphenyl)-5H-furan-2-on, F. 73°
von 2-(2,1,3-Benzothiadiazol-5-yl)-4-(3-chlor-4-methoxyphenyl)-4-oxobutansäureethylester mit "E"
   3-(2,1,3-Benzothiadiazol-5-yl)-4-(3,4,5-trimethoxybenzyl)-5-hydroxy-5-(3-chlor-4-methoxyphenyl)-5H-furan-2-on, F. 158°
von 2-(2,1,3-Benzothiadiazol-5-yl)-4-(3-methyl-4-methoxyphenyl)-4-oxobutansäureethylester mit "E"
   3-(2,1,3-Benzothiadiazol-5-yl)-4-(3,4,5-trimethoxybenzyl)-5-hydroxy-5-(3-methyl-4-methoxyphenyl)-5H-furan-2-on, F. 80°
von 2-(2,1,3-Benzothiadiazol-5-yl)-4-(2,5-dimethoxyphenyl)-4-oxobutansäureethylester mit 3,4-Diisopropoxy-5-methoxy-benzaldehyd
   3-(2,1,3-Benzothiadiazol-5-yl)-4-(3,4-diisopropoxy-5-methoxybenzyl)-5-hydroxy-5-(2,5-dimethoxyphenyl)-5H-furan-2-on, F. 70°
von 2-(2,1,3-Benzothiadiazol-5-yl)-4-(2,3-dihydro-benzofuran-5-yl)-4-oxobutansäureethylester mit "E"
   3-(2,1,3-Benzothiadiazol-5-yl)-4-(3,4,5-trimethoxybenzyl)-5-hydroxy-5-(2,3-dihydrobenzofuran-5-yl)-5H-furan-2-on, El 532
von 2-(2,1,3-Benzothiadiazol-5-yl)-4-(3-fluor-4-methoxyphenyl)-4-oxobutansäureethylester mit 3,5-Dimethoxy-4-isopropoxy-benzaldehyd
   3-(2,1,3-Benzothiadiazol-5-yl)-4-(3,5-dimethoxy-4-isopropoxybenzyl)-5-hydroxy-5-(3-fluor-4-methoxyphenyl)-5H-furan-2-on, El 566
von "A" mit 3,4-Dimethoxy-5-propoxy-benzaldehyd
   3-(2,1,3-Benzothiadiazol-5-yl)-4-(3,4-dimethoxy-5-propoxybenzyl)-5-hydroxy-5-(4-methoxyphenyl)-5H-furan-2-on, F. 70°.

Analog werden die nachstehenden Verbindungen erhalten
3-(2,1,3-Benzothiadiazol-5-yl)-4-(3,4-diisopropoxy-5-methoxybenzyl)-5-hydroxy-5-(4-propoxyphenyl)-5H-furan-2-on, F. 150°
3-(2,1,3-Benzothiadiazol-5-yl)-4-(3,4-diisopropoxy-5-methoxybenzyl)-5-hydroxy-5-(2,4-dimethoxyphenyl)-5H-furan-2-on, F. 164°
3-(2,1,3-Benzothiadiazol-5-yl)-4-(4-benzyloxy-2-methoxybenzyl)-5-hydroxy-5-(4-methoxyphenyl)-5H-furan-2-on, El 566
3-(2,1,3-Benzothiadiazol-5-yl)-4-(2, 3,4-trimethoxybenzyl)-5-hydroxy-5-(4-methoxyphenyl)-5H-furan-2-on, F. 70°
3-(2,1,3-Benzothiadiazol-5-yl)-4-(3,4,5-trimethoxybenzyl)-5-hydroxy-5-(2,4-dimethoxyphenyl)-5H-furan-2-on, F. 70°
3-(2,1,3-Benzothiadiazol-5-yl)-4-(3,4,5-triethoxybenzyl)-5-hydroxy-5-(4-methoxyphenyl)-5H-furan-2-on, F. 139°
3-(2,1,3-Benzothiadiazol-5-yl)-4-(3,4,5-trimethoxybenzyl)-5-hydroxy-5-(4-difluormethoxyphenyl)-5H-furan-2-on, FAB 557
3-(2,1,3-Benzothiad iazol-5-yl)-4-(3-hydroxy-4-methoxybenzyl)-5-hydroxy-5-(4-methoxyphenyl)-5H-furan-2-on, FAB 477
3-(2,1,3-Benzothiadiazol-5-yl)-4-(2,4-dimethoxybenzyl)-5-hydroxy-5-(4-methoxyphenyl)-5H-furan-2-on, El 490
3-(2,1,3-Benzothiadiazol-5-yl)-4-(2,4,5-trimethoxybenzyl)-5-hydroxy-5-(4-methoxyphenyl)-5H-furan-2-on, F. 70°
3-(2,1,3-Benzothiadiazol-5-yl)-4-(3,4,5-trimethoxybenzyl)-5-hydroxy-5-(3-fluor-4-isopropoxyphenyl)-5H-furan-2-on, FAB 567
3-(2,1,3-Benzothiadiazol-5-yl)-4-(3,4,5-trimethoxybenzyl)-5-hydroxy-5-(3-fluor-4-propoxyphenyl)-5H-furan-2-on, FAB 567
3-(2,1,3-Benzothiadiazol-6-methyl-5-yl)-4-(3,5-dimethoxy-4-isopropoxybenzyl)-5-hydroxy-5-(4-methoxyphenyl)-5H-furan-2-on, F. 74°
3-(2,1,3-Benzothiadiazol-5-yl)-4-(3,5-dimethoxy-4-benzyloxybenzyl)-5-hydroxy-5-(4-methoxyphenyl)-5H-furan-2-on, F. 61° und daraus durch Umsetzung mit Trifluoressigsäure/Thioanisol
3-(2,1,3-Benzothiadiazol-5-yl)-4-(3,5-dimethoxy-4-hydroxybenzyl)-5-hydroxy-5-(4-methoxyphenyl)-5H-furan-2-on, F. 69°
3-(2,1,3-Benzothiadiazol-5-yl)-4-(3,5-dimethoxy-4-propoxybenzyl)-5-hydroxy-5-(4-methoxyphenyl)-5H-furan-2-on, F. 60°
3-(2,1,3-Benzothiadiazol-5-yl)-4-(3,4-dimethoxy-5-isopropoxybenzyl)-5-hydroxy-5-(1,4-benzodioxan-6-yl)-5H-furan-2-on, FAB 577
3-(2,1,3-Benzothiadiazol-6-methyl-5-yl)-4-(3,4,5-trimethoxybenzyl)-5-hydroxy-5-(4-methoxyphenyl)-5H-furan-2-on, F. 183°
3-(2,1,3-Benzothiadiazol-5-yl)-4-(4-isopropoxybenzyl)-5-hydroxy-5-(4-methoxyphenyl)-5H-furan-2-on, FAB 489
3-(2,1,3-Benzothiadiazol-5-yl)-4-(4-hexyloxybenzyl)-5-hydroxy-5-(4-methoxyphenyl)-5H-furan-2-on, FAB 531
3-(2,1,3-Benzothiadiazol-5-yl)-4-(3,5-dimethoxy-4-isopropoxybenzyl)-5-hydroxy-5-(1,4-benzodioxan-6-yl)-5H-furan-2-on, F. 145°
3-(2,1,3-Benzothiadiazol-5-yl)-4-(3-methoxy-5-butoxybenzyl)-5-hydroxy-5-(4-methoxyphenyl)-5H-furan-2-on, FAB 533
3-(2,1,3-Benzothiadiazol-5-yl)-4-(3,4-diisopropoxybenzyl)-5-hydroxy-5-(4-methoxyphenyl)-5H-furan-2-on, F. 52°
3-(2,1,3-Benzothiadiazol-5-yl)-4-(3,4,5-trimethoxybenzyl)-5-hydroxy-5-(2-fluor-4-methoxyphenyl)-5H-furan-2-on, FAB 539
3-(2,1,3-Benzothiadiazol-5-yl)-4-(3,4-dimethoxy-5-isopropoxybenzyl)-5-hydroxy-5-(3-fluor-4-methoxyphenyl)-5H-furan-2-on, F. 126°
3-(2,1,3-Benzothiadiazol-5-yl)-4-(3,4-dimethoxy-5-benzyloxybenzyl)-5-hydroxy-5-(4-methoxyphenyl)-5H-furan-2-on, FAB 597
3-(2,1,3-Benzothiadiazol-5-yl)-4-(3,4,5-trimethoxybenzyl)-5-hydroxy-5-(4-fluor-2-methoxyphenyl)-5H-furan-2-on, FAB 539
3-(2,1,3-Benzothiadiazol-5-yl)-4-(3,5-dimethoxy-5-ethoxybenzyl)-5-hydroxy-5-(4-methoxyphenyl)-5H-furan-2-on, F. 123°
3-(2,1,3-Benzothiadiazol-5-yl)-4-(4-methoxycarbonyl-benzyl)-5-hydroxy-5-(4-methoxyphenyl)-5H-furan-2-on, F. 71°
3-(2,1,3-Benzothiadiazol-5-yl)-4-(3,4-diisopropoxybenzyl)-5-hydroxy-5-(3-fluor-4-methoxyphenyl)-5H-furan-2-on, FAB 565
3-(2,1,3-Benzothiadiazol-5-yl)-4-(3,4,5-trimethoxybenzyl)-5-hydroxy-5-(4-benzyloxyphenyl)-5H-furan-2-on, FAB 489
3-(2,1,3-Benzothiadiazol-4-methyl-5-yl)-4-(3,4,5-trimethoxybenzyl)-5-hydroxy-5-(4-methoxyphenyl)-5H-furan-2-on, F. 168°
3-(2,1,3-Benzothiadiazol-5-yl)-4-(3,5-dimethoxy-4-isobutoxybenzyl)-5-hydroxy-5-(4-methoxyphenyl)-5H-furan-2-on, F. 154° und

### Beispiel 14

Analog Beispiel 4 erhält man durch Umsetzung von "C" mit 2-(7-Methoxy-1,3-benzodioxol-5-yl)-4-(4-methoxyphenyl)-4-oxo-butansäureethylester
4-(2,1,3-Benzothiadiazol-5-ylmethyl)-3-(7-methoxy-1,3-benzodioxol-5-yl)-5-hydroxy-5-(4-methoxyphenyl)-5H-furan-2-on, F. 191°.

### Beispiel 15

Analog Beispiel 2 erhält man
aus 3-(2,1,3-Benzothiadiazol-5-yl)-4-(3,4-diisopropoxy-5-methoxybenzyl)-5-hydroxy-5-(3-fluor-4-methoxyphenyl)-5H-furan-2-on
   2-(2,1,3-Benzothiadiazol-5-yl)-3-(3,4-diisopropoxy-5-methoxybenzyl)-4-(3-fluor-4-methoxyphenyl)-4-oxo-2-butensäure Natriumsalz, FAB 617
aus 3-(2,1,3-Benzothiadiazol-5-yl)-4-(3,5-dimethoxy-4-isopropoxybenzyl)-5-hydroxy-5-(4-methoxyphenyl)-5H-furan-2-on
   2-(2,1,3-Benzothiadiazol-5-yl)-3-(3,5-dimethoxy-4-isopropoxybenzyl)-4-(4-methoxyphenyl)-4-oxo-2-butensäure Natriumsalz, F. 65°.

Analog werden nachstehende Verbindungen erhalten
2-(2,1,3-Benzothiadiazol-5-yl)-3-(3,4-dimethoxy-5-isopropoxybenzyl)-4-(4-methoxyphenyl)-4-oxo-2-butensäure Natriumsalz, F. 57° und
2-(2,1,3-Benzothiadiazol-5-yl)-3-(3,5-dimethoxy-4-isopropoxybenzyl)-4-(3-fluor-4-methoxyphenyl)-4-oxo-2-butensäure Natriumsalz, FAB 589.

Analog erhält man durch Behandlung mit KOH
2-(2,1,3-Benzothiadiazol-5-yl)-3-(3,4,5-trimethoxybenzyl)-4-(4-methoxyphenyl)-4-oxo-2-butensäure Kaliumsalz, F. 202°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 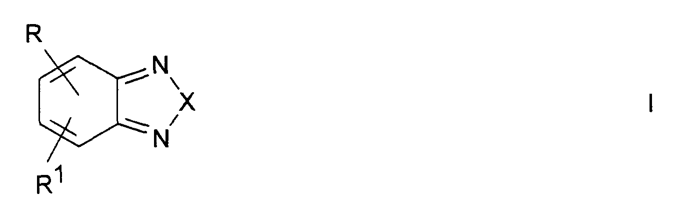 worin
R 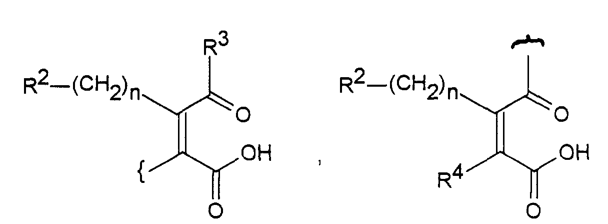 oder 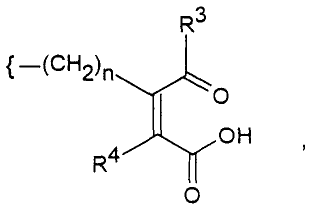
X O oder S,
R¹ H, Hal, OH, OA, A, Alkylen-O-A, NO₂, NH₂, NHAcyl, SO₂NH₂, SO₃-A, SO₂NHA, CN oder Formyl,
R², R³, R⁴ jeweils unabhängig voneinander eine unsubstituierte oder ein- oder mehrfach durch Hal, OH, OA, O-Alkylen-R⁵, A, S-A, SOA, SO₂A, SOR⁵, SO₂R⁵, NO₂, NH₂, NHA, NA₂, NHAcyl, NHSO₂A, NHSO₂R⁵, NASO₂A, NASO₂-R⁵, NH(CO)NH₂, NH(CO)NHA, Formyl, NH(CO)NHR⁵, NHCOOA, NAAcyl, NHCOOCH₂R⁵, NHSO₂CH₂R⁵, NHCOO-Alkylen-OA, NH(CO)NA₂,1-Piperidinyl-CO-NH, 1-Pyrrolidinyl-CONH, O(CH2)ₙCOOA, O(CH₂)ₙCOOH, O(CH₂)ₙOH, O(CH₂)ₙOA, CH₂OH, CH₂OA, COOH, COOA, CH₂COOH oder CH₂COOA substituierte Phenylgruppe,
eine 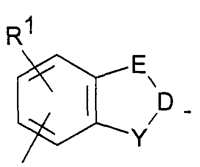 oder eine 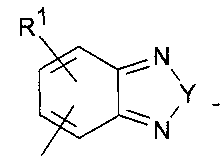 Gruppe, wobei
R² noch zusätzlich A oder Cycloalkyl bedeutet,
R⁵ eine unsubstituierte oder ein- oder mehrfach durch Hal, OH, OA, A, S-A, NO₂, NH₂, NHA, NA₂, NHAcyl, NHSO₂A, NASO₂A, NH(CO)NH₂, NH(CO)NHA, Formyl, NHCOOA, NAAcyl, NHCOO-Alkylen-OA, NH(CO)NA₂, N-Piperidinyl-CO-NH, N-Pyrrolidinyl-CONH, O(CH₂)ₙCOOA, O(CH₂)ₙCOOH, O(CH₂)ₙOH, O(CH₂)ₙOA, CH₂OH, CH₂OA, COOH, COOA, CH₂COOH oder CH₂COOA substituierte Phenylgruppe,
A Alkyl mit 1-6 C-Atomen, worin eine oder zwei CH₂-Gruppen durch O- oder S-Atome oder durch -CR⁶=CR^{6'}-Gruppen und/oder 1-7 H-Atome durch F ersetzt sein können,
D Carbonyl oder [C(R⁶R^{6'})]ₘ,
E CH₂, S oder O,
Y O oder S,
R⁶ und R^{6'} jeweils unabhängig voneinander H, F oder A,
Hal Fluor, Chlor, Brom oder lod,
n 1 oder 2 und
m 1 oder 2 bedeutet,
oder eine tautomere ringgeschlossene Form, sowie die (E)-Isomeren und die Salze aller Isomeren.

2. a) 2-(2,1,3-Benzothiadiazol-5-yl)-3-(3,4,5-trimethoxybenzyl)-4-(4-methoxyphenyl)-4-oxo-2-butensäure;
b) 2-(2,1,3-Benzothiadiazol-5-yl)-3-(3,4,5-trimethoxybenzyl)-4-(3-fluor-4-methoxyphenyl)-4-oxo-2-butensäure;
c) 3-(2,1,3-Benzothiadiazol-5-yl)-4-(3,4,5-trimethoxybenzyl)-5-hydroxy-5-(4-methoxyphenyl)-5H-furan-2-on;
d) 3-(2,1,3-Benzothiadiazol-5-yl)-4-(3,4,5-trimethoxybenzyl)-5-hydroxy-5-(3-fluor-4-methoxyphenyl)-5H-furan-2-on;
e) 2-(2,1,3-Benzothiadiazol-5-yl)-3-benzyl-4-(1,4-benzodioxan-6-yl)-4-oxo-2-butensäure;
f) 2-(2,1,3-Benzothiadiazol-5-yl)-3-benzyl-4-(3-fluor-4-methoxyphenyl)-4-oxo-2-butensäure;
g) 2-(2,1,3-Benzothiadiazol-5-yl)-3-cyclohexylmethyl-4(3-fluor-4-methoxyphenyl)-4-oxo-2-butensäure;
h) 2-(2,1,3-Benzothiadiazol-5-yl)-3-[3-(N,N-dimethylamino)-benzyl]-4-(4-methoxyphenyl)-4-oxo-2-butensäure;
sowie ihre Salze,
gemäß Anspruch 1.

3. Verfahren zur Herstellung von Verbindungen der Formel I nach Anspruch 1 sowie ihrer Salze,
worin
a) R 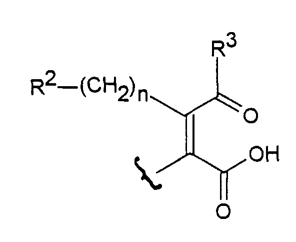 bedeutet,
**dadurch gekennzeichnet, daß** man
eine Verbindung der Formel II 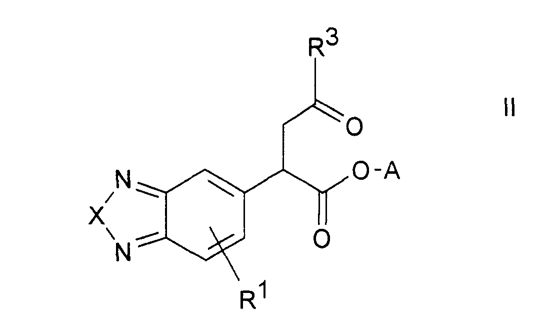 worin
R¹, R³ und X die in Anspruch 1 angegebene Bedeutung haben, und A Alkyl mit 1-4 C-Atomen oder Benzyl bedeutet,
mit einer Verbindung der Formel III
R²-(CH₂)ₖ-CHO III
worin
R² die in Anspruch 1 angegebene Bedeutung hat und
k 0 oder 1 bedeutet,
umsetzt
und anschließend den Ester spaltet,
oder daß man zur Herstellung von Verbindungen der Formel I nach Anspruch 1 sowie ihrer Salze,
worin
b) R 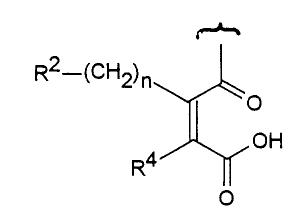 bedeutet,
eine Verbindung der Formel IV 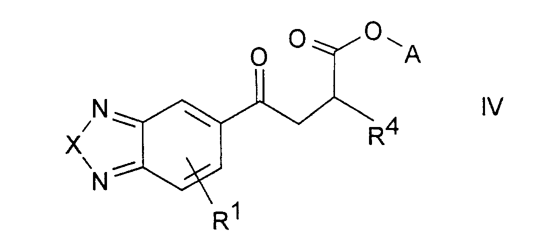 worin
R¹, R⁴ und X die in Anspruch 1 angegebene Bedeutung haben, und A Alkyl mit 1-4 C-Atomen oder Benzyl bedeutet,
mit einer Verbindung der Formel III, wie angegeben,
umsetzt,
und anschließend den Ester spaltet,
oder daß man zur Herstellung von Verbindungen der Formel I nach Anspruch 1 sowie ihrer Salze,
worin
c) R 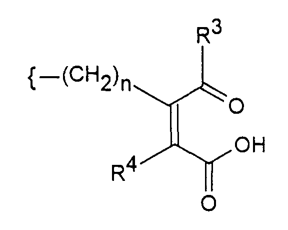 bedeutet,
eine Verbindung der Formel V 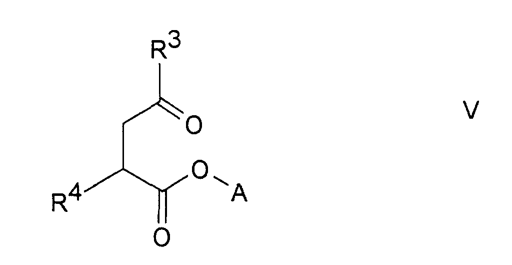 worin
R³ und R⁴ die in Anspruch 1 angegebene Bedeutung haben, und A Alkyl mit 1-4 C-Atomen oder Benzyl bedeutet,
mit einer Verbindung der Formel VI, 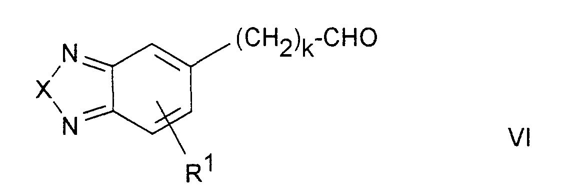 worin
R¹ und X die in Anspruch 1 angegebenen Bedeutungen haben, und k 0 oder 1 bedeutet,
umsetzt,
und anschließend den Ester spaltet,
und/oder daß man in einer Verbindung der Formel I einen oder mehrere Rest(e) R¹, R², R³ und/oder R⁴ in einen oder mehrere Reste R¹, R², R³ und/oder R⁴ umwandelt,
indem man beispielsweise
i) eine Nitrogruppe zu einer Aminogruppe reduziert,
ii) eine Aminogruppe acyliert oder alkyliert,
iii) eine Aminogruppe in eine Sulfonamidogruppe umwandelt,
und/oder eine Base oder Säure der Formel I in eines ihrer Salze umwandelt.

4. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Hypertonie, Herzinsuffizienz, Niereninsuffizienz, Hirninfarkt, koronarer Herzerkrankung, renaler, cerebraler und myocardialer Ischämie, subarachnoidaler Hämorrhagie, Entzündungen, Asthma und endotoxischem Schock.

7. Arzneimittel der Formel I nach Anspruch 1 und ihre physiologisch unbedenklichen Salze als Endothelin-Rezeptor-Antagonisten.

8. Verwendung von Verbindungen der Formel I nach Anspruch 1 und/oder ihre physiologisch unbedenklichen Salze zur Herstellung eines Arzneimittels.

## Claims

1. Compounds of the formula I 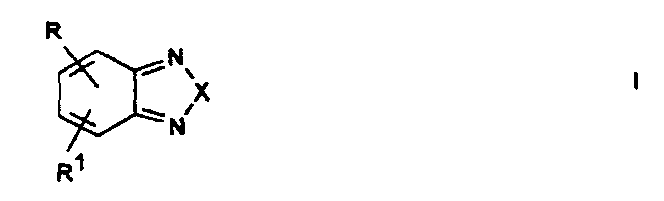 in which
R is 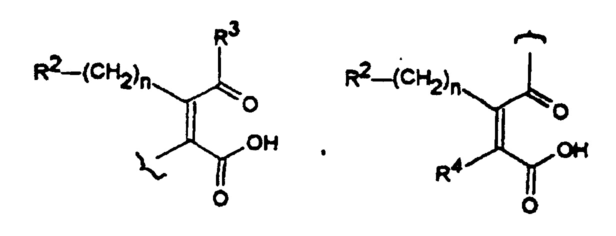 or 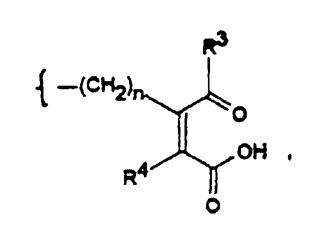
x is O or S,
R¹ is H, Hal, OH, OA, A, alkylene-O-A, NO₂, NH₂, NHacyl, SO₂NH₂, SO₃-A, SO₂NHA, CN or formyl,
R² , R³ , R⁴ in each case independently of one another are a phenyl group which is unsubstituted or mono- or polysubstituted by Hal, OH, OA, O-alkylene-R⁵, A, S-A, SOA, SO₂A, SOR⁵, SO₂R⁵, NO₂, NH₂, NHA, NA₂, NHacyl, NHSO₂A, NHSO₂R⁵, NASO₂A, NASO₂-R⁵, NH(CO)NH₂, NH(CO)NHA, formyl, NH(CO)NHR⁵, NHCOOA, NAacyl, NHCOOCH₂R⁵, NHSO₂CH₂R⁵, NHCOO-alkylene-OA, NH(CO)NA₂, 1-piperidinyl-CO-NH, 1-pyrrolidinyl-CONH, O(CH₂)ₙCOOA, O(CH₂)ₙCOOH, O(CH₂)ₙOH, O(CH₂)ₙOA, CH₂OH, CH₂OA, COOH, COOA, CH₂COOH or CH₂COOA,
a 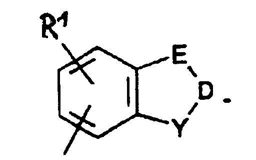 or a 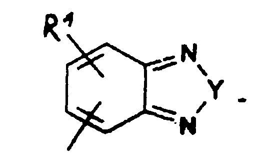 group, where
R² is additionally A or cycloalkyl,
R⁵ is a phenyl group which is unsubstituted or mono- or polysubstituted by Hal, OH, OA, A, S-A, NO₂, NH₂, NHA, NA₂, NHacyl, NHSO₂A, NASO₂A, NH(CO)NH₂, NH(CO)NHA, formyl, NHCOOA, NAacyl, NHCOO-alkylene-OA, NH(CO)NA₂, N-piperidinyl-CO-NH, N-pyrrolidinyl-CONH, O(CH₂)ₙCOOA, O(CH₂)ₙCOOH, O(CH₂)ₙOH, O(CH₂)ₙOA, CH₂OH, CH₂OA, COOH, COOA, CH₂COOH or CH₂COOA,
A is alkyl having 1-6 C atoms, in which one or two CH₂ groups can be replaced by O or S atoms or by -CR⁶=CR^{6'} groups and/or 1-7 H atoms can be replaced by F,
D is carbonyl or [C(R⁶R^{6'})]ₘ,
E is CH₂, S or O,
Y is O or S,
R⁶ and R^{6'} in each case independently of one another are H, F or A,
Hal is fluorine, chlorine, bromine or iodine,
n is 1 or 2 and
m is 1 or 2,
or a tautomeric ring-closed form, and the (E)isomers and the salts of all isomers.

2. a) 2-(2,1,3-Benzothiadiazol-5-yl)-3-(3,4,5-trimethoxybenzyl)-4-(4-methoxyphenyl)-4-oxo-2-butenoic acid;
b) 2-(2,1,3-benzothiadiazol-5-yl)-3-(3,4,5-trimethoxybenzyl)-4-(3-fluoro-4-methoxyphenyl)-4-oxo-2-butenoic acid;
c) 3-(2,1,3-benzothiadiazol-5-yl)-4-(3,4,5-trimethoxybenzyl)-5-hydroxy-5-(4-methoxyphenyl)-5H-furan-2-one;
d) 3-(2,1,3-benzothiadiazol-5-yl)-4-(3,4,5-trimethoxybenzyl)-5-hydroxy-5-(3-fluoro-4-methoxyphenyl)-5H-furan-2-one;
e) 2-(2,1,3-benzothiadiazol-5-yl)-3-benzyl-4-(1,4-benzodioxan-6-yl)-4-oxo-2-butenoic acid;
f) 2-(2,1,3-benzothiadiazol-5-yl)-3-benzyl-4-(3-fluoro-4-methoxyphenyl)-4-oxo-2-butenoic acid;
g) 2-(2,1,3-benzothiadiazol-5-yl)-3-cyclohexylmethyl-4-(3-fluoro-4-methoxyphenyl)-4-oxo-2-butenoic acid;
h) 2-(2,1,3-benzothiadiazol-5-yl)-3-[3-(N,N-dimethylamino)benzyl]-4-(4-methoxyphenyl)-4-oxo-2-butenoic acid;
and their salts,
according to Claim 1.

3. Process for the preparation of compounds of the formula I according to Claim 1 and their salts,
in which
a) R is 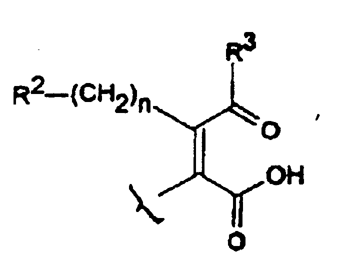 **characterized in that** a compound of the formula II 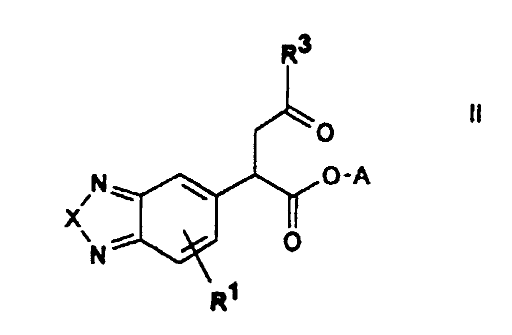 in which
R¹,R³ and X have the meaning indicated in Claim 1, and A is alkyl having 1-4 C atoms or benzyl,
is reacted with a compound of the formula III
R²-(CH₂)ₖ-CHO III
in which
R² has the meaning indicated in Claim 1 and
k is 0 or 1,
and then the ester is cleaved,
or in that, for the preparation of compounds of the formula I according to Claim 1 and their salts,
in which
b) R is 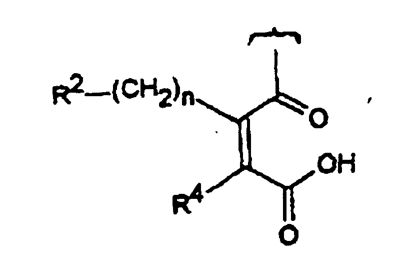 a compound of the formula IV 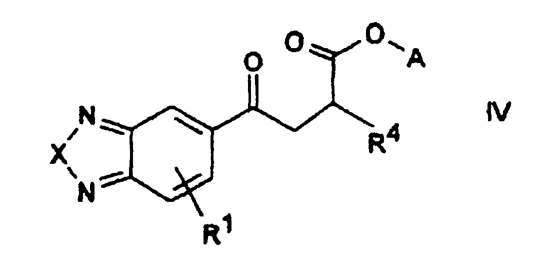 in which
R¹,R⁴ and X have the meaning indicated in Claim 1, and A is alkyl having 1-4 C atoms or benzyl,
is reacted with a compound of the formula III, as indicated,
and then the ester is cleaved,
or in that, for the preparation of compounds of the formula I according to Claim 1 and their salts,
in which
c) R is 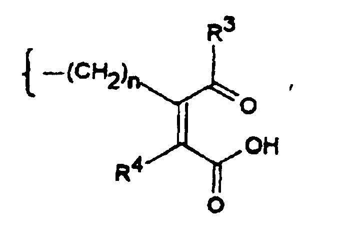 a compound of the formula V 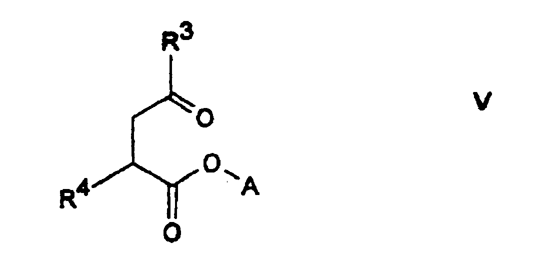 in which
R³ and R⁴ have the meaning indicated in Claim 1, and A is alkyl having 1-4 C atoms or benzyl,
is reacted with a compound of the formula VI, 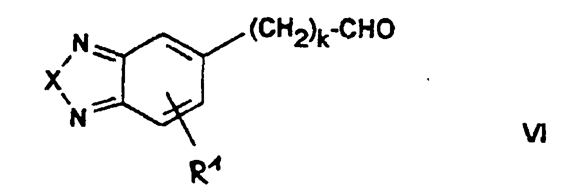 in which
R¹ and X have the meanings indicated in Claim 1, and k is 0 or 1,
and then the ester is cleaved,
and/or in that, in a compound of the formula I one or more radicals R¹,R²,R³ and/or R⁴ are converted into one or more radicals R¹,R²,R³ and/or
R⁴,
by, for example,
i) reducing a nitro group to an amino group,
ii) acylating or alkylating an amino group,
iii) converting an amino group into a sulfonamido group,
and/or converting a base or acid of the formula I into one of its salts.

4. Process for the production of pharmaceutical preparations, **characterized in that** a compound of the formula I according to Claim 1 and/or one of its physiologically acceptable salts is brought into a suitable dose form together with at least one solid, liquid or semiliquid excipient or auxiliary.

5. Pharmaceutical preparation, **characterized in that** it contains at least one compound of the formula I according to Claim 1 and/or one of its physiologically acceptable salts.

6. Compounds of the formula I according to Claim 1 and their physiologically acceptable salts for the control of hypertension, cardiac insufficiency, renal insufficiency, cerebral infarct, coronary heart disease, renal, cerebral and myocardial ischaemia, subarachnoid haemorrhage, inflammations, asthma and endotoxic shock.

7. Medicaments of the formula I according to Claim 1 and their physiologically acceptable salts as endothelin receptor antagonists.

8. Use of compounds of the formula I according to Claim 1 and/or their physiologically acceptable salts for the production of a medicament.

## Revendications

1. Composés de formule I 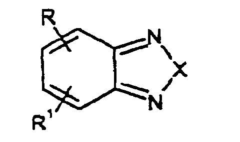 dans laquelle
R est 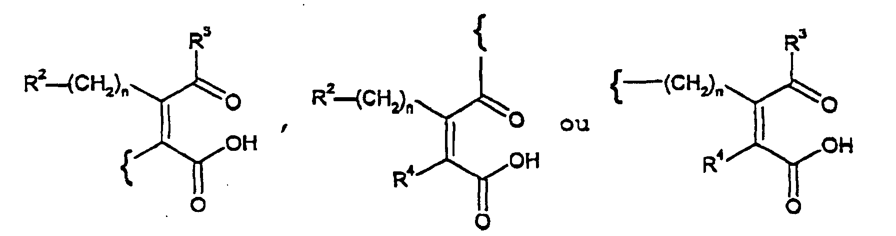
X est O ou S,
R¹ est H, Hal, OH, OA, A, un groupe alkylène-O-A, NO₂, NH₂, un groupe NH-acyle, SO₂NH₂, SO₃-A, SO₂NHA, CN ou le groupe formyle,
R², R³ et R⁴ représentent chacun indépendamment des autres un groupe phényle non-substitué ou une ou plusieurs fois substitué par Hal, OH, OA, un groupe O-alkylène-R⁵, A, S-A, SOA, SO₂A, SOR⁵, SO₂R⁵, NO₂, NH₂, NHA, NA₂, un groupe NH-acyle, NHSO₂A, NHSO₂R⁵, NaSO₂A, NASO₂-R⁵, NH(CO)NH₂, NH(CO)NHA, le groupe formyle, NH(CO)NHR⁵, NHCOOA, un groupe NA-acyle, NHCOOCH₂R⁵, NHSO₂CH₂R⁵, un groupe NHCOO-alkylène-OA, NH(CO)NA₂, le groupe 1-pipéridinyl-CO-NH, 1-pyrrolidinyl-CONH, O(CH₂)ₙCOOA, O(CH₂)ₙCOOH, O(CH₂)ₙOH, O(CH₂)ₙOA, CH₂OH, CH₂OA, COOH, COOA, CH₂COOH ou CH₂COOA,
un groupe 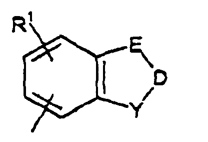 ou un groupe 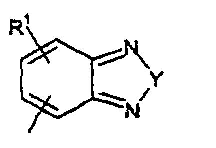 où R² représente encore et en outre A ou un groupe cycloalkyle,
R⁵ est un groupe phényle non-substitué ou une ou plusieurs fois substitué par Hal, OH, OA, A, S-A, NO₂, NH₂, NHA, NA₂, un groupe NH-acyle, NHSO₂A, NASO₂A, NH(CO)NH₂, NH(CO)NHA, le groupe formyle, NHCOOA, un groupe NA-acyle, NHCOO-alkylène-OA, NH(CO)NA₂, le groupe N-pipéridinyl-CO-NH, N-pyrrolidinyl-CONH, O(CR₂)ₙCOOA, O(CH₂)ₙCOOH, O(CH₂)ₙOH, O(CH₂)ₙOA, CH₂OH, CH₂OA, COOH, COOA, CH₂COOH ou CH₂COOA,
A est un groupe alkyle ayant de 1 à 6 atomes de carbone, où un ou deux groupes CH₂ peuvent être remplacés par des atomes 0 ou S ou par des groupes-CR⁶=CR^{6'}-, et/ou 1-7 atomes d'hydrogène peuvent être remplacés par F,
D est le groupe carbonyle ou [C(R⁶R^{6'})]ₘ,
E est CH₂, S ou O,
Y est O ou S,
R⁶ et R^{6'} représentent chacun indépendamment de l'autre H, F ou A,
Hal est le fluor, le chlore, le brome ou l'iode,
n vaut 1 ou 2, et
m vaut 1 ou 2,
ou une forme cyclisée tautomère, ainsi que les isomères (E) et les sels de tous les isomères.

2. a) Acide 2-(2,1,3-benzothiadiazole-5-yl)-3-(3,4,5-triméthoxybenzyl)-4-(4-méthoxyphényl)-4-oxo-2-buténoïque ;
b) acide 2-(2,1,3-benzothiadiazole-5-yl)-3-(3,4,5-triméthoxybenzyl)-4-(3-fluoro-4-méthoxyphényl)-4-oxo-2-buténoïque ;
c) 3-(2,1,3-benzothiadiazole-5-yl)-4-(3,4,5-triméthoxybenzyl)-5-hydroxy-5-(4-méthoxyphényl)-5H-furanne-2-one ;
d) 3-(2,1,3-benzothiadiazole-5-yl)-4-(3,4,5-triméthoxybenzyl)-5-hydroxy-5-(3-fluoro-4-méthoxyphényl)-5H-furanne-2-one ;
e) acide 2-(2,1,3-benzothiadiazole-5-yl)-3-benzyl-4-(1,4-benzodioxanne-6-yl)-4-oxo-2-buténoïque ;
f) acide 2-(2,1,3-benzothiadiazole-5-yl)-3-benzyl-4-(3-fluoro-4-méthoxyphényl)-4-oxo-2-buténoïque ;
g) acide 2-(2,1,3-benzothiadiazole-5-yl)-3-cyclohexylméthyl-4-(3-fluoro-4-méthoxyphényl)-4-oxo-2-buténoïque ;
h) acide 2-(2,1,3-benzothiadiazole-5-yl)-3-[3-(N,N-diméthylamino)-benzyl]-4-(4-méthoxyphényl)-4-oxo-2-buténoïque ;
ainsi que leurs sels, selon la revendication 1.

3. Procédé de préparation de composés de formule I selon la revendication 1, ainsi que de leurs sels, dans lequel
a) R est 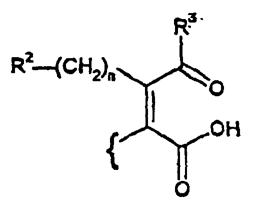 **caractérisé en ce qu'**on fait réagir un composé de formule II 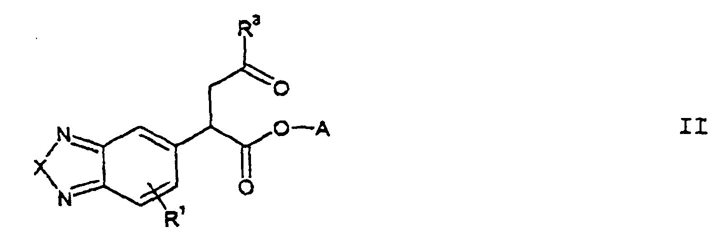 dans laquelle R¹, R³ et X ont les significations données dans la revendication 1, et A est un groupe alkyle ayant de 1-4 atomes de carbone ou le groupe benzyle,
avec un composé de formule III
R²-(CH₂)ₖ-CHO III
dans laquelle
R² a les significations données dans la revendication 1, et
k vaut 0 ou 1,
puis on élimine l'ester,
ou en ce que, pour préparer des composés de formule I selon la revendication 1 ainsi que leurs sels, où
b) R est 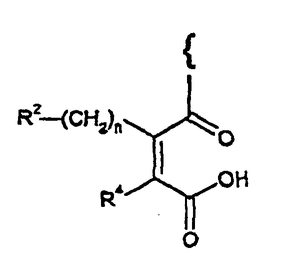 on fait réagir un composé de formule IV 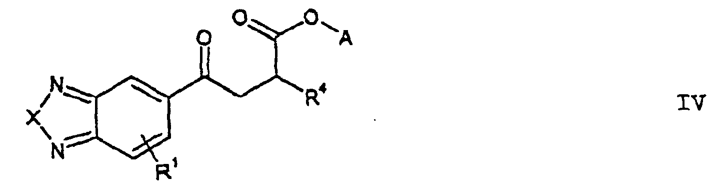 dans laquelle
R¹, R⁴ et X ont les significations données dans la revendication 1, et A est un groupe alkyle ayant de 1-4 atomes de carbone ou le groupe benzyle,
avec un composé de formule III tel qu'indiqué,
puis on élimine l'ester,
ou en ce que, pour préparer des composés de formule I selon la revendication 1, ainsi que leurs sels, où
c) R est 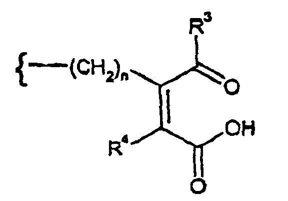 on fait réagir un composé de formule V 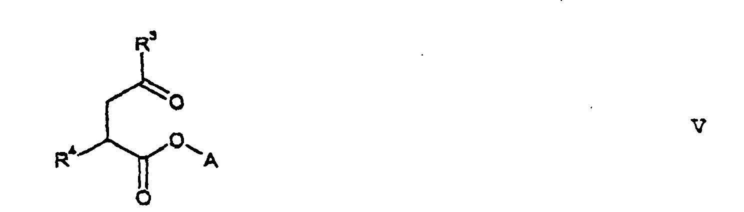 dans laquelle R³ et R⁴ ont les significations données dans la revendication 1 et A est un groupe alkyle ayant de 1 à 4 atomes de carbone ou le groupe benzyle,
avec un composé de formule VI 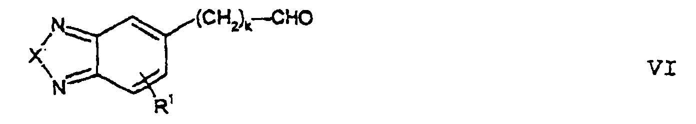 dans laquelle R¹ et X ont les significations données dans la revendication 1 et k vaut 0 ou 1,
puis on élimine l'ester,
et/ou en ce qu'on convertit, dans le composé de formule
I, un ou plusieurs radicaux R¹, R², R³ et/ou R⁴ en un ou plusieurs radicaux R¹, R², R³ et/ou R⁴,
ce pour quoi par exemple
i) on réduit un groupe nitro en un groupe amino,
ii) on acyle ou alkyle un groupe amino,
iii) on convertit un groupe amino en un groupe sulfonamido,
et/ou on convertit une base ou un acide de formule I en l'un de ses sels.

4. Procédé de fabrication de préparations pharmaceutiques, **caractérisé en ce qu'**on met sous une forme posologique appropriée un composé de formule I selon la revendication 1 et/ou l'un de sels inoffensifs du point de vue physiologique, en même temps qu'au moins un support ou adjuvant solide, liquide ou semi-solide.

5. Préparation pharmaceutique, **caractérisée en ce qu'**elle contient au moins un composé de formule I selon la revendication 1 et/ou l'un de ses sels inoffensifs du point de vue physiologique.

6. Composés de formule I selon la revendication 1, et ses sels inoffensifs du point de vue physiologique, pour lutter contre l'hypertonie, l'insuffisance cardiaque, l'insuffisance rénale, l'infarctus cérébral, les maladies coronariennes, l'ischémie rénale, cérébrale et myocardique, l'hémorragie sousarachnoïdienne, les inflammations, l'asthme et le choc endotoxique.

7. Médicaments de formule I selon la revendication 1 et leurs sels inoffensifs du point de vue physiologique, en tant qu'antagonistes du récepteur de l'endothéline.

8. Utilisation de composés de formule I selon la revendication 1 et/ou de ses sels inoffensifs d'un point de vue physiologique pour préparer un médicament.
